(19) Europäisches Patentamt
European Patent Office
Office européen des brevets

(11) EP 3 916 094 A1

(12) EUROPEAN PATENT APPLICATION
published in accordance with Art. 153(4) EPC

(43) Date of publication:
**01.12.2021 Bulletin 2021/48**

(21) Application number: **20745053.7**

(22) Date of filing: **22.01.2020**

(51) Int Cl.:
***C12N 15/113*** *(2010.01)* ***A61K 48/00*** *(2006.01)*
***A61K 31/713*** *(2006.01)* ***A61P 17/00*** *(2006.01)*

(86) International application number:
**PCT/CN2020/073663**

(87) International publication number:
**WO 2020/151726 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.01.2019 CN 201910067625**

(71) Applicant: **Ractigen Therapeutics**
**Nantong, Jiangsu 226400 (CN)**

(72) Inventors:
- **LI, Longcheng**
**Nantong City, Jiangsu 226400 (CN)**
- **KANG, MooRim**
**Nantong City, Jiangsu 226400 (CN)**

(74) Representative: **Høiberg P/S**
**Adelgade 12**
**1304 Copenhagen K (DK)**

# (54) OLIGONUCLEOTIDES FOR SKIN CARE

(57) Provided are small activating nucleic acid molecules for skin care and uses thereof. The small activating nucleic acid molecule of the present invention comprises two oligonucleotide strands of 16 to 35 nucleotides in length, wherein one nucleotide strand has at least 75% homology or complementarity to a target selected from a promoter region of a target gene. Also provided are skin care products comprising a small activating nucleic acid molecule targeting the promoter region of the AQP3, AQP9, ELN, COL1A1, COL1A2, COL3A1, HAS1, HAS2, HAS3 or MFAP2 genes or a nucleic acid encoding the same and optionally an carrier or other effective ingredients. Further provided are methods for upregulating the expression of a target gene in cells using the small activating nucleic acid molecule or the nucleic acid encoding the same and improving skin conditions using the skin care products.

EP 3 916 094 A1

## Description

TECHNICAL FIELD

The present invention relates to the technical field of nucleic acids, and in particular to an oligomeric nucleic acid molecule related with gene activation and use thereof in skin care.

BACKGROUND

The skin is an important organ composed of epidermis, dermis and subcutaneous tissues, which covers the whole surface of body to provide a barrier function for the body. The dermis, which is located under the epidermis to support skin structure, is composed of papillary dermis and reticular dermis and filled with a biological structure called extracellular matrix. The extracellular matrix of the skin is a complex macromolecular network composed of collagen, elastic fibers, glycoprotein, glycosaminoglycan, proteoglycan, *etc.,* which provides structural support and participates in the regulation of cell metabolism. Extracellular matrix protein are mainly synthesized and secreted by fibroblasts in the dermis, and more than 70% of the proteins are collagen providing strength for the skin. Other fibers forming the extracellular matrix of the dermis comprise elastic fibers, which are mainly composed of tropoelastin polymerized on microfibers. The production of the extracellular matrix and enzymes involved in the degradation thereof is controlled by fibroblasts to a great extent, and the balance between the production and degradation is a basis of keeping skin in a stable state.

Collagen

As a component of the extracellular matrix, collagen plays an important role in maintaining the elasticity and moisture of the skin and preventing bacterial or viral infection, skin diseases and invasion of allergens and other foreign objects. During the process of skin aging, the synthesis rate of collagen and elastin fibers decreases, and the rate of degradation increases rapidly. These two aging processes result in the reduction of the fiber numbers in the dermis, which cause changes in skin properties, leading to signs of skin aging such as flecks, wrinkles, skin sagging, and roughness.

Collagen expressed by the skin of human body includes collagens I, III, IV, V, VI, VII, XIV, XVII and XVIII, of which collagen I and collagen III account for 80% of the dry weight of normal adult skin. Both tof collagens are of a fiber-like structure. As a main component of the dermis, collagen I accounts for 80% of total collagen and has a very strong function of promoting cell adhesion and migration of cutin. Collagen III is also a main component of the extracellular matrix of the dermis, and accounts for 10% of dermis collagen. The COL1A1, COL1A2 and COL3A1 genes code for collagen type I $\alpha$1-chain, collagen type I $\alpha$2-chain, and collagen type III $\alpha$1-chain, respectively.

There are many reports on substances (such as plant extracts), polypeptides, and other materials for increasing skin collagen, but most of the materials are not effective and high in manufacturing cost. Therefore, a method that can promote the production and secretion of collagen by cells may become an effective means for anti-aging and skin beauty.

### Aquaporin

Aquaporins (AQPs) are responsible for transporting water and other small molecules to maintain the stable liquid state of cells. In human cells, there are 13 AQP family members (AQP0 to AQP12), which are expressed in various organs. The AQP family members can be classified into three categories according to the properties of the AQPs: the first category is AQP0 to AQP6, through which water can pass; the second category is AQP3 and AQP7, through which water, glycerin, and urea can pass; the third category is AQP9 and AQP10, through which water and neutral substances can pass. Among the AQP family members, AQP3 is mainly distributed in the skin. AQP3 can transport not only water but also urea, glycerin, and other substances into and out of the skin and is a key factor in maintaining skin hydration, therefore, the AQP3 gene is involved with locking water to moisturize the skin (Schrader et al., Skin Pharmacol Physiol 2012; 25 (4): 192-9).

The quality and normal function of skin are closely related to moisture contents of different layers of the epidermis. In normal epidermis, the proliferating layer contains about 70% of water and the horny layer contains about 10% to 15% of water. The moisturization of the horny layer is the result of three factors: water supply from the dermis, loss of water to the external environment, and ability of the horny layer in retaining water molecules. The reduction of skin function with aging is related to the reduction of moisture content of the skin. Ultraviolet rays can down-regulate the expression of AQP3, and thus damage the barrier function of the skin. Compared with the young undamaged skin, aged human skin exhibits the reduced expression of AQP3, which leads to a variety of skin defects, such as insufficient moisturizing function, damaged barrier function, prolonged wound healing time, and decreased elasticity (Li et al., Australasian Journal of Dermatology. 2010 May; 51(2): 106-12, Hammam et al., Advances in Environmental Biology. 2016 Jan; 10(1): 237-49). Therefore, the increase of AQP3 in the skin can result in better moisturization of the epidermis (Dumas et al., Journal of drugs in dermatology 2007 Jun; 6(6 Suppl): s20-4).

AQP9 is an important water channel molecule and is mainly expressed in the keratinocytes of the skin (i.e., the outermost layer of the skin), and decreases with aging (Karlsson et al., Biochem. Biophys. Res. Commun. 2013, 430, 993—998), which suggests that AQP9 may play an important role in resisting skin aging.

However, so far, there has not been any reports on methods capable of specifically activating endogenous water channel genes. In this view, the present invention provides a method for activating AQP3, AQP9, and other water channel genes, and compositions and methods for improving skin conditions.

Elastin

Elastin or elastic fiber is an extracellular matrix protein which is directly related to skin elasticity and produced by fibroblasts in the dermis of the skin. In addition to its function in skin, elastic fibers also provide structural support for arteries, lung, tendons, ligaments, and other tissues. In the process of skin aging, the synthesis rate of elastin fibers decreases and the degradation level significantly increases, resulting in the aging characteristics of fine lines, wrinkles, senile plaques and skin sagging appearing on the skin. In human cells, elastin is coded by the ELN (elastin) gene.

Hyaluronic Acid

Hyaluronic acid (HA), also called hyaluronan, is a glycosaminoglycan existing widely in intercelluar substances of various tissues of animals. All layers of the skin contain hyaluronic acid, but hyaluronic acid is mainly concentrated in dermal papillary layer and basement membrane zone. Many types of cells can produce HA, and the HA of the skin is mainly produced by dermal fibroblasts and synthesized by HA synthase. Mammalian cells have three highly homologous hyaluronic acid synthase (HAS) genes, which include HAS1, HAS2 and HAS3. HA is characterized by high viscosity and good lubricating effect. HA regulates water balance in the skin, through a sieve tube-like effect and a barrier effect, which provides elasticity and shear resistance for the skin and maintains the integrity of the skin in combination with other extracellular matrix components. Moreover, HA forms a loose network structure between molecular chains in aqueous solution which can combine and retain a large amount of water. HA is not only associated with maintaining moisture, keeping space between cells, and preserving and spreading cell growth factors and nutrients, but also to the division, differentiation, and migration of cells, and the moisturization and flexibility of the skin. Promoting the expression of hyaluronic acid synthase genes has a moisturizing and a skin-beautifying effect. The concentration of hyaluronic acid in the human skin decreases with aging, and leads to a loss of skin elasticity and a decrease in water-retaining properties (Biochem. Biophys. Acta. 279, 265-275, Carbohydr. Res. 159, 127-136, Int. J. Dermatol. 33, 119-122).

The amount of hyaluronic acid in human skin decreases with aging. It is considered in a study that the decrease of the amount of hyaluronic acid in skin is one of the main reasons for the decreases in skin elasticity and moisture content with aging (Fleischmajer et al., Biochem. Biophys. Acta. 1972; 279: 265-275). The use of HA in clinical and cosmetic and skin-care is based on the high viscosity and water-retaining property of HA. Therefore, injecting exogenous hyaluronic acid into the skin has become a popular cosmetic procedure. The method that can promote skin cells to produce endogenous hyaluronic acid and provides a safer and more effective cosmetic means.

Microfibril-associated Protein 2 (MFAP2)

MFAP2, also called MAGP-1 (microfibril-associated glycoprotein-1), is a glycine-rich acidic protein of the extracellular matrix, and plays a key role in elastic fiber assembly. As the level of the MFAP2 gene and protein in the human skin decreases with increase in age and light exposure, leading to the loss of a functional MFAP2 fiber network and the lack of structural support in the dermis. Moreover, the decrease in MFAP2 protein around hair follicles and pore regions results in the loss of structural support around hair follicles, fragile skin, skin sagging, and enlarged pores. In addition to providing structural support for the formation of elastic fibers of the skin, MFAP2 is a key factor participating in regulation of vascular integrity, wound healing, and body fat deposition at appropriate parts by regulating TGF-$\beta$ signaling pathways.

With the improvement of living standards, there is an increasing demand for products that can be externally applied to the skin to improve the health and appearance thereof. Consumers hope to use these products to reduce or delay natural aging or aged skin appearance caused by environmental factors, such as dry skin, dull skin, fine lines, wrinkles, loss of elasticity, sagging, lack of elasticity, lack of fullness, flecks, abnormal desquamation and large pores. At present, skin-care products in the market are known to improve skin appearance, but has a limited effect. So far, there has not been any safe and effective method in the market that can achieve the goal of improving skin conditions and resisting skin aging by specifically increasing the expression of one or more genes to increase the expression of the endogenous proteins thereof.

Therefore, the present invention provides a small nucleic acid molecule which can be applied to the skin. The molecule can achieve the effect of safely and effectively improving skin conditions and reducing skin aging by specifically activating the expression of genes related to skin physiology and pathology.

## SUMMARY

In order to solve the aforementioned problem, the present invention provides a small activating nucleic acid molecule based on the process of RNA activation, for example, a small activating RNA (saRNA) molecule, which can improve skin conditions by activating/upregulating the transcription of one or more of collagen genes, aquaporin genes, elastin genes, hyaluronic acid genes, and microfibril-associated protein genes to increase the expression of the corresponding endogenous protein.

One aspect of the present invention provides a small activating nucleic acid molecule, for example, a small activating RNA (saRNA) molecule, which can activate or upregulate the expression of at least one of AQP3, AQP9, ELN, COL1A1, COL1A2, COL3A1, HAS1, HAS2, HAS3 and MFAP2 genes in a cell. One strand of the small activating nucleic acid molecule of the present invention has at least more than 75% (e.g., at least about 79%, about 80%, about 85%, about 90%, about 95% or about 99%) homology or complementarity to a fragment of 16 to 35 nucleotides in length in the promoter region of any of the aforementioned genes. More specifically, for the AQP3 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 249-263; for the AQP9 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 264-276; for the ELN gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 277-286; for the COL1A1 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 287-294; for the COL1A2 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 295-306; for the COL3A1 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 307-317; for the HAS1 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 318-326; for the HAS2 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 327-340; for the HAS3 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 341-357; and for the MFAP2 gene, one strand of the small activating nucleic acid molecule of the present invention has at least 75% (e.g., at least about 79%, about 80%, about 85%, about 90%, about 95% or about 99%) homology or complementarity to any nucleotide sequence selected from SEQ ID NOs: 358-372.

In the present invention, the small activating nucleic acid molecule comprises a sense nucleic acid fragment and an antisense nucleic acid fragment, wherein the sense nucleic acid fragment and the antisense nucleic acid fragment comprise complementary regions capable of forming a double-stranded nucleic acid structure, and the double-stranded nucleic acid molecule promotes the expression of the aforementioned genes in cells via an RNA activation mechanism. The sense nucleic acid fragment and the antisense nucleic acid fragment of the small activating nucleic acid molecule can be present either on two different nucleic acid strands or on one nucleic acid strand. When the sense nucleic acid fragment and the antisense nucleic acid fragment are located on two different strands, at least one strand of the small activating nucleic acid molecule of the present invention has a 3' overhang of 0 to 6 nucleotides in length, such as a 3' overhang of 0, 1, 2, 3, 4, 5 or 6 nucleotides in length, and preferably, both strands have 3' overhangs of 2 or 3 nucleotides in length, wherein the nucleotide for the overhang is preferably dT. When the sense nucleic acid fragment and the antisense nucleic acid fragment are present on one nucleic acid strand, preferably the small activating nucleic acid molecule of the present invention is a hairpin single-stranded nucleic acid molecule, wherein the complementary regions of the sense nucleic acid fragment and the antisense nucleic acid fragment form a double-stranded nucleic acid structure. In the aforementioned small activating nucleic acid molecule, the sense nucleic acid fragment and the antisense nucleic acid fragment have 16 to 35 nucleotides (e.g., 16, 17, 18, 19, 20, 21, 22, 23, 24, 25, 26, 27, 28, 29, 30, 31, 32, 33, 34 or 35 nucleotides) in length.

In one embodiment, the sense strand of the small activating nucleic acid molecule for the AQP3 gene in the present invention has at least 75% (e.g., at least about 79%, about 80%, about 85%, about 90%, about 95%, about 99% or about 100%) homology or complementarity to any nucleotide sequence selected from SEQ ID NOs: 1-15, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 125-139; the sense strand of the small activating nucleic acid molecule for the AQP9 gene in the present invention has at least 75% (e.g., at least about 79%, about 80%, about 85%, about 90%, about 95%, about 99% or about 100%) homology or complementarity to any nucleotide sequence selected from SEQ ID NOs: 16-28, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 140-152; the sense strand of the small activating nucleic acid molecule for the ELN gene in the present invention has at least 75% (e.g., at least about 79%, about 80%, about 85%, about 90%, about 95%, about 99% or about 100%) homology or complementarity to any nucleotide sequence selected from SEQ ID NOs: 29-38, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 153-162; the sense strand of the small activating nucleic acid molecule for the COL1A1 gene in the present invention has at least 75% (e.g., at least about 79%, about 80%, about 85%, about 90%, about 95%, about 99% or about 100%) homology or complementarity to any nucleotide sequence selected from SEQ ID NOs: 39-46, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 163-170; the sense strand of the small activating nucleic acid molecule for the COL1A2 gene in the present invention has at least 75% (e.g., at least about 79%, about 80%, about 85%, about 90%, about 95%, about 99% or about 100%) homology or complementarity to any nucleotide sequence selected from SEQ ID NOs: 47-58, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 171-182; the sense strand of the small activating nucleic acid molecule for the COL3A1 gene in the present invention has at least 75% (e.g., at least about 79%, about 80%, about 85%, about 90%, about 95%, about 99% or about 100%) homology or complementarity to any nucleotide sequence selected from SEQ ID NOs: 59-69, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 183-193; the sense strand of the small activating nucleic acid molecule for the HAS1 gene in the present invention has at least 75% (e.g., at least about 79%, about 80%, about 85%, about 90%, about 95%, about 99% or about 100%) homology or complementarity to any nucleotide sequence selected from SEQ ID NOs: 70-78, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 194-202; the sense strand of the small activating nucleic acid molecule for the HAS2 gene in the present invention has at least 75% (e.g., at least about 79%, about 80%, about 85%, about 90%, about 95%, about 99% or about 100%) homology or complementarity to any nucleotide sequence selected from SEQ ID NOs: 79-92, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 203-216; the sense strand of the small activating nucleic acid molecule for the HAS3 gene in the present invention has at least 75% (e.g., at least about 79%, about 80%, about 85%, about 90%, about 95%, about 99% or about 100%) homology or complementarity to any nucleotide sequence selected from SEQ ID NOs: 93-109, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 217-233; and the sense strand of the small activating nucleic acid molecule for the MFAP2 gene in the present invention has at least 75% (e.g., at least about 79%, about 80%, about 85%, about 90%, about 95%, about 99% or about 100%) homology or complementarity to any nucleotide sequence selected from SEQ ID NOs: 110-124, and the antisense strand of the small activating nucleic acid molecule has at least 75% (e.g., at least about 79%, about 80%, about 85%, about 90%, about 95%, about 99% or about 100%) homology or complementarity to any nucleotide sequence selected from SEQ ID NOs: 234-248.

Specifically, the sense strand of the small activating nucleic acid molecule for the AQP3 gene in the present invention comprises or is selected from any of the nucleotide sequences set forth in SEQ ID NOs: 1-15 or consists of any nucleotide sequence selected from SEQ ID NOs: 1-15, and the antisense strand of the small activating nucleic acid molecule comprises or is selected from any of the nucleotide sequences set forth in SEQ ID NOs: 125-139 or consists of any nucleotide sequence selected from SEQ ID NOs: 125-139; the sense strand of the small activating nucleic acid molecule for the AQP9 gene in the present invention comprises or is selected from any of the nucleotide sequences set forth in SEQ ID NOs: 16-28 or consists of any nucleotide sequence selected from SEQ ID NOs: 16-28, and the antisense strand of the small activating nucleic acid molecule comprises or is selected from any of the nucleotide sequences set forth in SEQ ID NOs: 140-152 or consists of any nucleotide sequence selected from SEQ ID NOs: 140-152; the sense strand of the small activating nucleic acid molecule for the ELN gene in the present invention comprises or is selected from any of the nucleotide sequences set forth in SEQ ID NOs: 29-38 or consists of any nucleotide sequence selected from SEQ ID NOs: 29-38, and the antisense strand of the small activating nucleic acid molecule comprises or is selected from any of the nucleotide sequences set forth in SEQ ID NOs: 153-162 or consists of any nucleotide sequence selected from SEQ ID NOs: 153-162; the sense strand of the small activating nucleic acid molecule for the COL1A1 gene in the present invention comprises or is selected from any of the nucleotide sequences set forth in SEQ ID NOs: 39-46 or consists of any nucleotide sequence selected from SEQ ID NOs: 39-46, and the antisense strand of the small activating nucleic acid molecule comprises or is selected from any of the nucleotide sequences set forth in SEQ ID NOs: 163-170 or consists of any nucleotide sequence selected from SEQ ID NOs: 163-170; the sense strand of the small activating nucleic acid molecule for the COL1A2 gene in the present invention comprises or is selected from any of the nucleotide sequences set forth in SEQ ID NOs: 47-58 or consists of any nucleotide sequence selected from SEQ ID NOs: 47-58, and the antisense strand of the small activating nucleic acid molecule comprises or is selected from any of the nucleotide sequences set forth in SEQ ID NOs: 171-182 or consists of any nucleotide sequence selected from SEQ ID NOs: 171-182; the sense strand of the small activating nucleic acid molecule for the COL3A21 gene in the present invention comprises or is selected from any of the nucleotide sequences set forth in SEQ ID NOs: 59-69 or consists of any nucleotide sequence selected from SEQ ID NOs: 59-69, and the antisense strand of the small activating nucleic acid molecule comprises or is selected from any of the nucleotide sequences set forth in SEQ ID NOs: 183-193 or consists of any nucleotide sequence selected from SEQ ID NOs: 183-193; the sense strand of the small activating nucleic acid molecule for the HAS1 gene in the present invention comprises or is selected from any of the nucleotide sequences set forth in SEQ ID NOs: 70-78 or consists of any nucleotide sequence selected from SEQ ID NOs: 70-78, and the antisense strand of the small activating nucleic acid molecule comprises or is selected from any of the nucleotide sequences set forth in SEQ ID NOs: 194-202 or consists of any nucleotide sequence selected from SEQ ID NOs: 194-202; the sense strand of the small activating nucleic acid molecule for the HAS2 gene in the present invention comprises or is selected from any of the nucleotide sequences set forth in SEQ ID NOs: 79-92 or consists of any nucleotide sequence selected from SEQ ID NOs: 79-92, and the antisense strand of the small activating nucleic acid molecule comprises or is selected from any of the nucleotide sequences set forth in SEQ ID NOs: 203-216 or consists of any nucleotide sequence selected from SEQ ID NOs: 203-216; the sense strand of the small activating nucleic acid molecule for the HAS3 gene in the present invention comprises or is selected from any of the nucleotide sequences set forth in SEQ ID NOs: 93-109 or consists of any nucleotide sequence selected from SEQ ID NOs: 93-109, and the antisense strand of the small activating nucleic acid molecule comprises or is selected from any of the nucleotide sequences set forth in SEQ ID NOs: 217-233 or consists of any nucleotide sequence selected from SEQ ID NOs: 217-233; and the sense strand of the small activating nucleic acid molecule for the MFAP2 gene in the present invention comprises or is selected from any of the nucleotide sequences set forth in SEQ ID NOs: 110-124 or consists of any nucleotide sequence selected from SEQ ID NOs: 110-124, and the antisense strand of the small activating nucleic acid molecule comprises or is selected from any of the nucleotide sequences set forth in SEQ ID NOs: 234-248 or consists of any nucleotide sequence selected from SEQ ID NOs: 234-248.

All the nucleotides in the small activating nucleic acid molecule described herein may be natural or non-chemically modified nucleotides or at least one nucleotide may be a chemically modified nucleotide, and the chemical modification comprises, for example is selected from, one or a combination of the following modifications:

(1) modification of a phosphodiester bond of nucleotides in the nucleotide sequence of the small activating nucleic acid molecule;

(2) modification of 2'-OH of a ribose in the nucleotide sequence of the small activating nucleic acid molecule; and

(3) modification of a base in the nucleotide sequence of the small activating nucleic acid molecule;

(4) at least one nucleotide in the nucleotide sequence of the small activating nucleic acid molecule being a locked nucleic acid.

The chemical modification is well-known to those skilled in the art, and the modification of the phosphodiester bond refers to the modification of oxygen in the phosphodiester bond, including phosphorothioate modification and boranophosphate modification. Both modifications can stabilize an saRNA structure and maintain high specificity and high affinity for base pairing.

The ribose modification refers to the modification of 2'-OH in pentose of a nucleotide, i.e., the introduction of some substituents into hydroxyl positions of the ribose, such as 2'-fluoro modification, 2'-oxymethyl modification, 2'-oxyethylidene methoxy modification, 2,4'-dinitrophenol modification, locked nucleic acid (LNA), 2'-amino modification and 2'-deoxy modification.

The base modification refers to the modification of the base of a nucleotide, such as 5'-bromouracil modification, 5'-iodouracil modification, N-methyluracil modification and 2,6-diaminopurine modification.

These modifications can increase the bioavailability of the small activating nucleic acid molecule, improve affinity to a target sequence and enhance resistance to nuclease hydrolysis in a cell.

In addition, in order to promote the access of the small activating nucleic acid molecule into a cell, on the basis of the aforementioned modifications, a lipophilic group (such as cholesterol) can be introduced into the terminus of the sense strand or antisense strand of the small activating nucleic acid molecule to facilitate the interaction with the gene promoter region in the cell nucleus through cell membrane and nuclear membrane composed of lipid bilayers.

After contacting a cell, the small activating nucleic acid molecule provided by the present invention can effectively activate or upregulate the expression of the gene in a cell, preferably upregulate the expression by at least 10%.

The small activating nucleic acid molecule of the present invention may be operably linked to a vector, and preferably, the vector includes a plasmid vector, a lentiviral vector, an adenovirus vector or an adeno-associated virus vector.

One aspect of the present invention provides nucleic acids each encoding the small activating nucleic acid molecules disclosed herein targeting the promoter region of AQP3, AQP9, ELN, COL1A1, COL1A2, COL3A1, HAS1, HAS2, HAS3, or MFAP2 genes. In one embodiment, the small activating nucleic acid molecule disclosed herein is a small activating RNA (saRNA) molecule. In one embodiment, the nucleic acid is a DNA molecule.

One aspect of the present invention provides a cell which comprises one or more of the small activating nucleic acid molecules disclosed herein target the promoter region of the AQP3, AQP9, ELN, COL1A1, COL1A2, COL3A1, HAS1, HAS2, HAS3, or MFAP2 genes or one or more of the nucleic acids respectively encoding the small activating nucleic acid molecules disclosed herein targeting the promoter region of the AQP3, AQP9, ELN, COL1A1, COL1A2, COL3A1, HAS1, HAS2, HAS3, or MFAP2 genes. In one embodiment, the cell is a mammalian cell, preferably a human cell. The aforementioned cell may be *in vitro,* such as a cell line or a cell strain, or may exist in a mammalian body, such as a human body.

Another aspect of the present invention provides a composition or a formulation, which comprises an agonist interfering with the expression and function of at least one gene selected from human AQP3, AQP9, ELN, COL1A1, COL1A2, COL3A1, HAS1, HAS2, HAS3, and MFAP2 genes. In one embodiment, the agonist contained in the composition or formulation disclosed herein may be one or more of the aforementioned small activating nucleic acid molecules or one or more of the nucleic acids respectively encoding the small activating nucleic acid molecules disclosed herein targeting the promoter region of the AQP3, AQP9, ELN, COL1A1, COL1A2, COL3A1, HAS1, HAS2, HAS3, or MFAP2 genes. In another embodiment, the composition or formulation disclosed herein may comprise a carrier, such as a carrier acceptable to cosmetics, skin-care, and beauty products. In a further embodiment, the carrier acceptable to cosmetics, skin-care, and beauty products may include, for example, an aqueous carrier, a liposome, a macromolecular polymer, or a polypeptide. In a more further embodiment, the aqueous carrier may include, for example, RNase-free water.

Another aspect of the present invention provides a composition (such as a pharmaceutical composition) or a formulation, which comprises the aforementioned small activating nucleic acid molecules or the nucleic acids respectively encoding the small activating nucleic acid molecules disclosed herein targeting the promoter region of the AQP3, AQP9, ELN, COL1A1, COL1A2, COL3A1, HAS1, HAS2, HAS3, or MFAP2 genes and (optionally) a carrier. In one embodiment, the carrier may include a carrier acceptable to cosmetics, skin-care, and beauty products. In one embodiment, the carrier acceptable to cosmetics, skin-care, and beauty products may include or be selected from an aqueous carrier, a liposome, a macromolecular polymer, or a polypeptide. In one embodiment, the aqueous carrier disclosed herein may include, for example, RNase-free water or RNase-free buffer.

The composition or formulation disclosed herein may comprise the abovementioned small activating nucleic acid molecules or the nucleic acid encoding the small activating nucleic acid molecules disclosed herein which is 1 nM to 150 nM (e.g., 1 nM to 100 nM, e.g., 50 nM, e.g., 1 nM to 50 nM, e.g., 1 nM to 10 nM, 1 nM to 20 nM, 1 nM to 30 nM, or 1 nM to 40 nM, e.g., 10 nM to 100 nM, 10 nM to 50 nM, 20 nM to 30 nM, 20 nM to 40 nM, 20 nM to 50 nM, 20 nM to 60 nM, 20 nM to 70 nM, 20 nM to 80 nM, 20 nM to 90 nM, or 20 nM to 100 nM).

The composition or formulation disclosed herein may comprise any one or a combination of any two, three, four, five, six, seven, eight, nine, or ten of the small activating nucleic acid molecules disclosed herein targeting the promoter region of the AQP3, AQP9, ELN, COL1A1, COL1A2, COL3A1, HAS1, HAS2, HAS3, or MFAP2 genes. For example, the composition disclosed herein may comprise a combination of the small activating nucleic acid molecule targeting the promoter region of AQP3 and any one, two, three, four, five, six, seven, eight, or nine of the small activating nucleic acid molecules targeting the promoter region of the AQP9, ELN, COL1A1, COL1A2, COL3A1, HAS1, HAS2, HAS3, or MFAP2 genes; a combination of the small activating nucleic acid molecule targeting the promoter region of ELN and any one, two, three, four, five, six, seven, eight, or nine of the small activating nucleic acid molecules targeting the promoter region of the AQP3, AQP9, COL1A1, COL1A2, COL3A1, HAS1, HAS2, HAS3, or MFAP2 genes; a combination of the small activating nucleic acid molecule targeting the promoter region of AQP9 and any one, two, three, four, five, six, seven, eight, or nine of the small activating nucleic acid molecules targeting the promoter regions of the AQP3, ELN, COL1A1, COL1A2, COL3A1, HAS1, HAS2, HAS3, or MFAP2 genes; a combination of the small activating nucleic acid molecule targeting the promoter region of COL1A1 and any one, two, three, four, five, six, seven, eight, or nine of the small activating nucleic acid molecules targeting the promoter region of the AQP3, AQP9, ELN, COL1A2, COL3A1, HAS1, HAS2, HAS3, or MFAP2 genes; or a combination of the small activating nucleic acid molecule targeting the promoter region of COL1A2 and any one, two, three, four, five, six, seven, eight, or nine of the small activating nucleic acid molecules targeting the promoter region of the AQP3, AQP9, ELN, COL1A1, COL3A1, HAS1, HAS2, HAS3, or MFAP2 genes, *etc.*

Specifically, the composition or formulation disclosed herein may comprise, for example, the small activating nucleic acid molecule (the sense strand comprises or is selected from any of the nucleotide sequences set forth in SEQ ID NOs: 1-15, and the antisense strand comprises or is selected from any of the nucleotide sequences set forth in SEQ ID NOs: 125-139) targeting AQP3 gene, and/or the small activating nucleic acid molecule (the sense strand comprises or is selected from any of the nucleotide sequences set forth in SEQ ID NOs: 16-28, and the antisense strand comprises or is selected from any of the nucleotide sequences set forth in SEQ ID NOs: 140-152) targeting AQP9 gene, and/or the small activating nucleic acid molecule (the sense strand comprises or is selected from any of the nucleotide sequences set forth in SEQ ID NOs: 29-38, and the antisense strand comprises or is selected from any of the nucleotide sequences set forth in SEQ ID NOs: 153-162) targeting ELN gene, and/or the small activating nucleic acid molecule (the sense strand comprises or is selected from any of the nucleotide sequences set forth in SEQ ID NOs: 39-46, and the antisense strand comprises or is selected from any of the nucleotide sequences set forth in SEQ ID NOs: 163-170) targeting COL1A1 gene, and/or the small activating nucleic acid molecule (the sense strand comprises or is selected from any of the nucleotide sequences set forth in SEQ ID NOs: 47-58, and the antisense strand comprises or is selected from any of the nucleotide sequences set forth in SEQ ID NOs: 171-182) targeting COL1A2 gene, and/or the small activating nucleic acid molecule (the sense strand comprises or is selected from any of the nucleotide sequences set forth in SEQ ID NOs: 59-69, and the antisense strand comprises or is selected from any of the nucleotide sequences set forth in SEQ ID NOs: 183-193) targeting COL3A1 gene, and/or the small activating nucleic acid molecule (the sense strand comprises or is selected from any of the nucleotide sequences set forth in SEQ ID NOs: 70-78, and the antisense strand comprises or is selected from any of the nucleotide sequences set forth in SEQ ID NOs: 194-202) targeting HAS1 gene, and/or the small activating nucleic acid molecule (the sense strand comprises or is selected from any of the nucleotide sequences set forth in SEQ ID NOs: 79-92, and the antisense strand comprises or is selected from any of the nucleotide sequences set forth in SEQ ID NOs: 203-216) targeting HAS2 gene, and/or the small activating nucleic acid molecule (the sense strand comprises or is selected from any of the nucleotide sequences set forth in SEQ ID NOs: 93-109, and the antisense strand comprises or is selected from any of the nucleotide sequences set forth in SEQ ID NOs: 217-233) targeting HAS3 gene, and/or the small activating nucleic acid molecule (the sense strand comprises or is selected from any of the nucleotide sequences set forth in SEQ ID NOs: 110-124, and the antisense strand comprises or is selected from any of the nucleotide sequences set forth in SEQ ID NOs: 234-248) targeting MFAP2 gene.

The present invention provides the use of the agonist interfering with the expression and function of at least one gene selected from the human AQP3, AQP9, ELN, COL1A1, COL1A2, COL3A1, HAS1, HAS2, HAS3 and MFAP2 genes in preparing a skin-care composition. In one embodiment, the agonist may comprise one or more of the aforementioned small activating nucleic acid molecules or one or more of the nucleic acids respectively encoding the small activating nucleic acid molecules disclosed herein targeting the promoter region of the AQP3, AQP9, ELN, COL1A1, COL1A2, COL3A1, HAS1, HAS2, HAS3, or MFAP2 genes. Another aspect of the present invention relates to the use of the aforementioned small activating nucleic acid molecule, the nucleic acid encoding the small activating nucleic acid molecule disclosed herein, or the composition comprising the aforementioned small activating nucleic acid molecule or the nucleic acid encoding the small activating nucleic acid molecule disclosed herein in preparing a reagent or a formulation for activating/up-regulating the expression of genes (such as AQP3, AQP9, ELN, COL1A1, COL1A2, COL3A1, HAS1, HAS2, HAS3, and/or MFAP2 genes) related to skin conditions in a cell.

The present invention further relates to a method for activating/up-regulating the expression of genes related to skin conditions (such as the AQP3, AQP9, ELN, COL1A1, COL1A2, COL3A1, HAS1, HAS2, HAS3, and/or MFAP2 genes) in a cell, which comprises administering one or more of the aforementioned small activating nucleic acid molecules, one or more of the nucleic acids encoding the small activating nucleic acid molecules disclosed herein, or a composition comprising one or more of the aforementioned small activating nucleic acid molecules or one or more of the nucleic acids encoding the small activating nucleic acid molecules disclosed herein to the cell.

The aforementioned small activating nucleic acid molecule, the nucleic acid encoding the small activating nucleic acid molecule disclosed herein, or the composition comprising the aforementioned small activating nucleic acid molecule or the nucleic acid encoding the small activating nucleic acid molecule disclosed herein may be directly introduced into a cell, or may be produced in the cell after a nucleotide sequence encoding the small activating nucleic acid molecule is introduced into the cell. The cell is preferably a mammalian cell, more preferably a human cell. The aforementioned cell may be *in vitro,* such as a cell line or a cell strain, or may exist in a mammalian body, such as a human body. The human body is a person with skin conditions such as sagging, aging, fine lines, dry lines, wrinkles, and lack of moisture, and the small activating nucleic acid molecule, the nucleic acid encoding the small activating nucleic acid molecule disclosed herein, or the composition comprising the aforementioned small activating nucleic acid molecule or the nucleic acid encoding the small activating nucleic acid molecule disclosed herein is applied in a sufficient amount to improve the skin conditions.

Another aspect of the present invention provides an isolated acting site of a small activating nucleic acid molecule for the aforementioned genes (such as one or more of the AQP3, AQP9, ELN, COL1A1, COL1A2, COL3A1, HAS1, HAS2, HAS3, and/or MFAP2 genes), which has any continuous sequence of 16 to 35 nucleotides on the promoter region of any of the aforementioned genes (such as the AQP3, AQP9, ELN, COL1A1, COL1A2, COL3A1, HAS1, HAS2, HAS3, and/or MFAP2 genes). Preferably, for AQP3 gene, the acting site of the small activating nucleic acid molecule (such as the small activating RNA (saRNA) molecule) thereof comprises or is selected from any of the nucleotide sequences set forth in SEQ ID NOs: 249-263; for AQP9 gene, the acting site of the small activating nucleic acid molecule (such as the small activating RNA (saRNA) molecule) thereof comprises or is selected from any of the nucleotide sequences set forth in SEQ ID NOs: 264-276; for ELN gene, the acting site of the small activating nucleic acid molecule (such as the small activating RNA (saRNA) molecule) thereof comprises or is selected from any of the nucleotide sequences set forth in SEQ ID NOs: 277-286; for COL1A1 gene, the acting site of the small activating nucleic acid molecule (such as the small activating RNA (saRNA) molecule) thereof comprises or is selected from any of the nucleotide sequences set forth in SEQ ID NOs: 287-294; for COL1A2 gene, the acting site of the small activating nucleic acid molecule (such as the small activating RNA (saRNA) molecule) thereof comprises or is selected from any of the nucleotide sequences set forth in SEQ ID NOs: 295-306; for COL3A1 gene, the acting site of the small activating nucleic acid molecule (such as the small activating RNA (saRNA) molecule) thereof comprises or is selected from any of the nucleotide sequences set forth in SEQ ID NOs: 307-317; for HAS1 gene, the acting site of the small activating nucleic acid molecule (such as the small activating RNA (saRNA) molecule) thereof comprises or is selected from any of the nucleotide sequences set forth in SEQ ID NOs: 318-326; for HAS2 gene, the acting site of the small activating nucleic acid molecule (such as the small activating RNA (saRNA) molecule) thereof comprises or is selected from any of the nucleotide sequences set forth in SEQ ID NOs: 327-340; for HAS3 gene, the acting site of the small activating nucleic acid molecule (such as the small activating RNA (saRNA) molecule) thereof comprises or is selected from any of the nucleotide sequences set forth in SEQ ID NOs: 341-357; and for MFAP2 gene, the acting site of the small activating nucleic acid molecule (such as the small activating RNA (saRNA) molecule) thereof comprises or is selected from any of the nucleotide sequences set forth in SEQ ID NOs: 358-372.

In one aspect, the present invention provides a method for caring the skin, which comprises administering one or more of the small activating nucleic acid molecules disclosed herein, one or more of the nucleic acids encoding the small activating nucleic acid molecules disclosed herein, or a composition comprising one or more of the aforementioned small activating nucleic acid molecules or one or more of the nucleic acids encoding the small activating nucleic acid molecules disclosed herein to an individual. The individual may be a mammal, such as a human. In one embodiment, after the method for caring the skin disclosed herein is applied to care the skin, there are increased skin elasticity, reduced or eliminated wrinkles, increased moisture content of the skin, and tightened skin in the individual.

In another aspect, the present invention further provides a method for improving skin conditions, which comprises administering one or more of the small activating nucleic acid molecules disclosed herein, one or more of the nucleic acids encoding the small activating nucleic acid molecules disclosed herein, or the composition comprising one or more of the small activating nucleic acid molecules disclosed herein or one or more of the nucleic acids encoding the small activating nucleic acid molecules disclosed herein to an individual. The individual may be a mammal, such as a human. In one embodiment, the skin conditions include sagging, aging, fine lines, dry lines, wrinkles, lack of moisture and other conditions. In one embodiment, improving skin conditions includes, but is not limited to, increasing or restoring skin elasticity, improving skin sagging, increasing the moisture content of the skin, reducing or eliminating skin wrinkles, preventing the appearance of fine lines and dry lines, *etc.*

In yet another aspect, the present invention further provides a method for activating/up-regulating the expression of one or more of the human AQP3, AQP9, ELN, COL1A1, COL1A2, COL3A1, HAS1, HAS2, HAS3, and MFAP2 genes in a cell, which comprises administering one or more of the small activating nucleic acid molecules disclosed herein, one or more of the nucleic acids encoding the small activating nucleic acid molecules disclosed herein, or the composition comprising one or more of the aforementioned small activating nucleic acid molecules or one or more of the nucleic acids encoding the small activating nucleic acid molecules disclosed herein to the cell.

The method of the present invention comprises administering any one, two, three, four, five, six, seven, eight, nine, or ten of the small activating nucleic acid molecules disclosed herein targeting the promoter region of the AQP3, AQP9, ELN, COL1A1, COL1A2, COL3A1, HAS1, HAS2, HAS3, or MFAP2 genes to the cell. For example, the method of the present invention may comprise administering a combination of small activating nucleic acid molecules targeting the promoter region of AQP3 and any one, two, three, four, five, six, seven, eight, or nine of small activating nucleic acid molecules targeting the promoter region of the AQP9, ELN, COL1A1, COL1A2, COL3A1, HAS1, HAS2, HAS3, or MFAP2 genes; a combination of small activating nucleic acid molecules targeting the promoter region of ELN and any one, two, three, four, five, six, seven, eight, or nine of small activating nucleic acid molecules targeting the promoter region of the AQP3, AQP9, COL1A1, COL1A2, COL3A1, HAS1, HAS2, HAS3, or MFAP2 genes; a combination of small activating nucleic acid molecules targeting the promoter region of AQP9 and any one, two, three, four, five, six, seven, eight, or nine of small activating nucleic acid molecules targeting the promoter region of the AQP3, ELN, COL1A1, COL1A2, COL3A1, HAS1, HAS2, HAS3, or MFAP2 genes; a combination of small activating nucleic acid molecules targeting the promoter region of COL1A1 and any one, two, three, four, five, six, seven, eight, or nine of small activating nucleic acid molecules targeting the promoter region of the AQP3, AQP9, ELN, COL1A2, COL3A1, HAS1, HAS2, HAS3, or MFAP2 genes; a combination of small activating nucleic acid molecules targeting the promoter region of COL1A2 and any one, two, three, four, five, six, seven, eight, or nine of small activating nucleic acid molecules targeting the promoter region of the AQP3, AQP9, ELN, COL1A1, COL3A1, HAS1, HAS2, HAS3, or MFAP2 genes, or the like.

The aforementioned method of the present invention may comprise directly introducing one or more of the small activating nucleic acid molecules disclosed herein, one or more of the nucleic acids encoding the small activating nucleic acid molecules disclosed herein, or the composition comprising one or more of the aforementioned small activating nucleic acid molecules or one or more of the nucleic acids encoding the small activating nucleic acid molecules disclosed herein into a cell.

Specifically, the method of the present invention may comprise, for example, directly introducing one or more of the following into a cell: small activating nucleic acid molecules (the sense strand comprises or is selected from any of the nucleotide sequences set forth in SEQ ID NOs: 1-15, and the antisense strand comprises or is selected from any of the nucleotide sequences set forth in SEQ ID NOs: 125-139) targeting AQP3 gene, and/or small activating nucleic acid molecules (the sense strand comprises or is selected from any of the nucleotide sequences set forth in SEQ ID NOs: 16-28, and the antisense strand comprises or is selected from any of the nucleotide sequences set forth in SEQ ID NOs: 140-152) targeting AQP9 gene, and/or small activating nucleic acid molecules (the sense strand comprises or is selected from any of the nucleotide sequences set forth in SEQ ID NOs: 29-38, and the antisense strand comprises or is selected from any of the nucleotide sequences set forth in SEQ ID NOs: 153-162) targeting ELN gene, and/or small activating nucleic acid molecules (the sense strand comprises or is selected from any of the nucleotide sequences set forth in SEQ ID NOs: 39-46, and the antisense strand comprises or is selected from any of the nucleotide sequences set forth in SEQ ID NOs: 163-170) targeting COL1A1 gene, and/or small activating nucleic acid molecules (the sense strand comprises or is selected from any of the nucleotide sequences set forth in SEQ ID NOs: 47-58, and the antisense strand comprises or is selected from any of the nucleotide sequences set forth in SEQ ID NOs: 171-182) targeting COL1A2 gene, and/or small activating nucleic acid molecules (the sense strand comprises or is selected from any of the nucleotide sequences set forth in SEQ ID NOs: 59-69, and the antisense strand comprises or is selected from any of the nucleotide sequences set forth in SEQ ID NOs: 183-193) targeting COL3A1 gene, and/or small activating nucleic acid molecules (the sense strand comprises or is selected from any of the nucleotide sequences set forth in SEQ ID NOs: 70-78, and the antisense strand comprises or is selected from any of the nucleotide sequences set forth in SEQ ID NOs: 194-202) targeting HAS1 gene, and/or small activating nucleic acid molecules (the sense strand comprises or is selected from any of the nucleotide sequences set forth in SEQ ID NOs: 79-92, and the antisense strand comprises or is selected from any of the nucleotide sequences set forth in SEQ ID NOs: 203-216) targeting HAS2 gene, and/or small activating nucleic acid molecules (the sense strand comprises or is selected from any of the nucleotide sequences set forth in SEQ ID NOs: 93-109, and the antisense strand comprises or is selected from any of the nucleotide sequences set forth in SEQ ID NOs: 217-233) targeting HAS3 gene, and/or small activating nucleic acid molecules (the sense strand comprises or is selected from any of the nucleotide sequences set forth in SEQ ID NOs: 110-124, and the antisense strand comprises or is selected from any of the nucleotide sequences set forth in SEQ ID NOs: 234-248) targeting MFAP2 gene.

As described above, the term "one or more of the aforementioned small activating nucleic acid molecules" refers to any one, two, three, four, five, six, seven, eight, nine, or ten of the small activating nucleic acid molecules disclosed herein targeting the promoter region of the AQP3, AQP9, ELN, COL1A1, COL1A2, COL3A1, HAS1, HAS2, HAS3, or MFAP2 genes. For example, a combination of small activating nucleic acid molecules targeting the promoter region of AQP3 and any one, two, three, four, five, six, seven, eight, or nine of small activating nucleic acid molecules targeting the promoter region of the AQP9, ELN, COL1A1, COL1A2, COL3A1, HAS1, HAS2, HAS3, or MFAP2 genes; a combination of small activating nucleic acid molecules targeting the promoter region of ELN and any one, two, three, four, five, six, seven, eight, or nine of small activating nucleic acid molecules targeting the promoter region of the AQP3, AQP9, COL1A1, COL1A2, COL3A1, HAS1, HAS2, HAS3, or MFAP2 genes; a combination of small activating nucleic acid molecules targeting the promoter region of AQP9 and any one, two, three, four, five, six, seven, eight, or nine of the small activating nucleic acid molecules targeting the promoter region of the AQP3, ELN, COL1A1, COL1A2, COL3A1, HAS1, HAS2, HAS3, or MFAP2 genes; a combination of small activating nucleic acid molecules targeting the promoter region of COL1A1 and any one, two, three, four, five, six, seven, eight, or nine of the small activating nucleic acid molecules targeting the promoter region of the AQP3, AQP9, ELN, COL1A2, COL3A1, HAS1, HAS2, HAS3, or MFAP2 genes; a combination of small activating nucleic acid molecules targeting the promoter region of COL1A2 and any one, two, three, four, five, six, seven, eight, or nine of the small activating nucleic acid molecules targeting the promoter regions of the AQP3, AQP9, ELN, COL1A1, COL3A1, HAS1, HAS2, HAS3, or MFAP2 genes, or the like.

Specifically, the term "one or more of the aforementioned small activating nucleic acid molecules" refers to one or a combination of more of the following: small activating nucleic acid molecules (the sense strand comprises or is selected from any of the nucleotide sequences set forth in SEQ ID NOs: 1-15, and the antisense strand comprises or is selected from any of the nucleotide sequences set forth in SEQ ID NOs: 125-139) targeting AQP3 gene, small activating nucleic acid molecules (the sense strand comprises or is selected from any of the nucleotide sequences set forth in SEQ ID NOs: 16-28, and the antisense strand comprises or is selected from any of the nucleotide sequences set forth in SEQ ID NOs: 140-152) targeting AQP9 gene, small activating nucleic acid molecules (the sense strand comprises or is selected from any of the nucleotide sequences set forth in SEQ ID NOs: 29-38, and the antisense strand comprises or is selected from any of the nucleotide sequences set forth in SEQ ID NOs: 153-162) targeting ELN gene, small activating nucleic acid molecules (the sense strand comprises or is selected from any of the nucleotide sequences set forth in SEQ ID NOs: 39-46, and the antisense strand comprises or is selected from any of the nucleotide sequences set forth in SEQ ID NOs: 163-170) targeting COL1A1 gene, small activating nucleic acid molecules (the sense strand comprises or is selected from any of the nucleotide sequences set forth in SEQ ID NOs: 47-58, and the antisense strand comprises or is selected from any of the nucleotide sequences set forth in SEQ ID NOs: 171-182) targeting COL1A2 gene, small activating nucleic acid molecules (the sense strand comprises or is selected from any of the nucleotide sequences set forth in SEQ ID NOs: 59-69, and the antisense strand comprises or is selected from any of the nucleotide sequences set forth in SEQ ID NOs: 183-193) targeting COL3A1 gene, small activating nucleic acid molecules (the sense strand comprises or is selected from any of the nucleotide sequences set forth in SEQ ID NOs: 70-78, and the antisense strand comprises or is selected from any of the nucleotide sequences set forth in SEQ ID NOs: 194-202) targeting HAS1 gene, small activating nucleic acid molecules (the sense strand comprises or is selected from any of the nucleotide sequences set forth in SEQ ID NOs: 79-92, and the antisense strand comprises or is selected from any of the nucleotide sequences set forth in SEQ ID NOs: 203-216) targeting HAS2 gene, small activating nucleic acid molecules (the sense strand comprises or is selected from any of the nucleotide sequences set forth in SEQ ID NOs: 93-109, and the antisense strand comprises or is selected from any of the nucleotide sequences set forth in SEQ ID NOs: 217-233) targeting HAS3 gene and small activating nucleic acid molecules (the sense strand comprises or is selected from any of the nucleotide sequences set forth in SEQ ID NOs: 110-124, and the antisense strand comprises or is selected from any of the nucleotide sequences set forth in SEQ ID NOs: 234-248) targeting MFAP2 gene.

Likewise, the term "one or more of the nucleic acids encoding the small activating nucleic acid molecules described herein" has the similar meaning.

In one embodiment, in the aforementioned method, the small activating nucleic acid molecule disclosed herein is produced in a cell after a nucleotide sequence encoding the small activating nucleic acid molecule is introduced into the cell. In the method of the present invention, the cell may be a human cell.

## Advantages of the Present Invention

The small activating nucleic acid molecules for activating/up-regulating gene expression provided herein, such as the small activating RNA (saRNA) molecules, can efficiently and specifically up-regulate the expression of endogenous genes (such as one or more of the AQP3, AQP9, ELN, COL1A1, COL1A2, COL3A1, HAS1, HAS2, HAS3 and/or MFAP2 genes) with low toxic side effects, and can restore or increase skin elasticity, ameliorate skin sagging, increase the moisture content in skin, reduce or eliminate skin wrinkles, and prevent the appearance of fine lines and dry lines, thus can be used to prepare formulations or products for improving skin conditions and/or caring the skin, such as skin care products.

## DRAWINGS

**FIG. 1** shows that saRNAs targeting AQP3 activate AQP3 mRNA expression in normal human epidermal keratinocytes. The normal human epidermal keratinocytes (NHEKs) were transfected with the shown saRNAs (final concentration: 10 nM). 72 h later, RNAs were extracted from the collected cells using a Qiagen RNeasy kit. After reverse transcription, qPCR amplification of AQP3 was performed using a 7500FAST real-time PCR system. At the same time, HPRT1 and GAPDH genes were amplified with their means serving as internal references. Y axis represents the change value of AQP3 mRNA expression caused by each saRNA-treated sample relative to control treatment (Mock) after correction with an internal reference gene (mean ± SEM, n=2). dsControl and siAQP3 are sequence-independent double-stranded RNA control and small interfering RNA control, respectively. In the figure, the numbers on the bar graph represent change multiples of AQP3 mRNA expression relative to the control treatment (Mock) (mean ± SEM, n=2).

**FIG. 2** shows that saRNAs targeting AQP3 activate AQP3 protein expression in normal human epidermal keratinocytes. The NHEK cells were transfected with the shown saRNAs (final concentration: 10 nM). 72 h later, the amount of AQP3 protein in the collected cells was analyzed by Western blotting, and the tubulin was detected as an internal reference for the amount of protein loaded. The control treatment (Mock) is a blank treatment control, and dsControl and siAQP3 are sequence-independent double-stranded RNA control and small interfering RNA control, respectively. The intensity of Western blot bands was analyzed using Image Lab (BIO-RAD, Chemistry Doc$^{tm}$ MP imaging system) and represented as change multiples relative to the control treatment (Mock) in the drawing.

**FIG. 3** shows that saRNAs targeting AQP9 activate AQP9 mRNA expression in human NHEK cells. The NHEK cells were transfected for 72 h with the saRNAs (50 nM) shown in the drawing, and the mRNA expression of AQP9 gene in the transfected cells was analyzed using RT-qPCR. At the same time, GAPDH was amplified as an internal reference gene. The bar graph represents change multiples of AQP9 mRNA expression relative to the control treatment (Mock) (mean ± SEM, n=2). siAQP9 is an siRNA targeting AQP9.

**FIG. 4** shows that ELN saRNAs activate ELN mRNA expression in human fibroblasts. The normal human primary fibroblasts (NHDF cells and Hs27 cells) were transfected for 72 h with the saRNAs (50 nM) shown in the drawing. The mRNA expression of ELN gene was analyzed using RT-qPCR. At the same time, GAPDH or/and HPRT1 were amplified as internal reference genes. **FIG. 4A** shows the results of the preliminary screening for the ELN saRNAs in the Hs27 cells. In the figure, the numbers on the bar graph represent change multiples of ELN mRNA expression relative to control treatment (Mock). **FIG. 4B** and **FIG. 4C** show the changes of ELN mRNA expression caused by transfection of the shown saRNAs into the NHDF cells and the Hs27 cells. ELN/HPRT1 and ELN/GAPDH are the results of correcting the expression values of ELN with internal reference genes HPRT1 and GAPDH, respectively, (mean ± SEM, n=2). * , $P < 0.05$, ***, $P < 0.0003$, ****, $P < 0.0001$

**FIG. 5** shows that ELN saRNAs activate ELN protein expression in human fibroblasts. The Hs27 cells were transfected for 72 h with the saRNAs (50 nM) shown in the drawing. ELN protein expression was analyzed using Western blotting. At the same time, the level of GAPDH protein was detected as an internal reference. The control treatment (Mock) is a blank treatment control, and dsControl is a sequence-independent double-stranded RNA control. The intensity of Western blot bands was analyzed using Image Lab (BIO-RAD, Chemistry Doc$^{tm}$ MP imaging system) and represented as change multiples relative to the control treatment (Mock) in the drawing. **FIG. 5A** shows the Western blotting results of the ELN saRNAs in the Hs27 cells. **FIG. 5B** shows the relative change multiples of ELN protein expression caused by transfection of the shown saRNAs into the Hs27 cells.

**FIG. 6** shows that saRNAs targeting COL1A1 activate COL1A1 mRNA expression in human fibroblasts Hs27. The Hs27 cells were transfected for 72 h with the saRNAs (50 nM) shown in the drawing. The mRNA expression of the COL1A1 gene was analyzed using RT-qPCR. At the same time, GAPDH was amplified as an internal reference gene. In the figure, the numbers on the bar graph represent change multiples of COL1A1 mRNA expression relative to the control treatment (Mock) (mean ± SEM, n=2).

**FIG. 7** shows that saRNAs targeting COL1A2 activate COL1A2 mRNA expression in human fibroblasts. The human fibroblasts (NHDF cells) were transfected for 72 h with the saRNAs (50 nM) shown in the drawing. The mRNA expression of ELN gene was analyzed using RT-qPCR. At the same time, GAPDH was amplified as an internal reference gene. In the figure, the numbers on the bar graph represent change multiples of COL1A2 mRNA expression relative to the control treatment (Mock) (mean ± SEM, n=2).

**FIG. 8** shows that saRNAs targeting COL3A1 activate COL3A1 mRNA expression in human fibroblasts. The Hs27 cells were transfected for 72 h with the saRNAs (10 nM) shown in the drawing, and the mRNA expression of COL3A1 gene in the transfected cells was analyzed using RT-qPCR. At the same time, GAPDH was amplified as an internal reference gene. The bar graph represents change multiples of COL3A1 mRNA expression relative to the control treatment (Mock) (mean ± SEM, n=2). siCOL3A1 is an siRNA for COL3A1.

**FIG. 9** shows that saRNAs targeting HAS1 activate HAS1 mRNA expression in human fibroblasts. The Hs27 cells were transfected for 72 h with the saRNAs (10 nM) shown in the drawing, and the mRNA expression of HAS1 gene in the transfected cells was analyzed using RT-qPCR. At the same time, GAPDH was amplified as an internal reference gene. The bar graph represents change multiples of HAS1 mRNA expression relative to the control treatment (Mock) (mean ± SEM, n=2). siHAS1 is an siRNA for HAS1.

**FIG. 10** shows that saRNAs targeting HAS2 activate HAS2 mRNA expression in human fibroblasts. The Hs27 cells were transfected for 72 h with the saRNAs (10 nM) shown in the drawing, and the mRNA expression of HAS2 gene in the transfected cells was analyzed using RT-qPCR. At the same time, GAPDH was amplified as an internal reference gene. The bar graph represents change multiples of HAS2 mRNA expression relative to the control treatment (Mock) (mean ± SEM, n=2). siHAS2 was an siRNA for HAS2.

**FIG. 11** shows that HAS3 saRNAs activate HAS3 mRNA expression in human fibroblasts. The Hs27 cells were transfected for 72 h with the saRNAs (10 nM) shown in the drawing, and the mRNA expression of HAS3 gene in the transfected cells was analyzed using RT-qPCR. At the same time, GAPDH was amplified as an internal reference gene. The bar graph represents change multiples of HAS3 mRNA expression relative to the control treatment (Mock) (mean ± SEM, n=2). siHAS3 was an siRNA for HAS3.

**FIG. 12** shows that the saRNAs targeting HAS1, HAS2 and HAS3 promote the production of hyaluronic acid in human fibroblasts Hs27. Hs27 cells were transfected for 72 h with the saRNAs at the final concentration of 50 nM, the media of the cells were collected, and the contents of hyaluronic acid in the media were detected by ELISA. The drawing shows the changes of hyaluronic acid contents relative to the control (Mock) treatment (the mean of two study results ± SD). The dashed line indicates values of the Mock treatment.

**FIG. 13** shows that saRNAs targeting MFAP2 activate MFAP2 mRNA expression in human fibroblasts. The Hs27 cells were transfected for 72 h with the saRNAs (10 nM) shown in the drawing, and the mRNA expression of MFAP2 gene in the transfected cells was analyzed using RT-qPCR. At the same time, GAPDH was amplified as an internal reference gene. The bar graph represents change multiples of MFAP2 mRNA expression relative to the control treatment (Mock) (mean ± SEM, n=2). siMFAP2 was an siRNA for MFAP2.

## DETAILED DESCRIPTION

In the present invention, the related terms are defined as follows:
The term "complementarity" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity includes complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule without "mispairing". "Incomplete complementarity" means that not all the nucleotide units of the two strands are bonded with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95% or 99% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotides in deoxyribonucleic acid (DNA) or ribonucleic acid (RNA)). In the present invention, an oligonucleotide strand may have any of 16 to 35 nucleotides in length.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogeneous promoter adjacent to an endogenous coding sequence is introduced into a host cell). For example, the term "gene" comprises a nucleic acid sequence consisting of exons and introns. Protein-coding sequences are, for example, sequences contained within exons in an open reading frame between an initiation codon and a termination codon, and as used herein, "gene" can comprise such as a gene regulatory sequence, such as a promoter, an enhancer, and all other sequences known in the art for controlling the transcription, expression or activity of another gene, no matter whether the gene comprises a coding sequence or a non-coding sequence. In one case, for example, "gene" can be used to describe a functional nucleic acid comprising a regulatory sequence such as a promoter or an enhancer. The expression of a recombinant gene can be controlled by one or more types of heterogeneous regulatory sequences.

The term "target gene" as used herein can refer to nucleic acid sequences, transgenes, viral or bacterial sequences, chromosomes or extrachromosomal genes that are naturally present in organisms, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the up-regulation of expression of the target gene. "Sequence of a target gene promoter" refers to a non-coding sequence of the target gene, and the reference of the sequence of a target gene promoter in the phrase "complementary with the sequence of a target gene promoter" of the present invention refers to the coding strand of the sequence, also known as a non-template strand, i.e. a nucleic acid sequence having the same sequence as the coding sequence of the gene. "Target sequence" refers to a sequence fragment in the sequence of a target gene promoter, which is homologous or complementary with a sense oligonucleotide strand or an antisense oligonucleotide strand of a small activating nucleic acid molecule.

As used herein, the terms "sense strand" and "sense oligonucleotide strand" can be used interchangeably, and the sense oligonucleotide strand of a small activating nucleic acid molecule refers to the first nucleic acid strand of the small activating nucleic acid molecule duplex having sequence identity to the coding strand of the promoter sequence of a target gene.

As used herein, the terms "antisense strand" and "antisense oligonucleotide strand" can be used interchangeably, and the antisense oligonucleotide strand of a small activating nucleic acid molecule refers to the second nucleic acid strand of the small activating nucleic acid moledule duplex, which is complementary with the sense oligonucleotide strand.

The term "coding strand" as used herein refers to a DNA strand in the target gene which cannot be used for transcription, and the nucleotide sequence of this strand is the same as that of a RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, i.e. the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which plays a regulatory role for the transcription of a protein-coding or RNA-coding nucleic acid sequence by associating with them spatially. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, the promoter sequence may also exist in exon and intron sequences.

The term "transcription start site" as used herein refers to a nucleotide marking the transcription start on the template strand of a gene. The transcription start site may appear on the template strand of the promoter region. A gene can have more than one transcription start site.

The term "identity" or "homology" as used herein means that one oligonucleotide strand (sense or antisense strand) of an saRNA has similarity with a coding strand or a template strand in a region of the promoter sequence of a target gene. As used herein, the "identity" or "homology" may be at least about 75%, about 79%, about 80%, about 85%, about 90%, about 95% or 99%.

The term "overhang" as used herein refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation" or "activating gene expression" and "gene up-regulation" or "up-regulating gene expression" can be used interchangeably, and mean an increase in transcription, translation, expression or activity of a certain nucleic acid as determined by measuring the transcriptional level of a gene, mRNA level, protein level, enzymatic activity, methylation state, chromatin state or configuration, translation level, or the activity or state in a cell or biological system. These activities or states can be determined directly or indirectly. In addition, "gene activation", "activating gene expression", "gene up-regulation" or "up-regulating gene expression" refers to an increase in activity associated with a nucleic acid sequence, regardless of the mechanism of such activation. For example, gene activation occurs at the transcriptional level to increase transcription into RNA and the RNA is translated into a protein, thereby increasing the expression of the protein.

As used herein, the terms "small activating RNA", "saRNA", and "small activating nucleic acid molecule" can be used interchangeably, and refer to a nucleic acid molecule that can up-regulate target gene expression and can be composed of a first nucleic acid fragment (antisense strand, also referred to as antisense oligonucleotide strand) comprising a nucleotide sequence having sequence identity to the non-coding nucleic acid sequence of a target gene (for example, a promoter and an enhancer) and a second nucleic acid fragment (sense strand, also referred to as sense oligonucleotide strand) comprising a nucleotide sequence complementary with the first nucleic acid fragment, wherein the first nucleic acid fragment and the second nucleic acid fragment form a duplex. The small activating nucleic acid molecule can also be composed of a synthesized or vector-expressed single-stranded RNA molecule that can form a hairpin structure by two complementary regions within the molecule, wherein the first region comprises a nucleotide sequence having sequence identity to the target sequence of a promoter of a gene, and the second region comprises a nucleotide sequence which is complementary with the first region. The length of the duplex region of the small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also comprise nucleic acids other than the ribonucleotide, including, but not limited to, modified nucleotides or analogues.

As used herein, the term "hot spot" refers to a gene promoter region of at least 30 bp in length. The gathering of functional small activating nucleic acid molecules appears in these hot spot regions, wherein at least 30% of the small activating nucleic acid molecules targeting these hot spot regions can induce a 1.2-fold or more change in the mRNA expression of a target gene.

As used herein, the term "synthesis" refers to a method for synthesis of an oligonucleotide, including any method allowing RNA synthesis, such as chemical synthesis, *in vitro* transcription, and/or vector-based expression.

The present invention provides a method for preparing the small activating nucleic acid molecule, which comprises sequence design and sequence synthesis.

The synthesis of the sequence of the small activating nucleic acid molecule can adopt a chemical synthesis or can be entrusted to a biotechnology company specialized in nucleic acid synthesis.

Generally speaking, the chemical synthesis comprises the following four steps: (1) synthesis of oligomeric ribonucleotides; (2) deprotection; (3) purification and isolation; (4) desalination and annealing.

For example, the specific steps for chemically synthesizing the saRNA described herein are as follows:

(1) Synthesis of oligomeric ribonucleotides

Synthesis of 1 μM RNA was set in an automatic DNA/RNA synthesizer (e.g., Applied Biosystems EXPEDITE8909), and the coupling time of each cycle was also set as 10 to 15 min. With a solid phase-bonded 5'-O-p-dimethoxytriphenylmethyl-thymidine substrate as an initiator, one base was bonded to the solid phase substrate in the first cycle, and then, in the nth ($19 \geq n \geq 2$) cycle, one base was bonded to the base bonded in the n-1th cycle. This process was repeated until the synthesis of the whole nucleic acid sequence was completed.

(2) Deprotection

The solid phase substrate bonded with the saRNA was put into a test tube, and 1 mL of a mixed solution of ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluents were combined, and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of *n*-butanol. Precipitate was collected to give a single-stranded crude product of saRNA by high-speed centrifugation.

(3) Purification and isolation

The resulting crude product of saRNA was dissolved in 2 mL of aqueous ammonium acetate solution with a concentration of 1 mol/mL, and the solution was separated by a reversed-phase C18 column of high pressure liquid chromatography to give a purified single-stranded product of saRNA.

(4) Desalination and annealing

Salts were removed by gel filtration (size exclusion chromatography). A single sense oligomeric ribonucleic acid strand and a single antisense oligomeric ribonucleic acid strand were mixed into 1 to 2 mL of buffer (10 mM Tris, pH = 7.5-8.0, 50 mM NaCl) at a molar ratio of 1:1. The solution was heated to 95 °C, and was then slowly cooled to room temperature to give a solution containing saRNA.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer.

## EXAMPLES

### Example 1: Design and Synthesis of Small Activating Nucleic Acid Molecule Targeting Gene Promoter

Sense promoter sequence of target genes (Table 1) from the transcription start site (TSS) to upstream -500 bp or -1000 bp was obtained from the UCSC genome database (genome.ucsc.edu). With the sequence as a template, targets with a size of 19 bp were selected from the TSS to the most upstream. The targets must have the following features: (1) the GC content is between 35% and 65%; (2) five or more continuous identical nucleotides, such as "AAAAA", are not contained; (3) more than three dinucleotide repeat sequences, such as "CTCTCTCT", are not contained; (4) more than three trinucleotide repeat sequences, such as "ACTACTACTACT", are not contained. On the basis of meeting the aforementioned criteria, the targets having the following features were preferred as candidate saRNA targets: (1) the target region is located in the gene promoter from -100 bp to -500 bp or from -100 bp to -1000 bp; (2) the saRNA duplex corresponding to the target has asymmetric thermodynamic stability, that is, the thermodynamic stability of the 3' terminus is lower than that of the 5' terminus.

Table 1: Design of saRNAs targeting skin-related genes

| Gene | Promoter sequence length for saRNA design (bp) | Number of candidate saRNAs |
|---|---|---|
| AQP3 | 1000 | 20 |
| AQP9 | 500 | 20 |
| ELN | 1000 | 12 |
| COL1A1 | 500 | 11 |
| COL1A2 | 500 | 12 |
| COL3A1 | 500 | 20 |
| HAS1 | 500 | 12 |
| HAS2 | 500 | 20 |
| HAS3 | 500 | 10 |
| MFAP2 | 500 | 20 |

The promoter sequence of each aforementioned gene used is as follows:

AQP3 promoter sequence (1000 bp) (SEQ ID NO: 373)

–1000 gtggaggttg cagggagccg agatcacgcc attgcactcc acctaggcga
–950 cagagagaga ctccgtctaa aaaaaaaaaa gagacagact cttccttggc
–900 tgggggtaag tcagatggga gaggagaggg ttaaaaacag ctgggactca
–850 gcctgctggc aaacatgtgg catgtggcat gtcggggcaa ctgcagctca
–800 gcctctggag ccatgtgagc aatgcacgca ggtacacgtg tgacaagcta
–750 ggtcacctag ccatgttcaa caggcatgtg cacagccacg aggaatgccc
–700 agccgtacaa ttaggcacac aggacatccg ccatgtgtag acacagctgt
–650 ggacatagct ggccaggaca tgcgacacac gacgtgctca tagcacaggg
–600 agaagggccc atgaagtctg gttggaactc agcacgtgtg tctgtgtgcc

–550 cacctgagtc tggactgctg cccctctgac actagctgtc cccttgaagg
–500 gtcggtgcct tatctgtcct gacagaagag acagtgttgc ttctcacttg
–450 gggctcgcag cctcctcctc ctgcctcgaa ctgaggatct gttgggtcca
–400 gtcatcctgg agagatgcgg ccagtttctt tctgacaggt ctcctcctgc
–350 ccgcaaggaa gtggggtgat cacagggcgc aggtggtctc tatgacagct
–300 gcatcctctc cagccatggc cctgaaccct gcctataatc ccaccattgg
–250 ctctcagatc tgcctaagcc tctcagcccc cttgacgtcc cctcccttaa
–200 gcgccctccg aaggccaccc gtccctcaaa gctcctcaca ctccatgccc
–150 gcagctccct ccacccggcg tccgcaccag cctcccagcc gaggtggggc
–100 gggggcgagg gggcgcgcac tcctcggcgc tccgggactg cagggcgggg
–50 ctgcagggcg ggcggggccc gtgtctccag cgctcctata aagggagcca

AQP9 promoter sequence (500 bp) (SEQ ID NO: 374)

–500 tattttcaaa ctggaatgct tcttcagaag aaacccaaac tggaatgttt
–450 cttcagaaga aacttcagaa gtcaccaaaa aggtgactcc tccatctttc
–400 tgattcttgg atcctaaagt atcttcagca agtgaagccc agtttcataa
–350 ccgaagaggt gactccatct gtcctacaga cttgcagtgt gaagcttgca
–300 atggtttctc tgtatgttaa tttctccatc tctaggatgg cctatgtggt
–250 tcttactaac ctcagagtgg tatcatgaag ccactgaaat gataccagaa
–200 gaaaacaaga gtgattaatc ttctaggatg ataaatctat attttcttca
–150 aattcttcat tttgcataaa aaccttttc agaaaagaat gttaacattg
–100 gatatatcta cacctgattt gacagttcca aactacaccc caaccaccca
–50 cttctgaatg agaagaaaaa aaaatcagaa gcctgcaatt gtgtaacatg

ELN promoter sequence (1000 bp) (SEQ ID NO: 375)

–1000 tcctattctc taggtctcac atttcttctc ctctagcagt agtgggaagt
–950 gaggggtggg ggacacgacc ctcccctgtt ccatccccaca ctccaacccc
–900 caaaatcccc cagggtcccc gtccagctca gtcctggggg cagaaatgca
–850 gagttctcca ggaacgtggt cccagctgtt tcagtgcagg ccgcccccctc
–800 ctggccacca gcggaatgtc agccttccca gaggggccgg gagaacagca
–750 gtcgagaagc tcccagactg gtgtgggcgc tagctgtgct cagcgtgggg
–700 atgggaggtg acccagtgat aatgggaagc tgggctgcct gtcagtctgc
–650 ggggggctcc cacctccctg ttcccccaca gggcacctgg ggatccagcc

–600 tgatttttac cagaccctgc ggcctgcatg gggctgggta tagggctgtg
–550 accttgaccc atgcagaata gaaccctgtg tgtcgggatc ctccatgtgc
–500 tccagatgcc cctggggaca gcaccaacat ggccttaact cccaagccat
–450 tcccctgcct ctaaccccct ggcatctgca ggcatccacc ccagacccac
–400 ccaacacctc ctccccagct tcaggcgcta ggcagagacc ttggcccctg
–350 cagaatgcag ccctgtccag ggtcccctac cttcccccca gatccctccc
–300 agagcaatac caacccgggc ctaccttcca ggccattcaa cctgcagccc
–250 cccggcctct gtagacatcg caccccccaa acccccagac ctgcccaatg
–200 cctcccctcc ccagctttgg gcagaacctg tctctagcca gacctggggg
–150 tgttggggag tctggagggc cggggtgggg gctgaggcgc gggacagctg
–100 gcccgtatcc tcacactggg cccggggccc agccggaggg gcgggggcct
–50 ggccactcgg gccttggctg gggctgggat ttttggcctg gccgccaggc

COL1A1 promoter sequence (500 bp) (SEQ ID NO: 376)

–500 gttttggaga ggtcctcagc atgcctcttt atgccccctcc cttagctctt

–450 gccaggatat cagagggtga ctggggcaca gccaggagga ccccctcccc

–400 aacacccccca acccttccac ctttggaagt ctccccaccc agctccccag

–350 ttccccagtt ccacttcttc tagattggag gtcccaggaa gagagcagag

–300 gggcacccct acccactggt tagcccacgc cattctgagg acccagctgc

–250 acccctacca cagcacctct ggcccaggct gggctggggg gctggggagg

–200 cagagctgcg aagaggggag atgtggggtg gactcccttc cctcctcctc

–150 cccctctcca ttccaactcc caaattgggg gccgggccag gcagctctga

–100 ttggctgggg cacgggcggc cggctccccc tctccgaggg gcagggttcc

–50 tccctgctct ccatcaggac agtataaaag ggcccgggc cagtcgtcgg

COL1A2 promoter sequence (500 bp) (SEQ ID NO: 377)

–500 atgagcctca gcaaaggcaa gctaggaggt cgaaggactt ccccaggtga

–450 ctcggtctag tctagagttc gcaaagccta tcctccctgt agccgggtgc

–400 caagcagcct cgagcctgct ccccagccca cctgccaaca aaaggcgccc

–350 tccgactgca acccagccct ccacagacag gacccgccct ttcccgaagt

–300 cataagacaa agagagtgca tcactgctga aacagtgggc gcacacgagc

–250 cccaaagcta gagaaaagct ggacggggct gggggcgggg tgcaggggtg

–200 gaggggcggg gaggcgggct ccggctgcgc cacgctatcg agtcttccct

–150 ccctccttct ctgccccctc cgctcccgct ggagccctcc accctacaag

–100 tggcctacag ggcacaggtg aggcgggact ggacagctcc tgctttgatc

–50 gccggagatc tgcaaattct gcccatgtcg gggctgcaga gcactccgac

COL3A1 promoter sequence (500 bp) (SEQ ID NO: 378)

–500 tgatatttgc ctgaaactta acttctagga cccagggtgg gtggatgagt

–450 cgaaggggca tacggtggca tttctttccg tgagtctctt acagtctcct

–400 atttaaattg agttaggatt acttctggca aaatcccaac ataaaaatct

–350 tctaggaaga tcagttctgt aaattagaca tactagataa atgggcatca

–300 agcagttttt caaaattatg cagttgttaa cttcataagg ggaaataaaa

–250 atgtatgcat ttacattgta tgtattaaaa caaggcagag catttctata

–200 cgttcctaag ttatacaaac atatatgtaa gagtgaaata tgtaaaaaaa

–150 cttttacata agcagatgca tacaaactcc agatgtgctc tttttcttac

–100 tgtgggttgt gtcttctata agggaaaaag aaatatttat catttctttt

–50 actgctgagg ggatgggtgc ggctctcata tttcagaaag gggctggaaa

HAS1 promoter sequence (500 bp) (SEQ ID NO: 379)

–500 agagaagtgg agaggaagaa agggatggag agaaagacag aaacttggag

–450 aaatgtggta atggggagag agatgagggt accaacaaag agatgaggag

–400 aaatagtggg ttagggagaa agaaagggat ggagggaaat acagggatag

–350 gggatggggg gatgagggga acccccaaaa tgcaagtgag gtggaagaga

–300 aaaatcaggg gagacaaggg acgagagggg ggaggaagag agaagaagac

–250 atagaggagg gagggagagg agggggagtg atggcagaga ggactagagg

–200 aggggcgcgg gtagacatgg gggcctggga agggaggccc gtctggctga

–150 ggggggtggg agtaatagcg ggaagcgggt ggagctgccc ggctgggccg

–100 tgacctcaga gcccctggcc cagcttagcc tgactgacgt cagcgctcgc

–50 tcccgccccc gcctgcgctg gtcttcaaat gacccccctc cagctctctg

HAS2 promoter sequence (1000 bp) (SEQ ID NO: 380)

–1000 ggagtggtgg ggggaggagt aaccaaaaaa cgaactctac ccagccttgg

–950 ggcattgtga aaacgacgag gaaaggccat ctccaagcaa gacatatttg

–900 tcctcgtcaa ctttgcagaa ggcttgcaaa aagcaaaagc cagttaagcc

–850 atttccttca agatggctaa atataagtcc tccagaaaac aatttttatt

–800 attttatttt taatgcgcgc tgtttgagta tgtttacgtt aggggaccag

–750 attgagcaaa aatatacata taattacttt tctatgtttt ccgcccaccc
–700 acggcagaaa cctctttatg agaaaggctt tgacacttga cgtcagctca
–650 gaaacttttg agttggcggg aagaaagggt taacaagtgg tggaccagcg
–600 cgatcttttt agagtcccag ccggctgtcg aaggctccag gtacacacac
–550 acacgcacac acttacacac acacacacac acgtgcacac acccctccca
–500 actgttcctc cttgggtcgg ttacttagct gaagggcacc atttggcttt
–450 aaaacaaatt acttcagcgc cacagggagt ttatcgcttg gagggaactt
–400 gtaacgcggg agttcaggga aatccaagag gcgagggctc ccacctccgc
–350 cgccagagga aagtaaggaa tcaggtggtg gccccctatg tgtcttgcat
–300 ttctctgcac acgttagcaa tcaagttaat tgaattcatt ggagtttaga
–250 accggcctgt agctcagaga aggctttgaa tggccaattt ctctctctcc
–200 ctctccccct ccccgcctcc cgctcgcccg cccgcccgcg ctcccagttc
–150 cctcccctca gggttcccca gtccacacct ccctctccac ttccctcacc
–100 cccccactcc ctccgccgcc ctattaaaac acccaccagc tcacttgtta
–50 agaccccctt aagttggagg aggcagaagg gcaacaacgg cggggaagga

HAS3 promoter sequence (500 bp) (SEQ ID NO: 381)

–500 gtggtgcgat ttcagctcac tgcaacctcc acctgcgggg ttcaagcgat
–450 tctcctgcct cagcctcccg agtagctggg attacgggca tggtgcccgt
–400 aacgccgaaa gtgctgagat tacaggcttg agccacggcg cccggccact
–350 cattttttaa aaactagtta ttaattataa tttctcttca agttttcctg
–300 agatgtaaaa actcatcgcc ttcagctggt tctaaacttc ttgtctctgc
–250 taacaactct ccccccctcct cgatctcacc ccaccccagt gcaccccaac
–200 tcacttcatc tcactttact cgagtctagc actatctttt agggacctga
–150 aaactactgt cgataaggtc agttgggaat ttaggaggtc atggatgtcg
–100 gaaataaagg tgtgaaaaag aagaggagga attgttttgg ctttaagaca
–50 ccggaacctc caaggttcct acaaaaccgt ttgcagcttt tgcaaaccgc

MFAP2 promoter sequence (500 bp) (SEQ ID NO: 382)

–500 ggacatttgt gggacacata ggctgggtca gggctgaaag aggtgctggt
–450 tatggccggg ggcagggact catgcctgta atcccaacag cccaggagga
–400 tgagacagga ggaatgcttg aggccaaaat ttcgaggccg gaagttccag
–350 acgagcctgg gcaacacagc aagaccctgt ctctagaaaa ggaaagaaag

–300 aaccgctggt tgtggaagcc agccatggcc cagagctcag cagtgtagga

–250 gaggagggtg cgggcctgag agaggcagca ggcttggctg gagaggcaga

–200 aggaaaacca aggcaggaga gtgtcctgga agctgggaga aggcagaggg

–150 agatcgaggc ttactttctg gctgaggggc ctagggtgag tcactttggg

–100 aggcttgatt tcctcctctg tgaaatgggc aacacaccta cccttgccca

–50 cctcaccccc ctcaccccgt cgacgtcgag tgaggagcag ctgtgaggag

After the target sequences of the saRNAs were determined, saRNA duplex sequences were generated. The steps were as follows: (1) based on the DNA sequences of the targets, sense strand ribonucleotide sequences of saRNAs of 19 nucleotides in length were generated, which were identical with the target sequences except that "T" was substituted by "U"; (2) with the sense strands of the saRNAs as templates, antisense strand ribonucleotide sequences of saRNAs of 19 nucleotides in length which were complementary with the sense strands were generated; (3) two deoxythymidines "dTdT" were respectively added to the 3' termini of the sense strand sequence and the antisense strand sequence, and the length of each of the sense strand and antisense strand of the saRNA obtained ultimately was 21 nucleotides.

Corresponding double-stranded small activating RNAs were chemically synthesized based on these candidate sequences. Each of the sense strand and antisense strand in the double-stranded small activating RNA used in the study had 21 nucleotides in length. The 19 nucleotides in the 5' region of the first ribonucleic acid strand (sense strand) of the double-stranded saRNA had 100% identity with the target sequence of the promoter, and the 3' terminus of the first ribonucleic acid strand contained a dTdT sequence. The 19 nucleotides in the 5' region of the second ribonucleic acid strand were fully complementary with the first ribonucleic acid strand sequence, and the 3' terminus of the second ribonucleic acid strand contained a dTdT sequence. The aforementioned two strands of the double-stranded saRNA were mixed at a molar ratio of 1:1, and after annealing, a double-stranded saRNA was formed.

<u>Example 2: Screening for Active saRNAs for Aquaporin Genes, Elastin Genes, Collagen Genes, Hyaluronic Acid Genes and Microfibril-associated Protein Genes</u>

**(1) Cell culture and transfection**

Normal human primary fibroblasts Hs27 and NHDF were purchased from BNBIO, and the product numbers were BNCC341921 and BNCC340692, respectively. Normal human epidermal keratinocytes (NHEKs) were purchased from BNBIO, and the product number was BNCC340593. The cells were cultured in high-glucose DMEM media (Gibco); and all the media contained 10% of fetal bovine serum (Gibco) and 1% of penicillin/streptomycin (Gibco). The cells were cultured at 5% $CO_2$ and 37 °C. The cells were inoculated in a 6-well plate at $5 \times 10^6$ cells/well. saRNAs were transfected into the cells at a final concentration of 10 nM (unless otherwise specified) according to a reverse transfection method by using RNAiMAX transfection reagent (Invitrogen, Carlsbad, Calif.) for 72 h (unless otherwise specified). Control treatments included Mock treatment (transfection treatment of omitting nucleic acid duplexes) and control-independent nucleic acid duplex (dsControl) treatment. The sequences of the sense strand and antisense strand of dsControl were respectively 5'-ACUACUGAGUGACAGUAGATT-3' (SEQ ID NO: 383) and 5'-UCUACUGUCACUCAGUAGUTT-3' (SEQ ID NO: 384), which did not have obvious homology with known sequences of the human genome.

**(2) RNA extraction and RT-qPCR**

At the end of the transfection, total cellular RNA was extracted using an RNeasy Plus Mini kit (Qiagen) according to its manual. The resulting RNA (1 μg) was reverse transcribed into cDNA using a PrimeScript RT kit containing gDNA Eraser (Takara, Shiga, Japan). The resulting cDNA were amplified by RT-qPCR using an ABI 7500 rapid real-time PCR system (Applied Biosystems) and SYBR Premix Ex Taq II (Takara, Shiga, Japan) reagent, and each sample was amplified in another 3 wells. The reaction conditions were: 95°C for 3 s, 60°C for 30 s, and 40 cycles. HPRT1 or GAPDH was taken as an internal reference gene. All the primer sequences used are listed in Table 2.

Table 2: Primer sequences for RT-qPCR analysis

| Gene | Primer | Sequence (5'-3') |
|---|---|---|
| AQP3 | AQP3-OSqF2 | CACTCTGGGCATCCTCATCG (SEQ ID NO: 385) |
| | AQP3-OSqR2 | GCGAAGTGCCAGATTGCATCATA (SEQ ID NO: 386) |
| AQP9 | AQP9-F | TCCTCAGAGAAGCCCCAAGA (SEQ ID NO: 387) |
| | AQP9-R | AGCCACATCCAAGGACAATCA (SEQ ID NO: 388) |
| COL1A1 | COL1A1-OSqF2 | CGGCTCAGAGTCACCCAC (SEQ ID NO: 389) |
| | COL1A1-OSqR2 | AAGTCCAGGCTGTCCAGGG (SEQ ID NO: 390) |
| COL1A2 | COL1A2-OSqF1 | CAGCCGGAGATAGAGGACCA (SEQ ID NO: 391) |
| | COL1A2-OSqR1 | CAGCAAAGTTCCCACCGAGA (SEQ ID NO: 392) |
| COL3A1 | COL3A1-F | CCAGGAGCTAACGGTCTCAG (SEQ ID NO: 393) |
| | COL3A1-R | CAGGGTTTCCATCTCTTCCA (SEQ ID NO: 394) |
| ELN | ELN-OSqF1 | TCCTGGTGGAGTTCCTGGAG (SEQ ID NO: 395) |
| | ELN-OSqR1 | CCGGGAACTGGCTTAAGAGG (SEQ ID NO: 396) |
| HAS1 | HAS1-F | TAGGAATAACCTCTTGCAGCAGT (SEQ ID NO: 397) |
| | HAS1-R | GACCTGGAGGTGTACTTGGT (SEQ ID NO: 398) |
| HAS2 | HAS2-F | CAGAATCCAAACAGACAGTTC (SEQ ID NO: 399) |
| | HAS2-R | TAAGGTGTTGTGTGTGACTGA (SEQ ID NO: 400) |
| HAS3 | HAS3-F | GGAAGGTTTTGCTGCCTTGG (SEQ ID NO: 401) |
| | HAS3-R | GCACCGGCATCCTGCAA (SEQ ID NO: 402) |
| MFAP2 | MFAP2-F | CAACTTGGTCTCACAGTGGCT (SEQ ID NO: 403) |
| | MFAP2-R | GTGTCAGAGAGGACAGCTGAAAA (SEQ ID NO: 404) |
| HPRT1 | HPRT1-F | ATGGACAGGACTGAACGTCTT (SEQ ID NO: 405) |
| | HPRT1-R | TCCAGCAGGTCAGCAAAGAA (SEQ ID NO: 406) |
| GAPDH | GAPDH-qF3 | ATCACCATCTTCCAGGAGCGA (SEQ ID NO: 407) |
| | GAPDH-qR3 | TTCTCCATGGTGGTGAAGACG (SEQ ID NO: 408) |

(3) RT-qPCR data analysis

For RT-qPCR data analysis amplifying 2 internal reference genes simultaneously, the Ct values of the target gene and the 2 internal reference genes were substituted into formula 1 in order to calculate the expression value (Erel) of a target gene of an saRNA-transfected sample relative to the control treatment (Mock).

$$E_{rel} = 2^{(CtTm-CtTs)} / ((2^{(CtR1m-CtR1s)} * 2^{(CtR2m-CtR2s)})^{(1/2)})$$

**(formula 1)**

wherein CtTm was the Ct value of the target gene from the control treatment (Mock) sample; CtTs was the Ct value of the target gene from the saRNA-treated sample; CtR1m was the Ct value of the internal reference gene 1 from the control treatment (Mock)-treated sample; CtR1s was the Ct value of the internal reference gene 1 from the saRNA-treated sample; CtR2m was the Ct value of the internal reference gene 2 from the control treatment (Mock)-treated sample; and CtR2s was the Ct value of the internal reference gene 2 from the saRNA-treated sample.

For RT-qPCR data analysis using 1 internal reference gene, the Ct values of the target gene and the internal reference gene were substituted into formula 2 in order to calculate the expression value (Erel) of the target gene of an saRNA-transfected sample relative to control treatment (Mock).

$$E_{rel} = 2^{-(CtTs-CtRs)-(CtTm-CtRm)}$$

**(formula 2)**

wherein CtTm was the Ct value of the target gene from the control treatment (Mock) sample; CtTs was the Ct value of the target gene from the saRNA-treated sample; CtRm was the Ct value of the internal reference gene from the control treatment (Mock)-treated sample; and CtRs was the Ct value of the internal reference gene from the saRNA-treated sample.

**(4) Western blotting**

Cells were collected and lysed with cell lysis buffer (1× RIPA buffer, CST, #9806). Protease inhibitor (Sigma, Lot#126M4015v) was added to the lysis buffer. The protein samples were quantified by the BCA method, and separated by polyacrylamide gel electrophoresis. Upon completion of electrophoresis, the protein samples were transferred to a 0.45 μm of PVDF membrane. A rabbit anti-human AQP3 polyclonal antibody (Invitrogen, Cat # PA5-53257), a rabbit anti-ELN polyclonal antibody (Invitrogen, Cat # PA5-63369), a rabbit anti-ab-tubulin polyclonal antibody (CST, #2148) or a mouse anti-human GAPDH monoclonal antibody (Sigma, G9295) was used to detect the Western blot corresponding to the antibody, and an anti-mouse IgG HRP-linked antibody (CST, #7076) or an anti-rabbit IgG HRP- linked antibody (CST, #7074) was used as a secondary antibody. The membrane was scanned using Image Lab (BIO-RAD, Chemistry Doctm MP imaging system) to detect signals.

**(5) Activation of saRNAs on aquaporin gene**

As the epidermis of the human body is most important for skin moisturization, AQP3 is the most important water channel molecule of skin cells. The specific activation of AQP3 will provide beneficial effects for the moisturization of the epidermis and the improvement of skin appearance. In order to prove that saRNAs targeting the promoter of AQP3 can activate the expression of AQP3, 20 saRNAs targeting the promoter of AQP3 were designed and synthesized. These saRNAs were transfected into normal human epidermal keratinocytes (NHEKs) at a final concentration of 10 nM. After 72 h of transfection, the mRNA expression of the AQP3 gene was analyzed by RT-qPCR. The result is shown in **FIG. 1**. It can be seen that a total of 11 of the 20 saRNAs increased AQP3 mRNA expression level by at least 1.5-fold. Among them, 6 saRNAs (AQP3-4, AQP3-6, AQP3-7, AQP3-8, AQP3-11, and AQP3-15) increased the AQP3 mRNA expression level by at least 2-fold, and 5 saRNAs (AQP3-3, AQP3-5, AQP3-14, AQP3-16, and AQP3-19) increased AQP3 mRNA expression level by at least 1.5-fold to 2-fold. In order to further verify whether the up-regulation of AQP3 expression mediated by the saRNAs led to the up-regulation of protein level, 7 saRNAs (AQP3-4, AQP3-5, AQP3-6, AQP3-7, AQP3-8, AQP3-11 and AQP3-15) were selected to transfect the NHEK cells. 72 h later, the protein expression of AQP3 was analyzed using Western blotting. The result is shown in **FIG. 2.** Consistent with the results of mRNA expression, the treatment of the cells by all these saRNAs led to significant up-regulation of AQP3 protein level (compared with control treatment (Mock), ≥ 2-fold). As the control treatment, AQP3 siRNA (siAQP3, sense strand 5'-CCGGCAUCUUUGCUACCUATT-3' (SEQ ID NO: 409); antisense strand 5'-UAGGUAGCAAAGAUGCCGGTT-3' (SEQ ID NO: 410)) down-regulated the protein expression of AQP3, as expected. These results demonstrate that the AQP3 promoter targeted specific saRNAs can up-regulate the expression of AQP3 gene in human primary cultured fibroblasts at mRNA level and protein level, respectively.

AQP9 is mainly expressed in the keratinocytes of the skin. The 20 saRNAs targeting the promoter of AQP9 were each transfected into the NHEKs at a final concentration of 10 nM. 72 h later, the mRNA expression of the AQP9 gene was analyzed by RT-qPCR. As shown in **FIG. 3**, compared with the control treatment (Mock), a total of 10 saRNAs up-regulated the mRNA expression of AQP9 gene by more than 1.5-fold. Among them, 7 (35%) saRNAs (AQP9-2, AQP9-3, AQP9-8, AQP9-10, AQP9-11, AQP9-12 and AQP9-15) up-regulated the mRNA expression of AQP9 gene by more than 2-fold, and 3 (15%) saRNAs (AQP9-5, AQP9-14 and AQP9-17) up-regulated the mRNA expression of AQP9 gene by 1.5-fold to 2-fold. As the control treatment, AQP9 siRNA (siAQP9, sense strand 5'-GCAGUUGCAAUGGCCAUUUTT-3' (SEQ ID NO: 411); antisense strand 5'-AAAUGGCCAUUGCAACUGCT-3' (SEQ ID NO: 412)) down-regulated AQP9 mRNA expression, as expected. These results demonstrate that the AQP9 promoter targeted specific saRNAs can up-regulate the expression of AQP9 gene at the mRNA level in human primary cultured fibroblasts.

**(6) Activation of saRNAs on expression of ELN gene**

Elastin encoded by the ELN gene is mainly produced from fibroblasts in the skin dermis. In order to assay whether the saRNAs can activate the expression of the ELN gene in fibroblasts, 12 saRNAs targeting the promoter of ELN were designed and synthesized. These saRNAs were each transfected into human primary fibroblasts Hs27 at a concentration of 50 nM. 72 h later, ELN mRNA expression in the cells was analyzed by RT-qPCR. The result shows that 4 saRNAs (ELN-1, ELN-5, ELN-9, and ELN-10) (about 33.3%) up-regulated ELN mRNA expression by more than 2-fold (**FIG. 4A**). In order to verify the results of preliminary screening, 3 saRNAs (ELN-1, ELN-5, and ELN-10) were transfected into NHDF cells and Hs27 cells, respectively, at a final concentration of 50 nM for 72 h, and ELN mRNA expression was analyzed by RT-qPCR. The result shows that both ELN-5 and ELN-10 significantly up-regulated ELN mRNA expression in these two types of cells (**FIG. 4B** and **FIG. 4C**). ELN-1 induced ELN expression by more than 1.5-fold in these two types of cells. As the control treatment, ELN siRNA (siELN, sense strand 5'-GUCUCGCUGUGAUAGAUCATT-3' (SEQ ID NO: 413); antisense strand 5'-UGAUCUAUCACAGCGAGACTT-3' (SEQ ID NO: 414)) down-regulated ELN mRNA expression, as expected.

In order to further verify that the saRNAs upregulated ELN expression at the protein level, the saRNAs (50 nM) were transfected into Hs27 cells for 72 h, then the proteins were extracted, and the ELN protein expression level was analyzed. As shown in **FIG. 5**, consistent with the results of mRNA expression, ELN-1, ELN-5, ELN-9 and ELN-10 up-regulated ELN protein expression to different degrees, and the most is ELN-9. These results demonstrate that the expression of the ELN gene can be activated by the ELN promoter targeted specific saRNAs of the present invention, thus leading to the up-regulation of the protein level of the LEN gene.

**(7) Activation of saRNAs on expression of collagen genes COL1A1, COL1A2 and COL3A1**

The COL1A1 and COL1A2 genes encode type I $\alpha$1-chain and $\alpha$2-chain collagens, respectively, and the COL3A1 gene encodes type III $\alpha$1-chain collagen. Collagen is the main component of the extracellular matrix of the dermis, which is mainly secreted by fibroblasts. In order to verify whether the expression of the COL1A1 and COL1A2 genes can be activated by the saRNAs, 11 saRNAs targeting the promoter of the COL1A1 gene (COL1A1 saRNAs), 12 saRNAs targeting the promoter of the COL1A2 gene (COL1A2 saRNAs) and 20 saRNAs targeting the promoter of the COL3A1 gene (COL3A1 saRNAs) were designed and screened (Table 3). The 11 COL1A1 saRNAs were first transfected into human fibroblasts Hs27. 72 h later, the mRNA expression of the AQP9 gene in the cells was analyzed by RT-qPCR. The result shows that at a transfection concentration of 50 nM, 2 saRNAs (COL1A1-5 and COL1A1-8) up-regulated COL1A1 mRNA expression by 2.0-fold, and 3 saRNAs (COL1A1-2, COL1A1-3, and COL1A1-4) up-regulated COL1A1 mRNA expression by 1.5-fold (**FIG. 6**). As the control treatment, COL1A1 siRNA (siCOL1A1, sense strand 5'-CAAUCACCUGCGUACAGAATT-3' (SEQ ID NO: 415); antisense strand 5'-UUCUGUACGCAGGUGAUUGTT-3' (SEQ ID NO: 416)) down-regulated COL1A1 mRNA expression, as expected.

Likewise, the 12 COL1A2 saRNAs were each transfected into NHDF cells at a final concentration of 50 nM. 72 h later, COL1A2 mRNA expression in the cells was analyzed by RT-qPCR. The result shows that all the 12 saRNAs up-regulated COL1A2 mRNA expression to different degrees, and among them, 7 (58.3%) saRNAs (COL1A2-1, COL1A2-2, COL1A2-3, COL1A2-4, COL1A2-6, COL1A2-9, and COL1A2-12) up-regulated COL1A2 mRNA expression by more than 2-fold (**FIG. 7**). As the control treatment, COL1A2 siRNA (siCOL1A2, sense strand 5'-GCUGCUUGCAGUAACCUUATT-3' (SEQ ID NO: 417); antisense strand 5'-UAAGGUUACUGCAAGCAGCTT-3' (SEQ ID NO: 418)) down-regulated COL1A2 mRNA expression by 0.13-fold, as expected.

The 20 COL3A1 saRNAs were transfected into Hs27 cells at a final concentration of 10 nM. 72 h later, COL3A1 mRNA expression in the cells was analyzed by RT-qPCR. The result shows that 5 saRNAs (COL3A1-1, COL3A1-3, COL3A1-6, COL3A1-13, and COL3A1-14) up-regulated COL3A1 mRNA expression by more than 2-fold (**FIG. 8**). As the control treatment, COL3A1 siRNA (siCOL3A1, sense strand 5'-GCUCUGCUUCAUCCCACUATT-3' (SEQ ID NO: 419); antisense strand 5'-UAGUGGGAUGAAGCAGAGCTT-3' (SEQ ID NO: 420)) down-regulated COL3A1 mRNA expression, as expected.

**(8) Up-regulation of expression of HAS1, HAS2 and HAS3 genes by saRNAs**

Hyaluronic acid (HA) is encoded by 3 highly homologous hyaluronic acid synthetase (HAS) genes HAS1, HAS2, and HAS3. The differences between them are the different synthesis rates and the different chain lengths of the synthesized HA proteins. For HAS1, HAS2, and HAS3 genes, 12, 20, and 20 saRNAs targeting their respective promoters were designed, respectively (Table 3). These saRNAs (final concentration: 10 nM) were transfected into Hs27 cells. 72 h later, the expression of HAS1, HAS2, and HAS3 were respectively analyze by RT-qPCR.

As shown in **FIG. 9**, compared with the control treatment (Mock) group, among the 12 saRNAs targeting the promoter of HAS1, 2 (16.7%) saRNAs (HAS1-5 and HAS1-9) up-regulated HAS1 mRNA expression by more than 2-fold, and 5 (41.7%) saRNAs (HAS1-2, HAS1-3, HAS1-4, HAS1-6, and HAS1-10) up-regulated HAS1 mRNA expression by 1.5-fold to 2-fold. As shown in **FIG. 10**, among the 20 saRNAs targeting the promoter of HAS2, 7 (35%) saRNAs (HAS2-2, HAS2-7, HAS2-8, HAS2-9, HAS2-14, HAS2-18, and HAS2-19) up-regulated HAS2 mRNA expression by more than 2-fold, and 3 (15%) saRNAs (HAS2-1, HAS2-4, and HAS2-17) up-regulated HAS2 mRNA expression by 1.5-fold to 2-fold. As shown in **FIG. 11**, among the 20 saRNAs targeting the promoter of HAS3, 3 (15%) saRNAs (HAS3-6, HAS3-13, and HAS3-14) activated HAS3 expression by more than 2-fold, and 12 (60%) saRNAs (HAS3-1, HAS3-2, HAS3-3, HAS3-7, HAS3-9, HAS3-11, HAS3-12, HAS3-15, HAS3-16, HAS3-18, HAS3-19, and HAS3-20) up-regulated HAS expression by 1.5-fold to 2-fold.

The siRNA sequences of the coding genes of hyaluronic acid as control treatment are as follows:

siHAS1, sense strand 5'- CUGCAUCAGCGGUCCUCUATT-3' (SEQ ID NO: 421); antisense strand 5'-UAGAGGACCGCUGAUGCAGTT-3' (SEQ ID NO: 422)

siHAS2, sense strand 5'-CCAGACUACUUAAGGAAAUTT-3' (SEQ ID NO: 423); antisense strand 5'-AUUUCCUUAAGUAGUCUGGTT-3' (SEQ ID NO: 424)

siHAS3, sense strand 5'-CCUACAUGCUGGACAUCUUTT-3' (SEQ ID NO: 425); antisense strand 5'-AAGAUGUCCAGCAUGUAGGTT-3' (SEQ ID NO: 426)

As shown in **FIGs. 9**—**11**, the aforementioned siRNAs each inhibited the expression of the corresponding genes, as expected.

**(9) Activation of saRNAs on expression of MFAP2**

The MFAP2 gene encodes a microfibril-associated protein, and plays a key role in elastic fiber assembly of the extracellular matrix. In order to activate the expression of the MFAP2 gene, 20 saRNAs targeting the promoter of the MFAP2 gene (Table 3) were designed, and these saRNAs (10 nm) were transfected into Hs27 cells for 72 h. MFAP2 gene expression was analyzed by RT-qPCR. As shown in **FIG. 13**, 8 (40%) saRNAs (MEAP2-1, MEAP2-3, MEAP2-7, MEAP2-8, MEAP2-14, MEAP2-17, MEAP2-18, and MEAP2-19) up-regulated MEAP2 mRNA expression by more than 2-fold, and 3 (15%) saRNAs (MEAP2-6, MEAP2-16, and MEAP2-20) up-regulated MEAP2 mRNA expression by 1.5-fold to 2-fold. The MFAP2 siRNA (siMFAP2) sequence as the control is: sense strand 5'-CCCACUAUAGCGACCAGAUTT-3' (SEQ ID NO: 427); antisense strand 5'-AUCUGGUCGCUAUAGUGGGT-3' (SEQ ID NO: 428).

As shown in **FIG. 13**, the aforementioned siMFAP2 inhibited the expression of the MFAP2 gene, as expected.

Table 3: Active saRNA sequences and active target sequences thereof

| saRNA | Active saRNA target sequence (5'-3') | Sense sequence (5'-3') | Antisense sequence (5'-3') |
|---|---|---|---|
| **AQP3-2** | GCAGGTACACGTGTGACAA (SEQ ID NO: 249) | GCAGGUACACGUGUGACAATT (SEQ ID NO: 1) | UUGUCACACGUGUACCUGCTT (SEQ ID NO: 125) |
| **AQP3-3** | CACTAGCTGTCCCCTTGAA (SEQ ID NO: 250) | CACUAGCUGUCCCCUUGAATT (SEQ ID NO: 2) | UUCAAGGGGACAGCUAGUGTT (SEQ ID NO: 126) |
| **AQP3-4** | TTGAAGGGTCGGTGCCTTA (SEQ ID NO: 251) | UUGAAGGGUCGGUGCCUUATT (SEQ ID NO: 3) | UAAGGCACCGACCCUUCAATT (SEQ ID NO: 127) |
| **AQP3-5** | CCCTGAACCCTGCCTATAA (SEQ ID NO: 252) | CCCUGAACCCUGCCUAUAATT (SEQ ID NO: 4) | UUAUAGGCAGGGUUCAGGGTT (SEQ ID NO: 128) |
| **AQP3-6** | GGCTCTCAGATCTGCCTAA (SEQ ID NO: 253) | GGCUCUCAGAUCUGCCUAATT (SEQ ID NO: 5) | UUAGGCAGAUCUGAGAGCCTT (SEQ ID NO: 129) |
| **AQP3-7** | TTGACGTCCCCTCCCTTAA (SEQ ID NO: 254) | UUGACGUCCCCUCCCUUAATT (SEQ ID NO: 6) | UUAAGGGAGGGGACGUCAATT (SEQ ID NO: 130) |
| **AQP3-8** | TGAAGGGTCGGTGCCTTAT (SEQ ID NO: 255) | UGAAGGGUCGGUGCCUUAUTT (SEQ ID NO: 7) | AUAAGGCACCGACCCUUCATT (SEQ ID NO: 131) |
| **AQP3-9** | CTTATCTGTCCTGACAGAA (SEQ ID NO: 256) | CUUAUCUGUCCUGACAGAATT (SEQ ID NO: 8) | UUCUGUCAGGACAGAUAAGTT (SEQ ID NO: 132) |
| **AQP3-10** | TGGCTCTCAGATCTGCCTA (SEQ ID NO: 257) | UGGCUCUCAGAUCUGCCUATT (SEQ ID NO: 9) | UAGGCAGAUCUGAGAGCCATT (SEQ ID NO: 133) |
| **AQP3-11** | GTGCCTTATCTGTCCTGAC (SEQ ID NO: 258) | GUGCCUUAUCUGUCCUGACTT (SEQ ID NO: 10) | GUCAGGACAGAUAAGGCACTT (SEQ ID NO: 134) |
| **AQP3-14** | TCTCTATGACAGCTGCATC (SEQ ID NO: 259) | UCUCUAUGACAGCUGCAUCTT (SEQ ID NO: 11) | GAUGCAGCUGUCAUAGAGATT (SEQ ID NO: 135) |
| **AQP3-15** | TGACACTAGCTGTCCCCTT (SEQ ID NO: 260) | UGACACUAGCUGUCCCCUUTT (SEQ ID NO: 12) | AAGGGGACAGCUAGUGUCATT (SEQ ID NO: 136) |
| **AQP3-16** | GTCCTGACAGAAGAGACAG (SEQ ID NO: 261) | GUCCUGACAGAAGAGACAGTT (SEQ ID NO: 13) | CUGUCUCUUCUGUCAGGACTT (SEQ ID NO: 137) |
| **AQP3-17** | ACCCTGCCTATAATCCCAC (SEQ ID NO: 262) | ACCCUGCCUAUAAUCCCACTT (SEQ ID NO: 14) | GUGGGAUUAUAGGCAGGGUTT (SEQ ID NO: 138) |
| **AQP3-19** | GGTCTCTATGACAGCTGCA (SEQ ID NO: 263) | GGUCUCUAUGACAGCUGCATT (SEQ ID NO: 15) | UGCAGCUGUCAUAGAGACCTT (SEQ ID NO: 139) |

(continued)

| saRNA | Active saRNA target sequence (5'-3') | Sense sequence (5'-3') | Antisense sequence (5'-3') |
|---|---|---|---|
| **AQP9-1** | CTGGAATGCTTCTTCAGAA (SEQ ID NO: 264) | CUGGAAUGCUUCUUCAGAATT (SEQ ID NO: 16) | UUCUGAAGAAGCAUUCCAGTT (SEQ ID NO: 140) |
| **AQP9-2** | ATGGCCTATGTGGTTCTTA (SEQ ID NO: 265) | AUGGCCUAUGUGGUUCUUATT (SEQ ID NO: 17) | UAAGAACCACAUAGGCCAUTT (SEQ ID NO: 141) |
| **AQP9-3** | CCTGATTTGACAGTTCCAA (SEQ ID NO: 266) | CCUGAUUUGACAGUUCCAATT (SEQ ID NO: 18) | UUGGAACUGUCAAAUCAGGTT (SEQ ID NO: 142) |
| **AQP9-5** | GGTGACTCCATCTGTCCTA (SEQ ID NO: 267) | GGUGACUCCAUCUGUCCUATT (SEQ ID NO: 19) | UAGGACAGAUGGAGUCACCTT (SEQ ID NO: 143) |
| **AQP9-8** | CCATCTCTAGGATGGCCTA (SEQ ID NO: 268) | CCAUCUCUAGGAUGGCCUATT (SEQ ID NO: 20) | UAGGCCAUCCUAGAGAUGGTT (SEQ ID NO: 144) |
| **AQP9-10** | TGCAGTGTGAAGCTTGCAA (SEQ ID NO: 269) | UGCAGUGUGAAGCUUGCAATT (SEQ ID NO: 21) | UUGCAAGCUUCACACUGCATT (SEQ ID NO: 145) |
| **AQP9-11** | TGGTATCATGAAGCCACTG (SEQ ID NO: 270) | UGGUAUCAUGAAGCCACUGTT (SEQ ID NO: 22) | CAGUGGCUUCAUGAUACCATT (SEQ ID NO: 146) |
| **AQP9-12** | GACTCCTCCATCTTTCTGA (SEQ ID NO: 271) | GACUCCUCCAUCUUUCUGATT (SEQ ID NO: 23) | UCAGAAAGAUGGAGGAGUCTT (SEQ ID NO: 147) |
| **AQP9-14** | GCAATGGTTTCTCTGTATG (SEQ ID NO: 272) | GCAAUGGUUUCUCUGUAUGTT (SEQ ID NO: 24) | CAUACAGAGAAACCAUUGCTT (SEQ ID NO: 148) |
| **AQP9-15** | GCCTATGTGGTTCTTACTA (SEQ ID NO: 273) | GCCUAUGUGGUUCUUACUATT (SEQ ID NO: 25) | UAGUAAGAACCACAUAGGCTT (SEQ ID NO: 149) |
| **AQP9-17** | CCACTTCTGAATGAGAAGA (SEQ ID NO: 274) | CCACUUCUGAAUGAGAAGATT (SEQ ID NO: 26) | UCUUCUCAUUCAGAAGUGGTT (SEQ ID NO: 150) |
| **AQP9-19** | GTCCTACAGACTTGCAGTG (SEQ ID NO: 275) | GUCCUACAGACUUGCAGUGTT (SEQ ID NO: 27) | CACUGCAAGUCUGUAGGACTT (SEQ ID NO: 151) |
| **AQP9-20** | GCCCAGTTTCATAACCGAA (SEQ ID NO: 276) | GCCCAGUUUCAUAACCGAATT (SEQ ID NO: 28) | UUCGGUUAUGAAACUGGGCTT (SEQ ID NO: 152) |
| **ELN-1** | GACCTTGACCCATGCAGAA (SEQ ID NO: 277) | GACCUUGACCCAUGCAGAATT (SEQ ID NO: 29) | UUCUGCAUGGGUCAAGGUCTT (SEQ ID NO: 153) |
| **ELN-2** | GATCCCTCCCAGAGCAATA (SEQ ID NO: 278) | GAUCCCUCCCAGAGCAAUATT (SEQ ID NO: 30) | UAUUGCUCUGGGAGGGAUCTT (SEQ ID NO: 154) |

(continued)

| saRNA | Active saRNA target sequence (5'-3') | Sense sequence (5'-3') | Antisense sequence (5'-3') |
|---|---|---|---|
| **ELN-4** | CCTTGACCCATGCAGAATA (SEQ ID NO: 279) | CCUUGACCCAUGCAGAAUATT (SEQ ID NO: 31) | UAUUCUGCAUGGGUCAAGGTT (SEQ ID NO: 155) |
| **ELN-5** | CAGCACCAACATGGCCTTA (SEQ ID NO: 280) | CAGCACCAACAUGGCCUUATT (SEQ ID NO: 32) | UAAGGCCAUGUUGGUGCUGTT (SEQ ID NO: 156) |
| **ELN-6** | ATGCAGAGTTCTCCAGGAA (SEQ ID NO: 281) | AUGCAGAGUUCUCCAGGAATT (SEQ ID NO: 33) | UUCCUGGAGAACUCUGCAUTT (SEQ ID NO: 157) |
| **ELN-7** | GGAGGTGACCCAGTGATAA (SEQ ID NO: 282) | GGAGGUGACCCAGUGAUAATT (SEQ ID NO: 34) | UUAUCACUGGGUCACCUCCTT (SEQ ID NO: 158) |
| **ELN-8** | GACCCAGTGATAATGGGAA (SEQ ID NO: 283) | GACCCAGUGAUAAUGGGAATT (SEQ ID NO: 35) | UUCCCAUUAUCACUGGGUCTT (SEQ ID NO: 159) |
| **ELN-9** | TGACCCATGCAGAATAGAA (SEQ ID NO: 284) | UGACCCAUGCAGAAUAGAATT (SEQ ID NO: 36) | UUCUAUUCUGCAUGGGUCATT (SEQ ID NO: 160) |
| **ELN-10** | ACAGCACCAACATGGCCTT (SEQ ID NO: 285) | ACAGCACCAACAUGGCCUUTT (SEQ ID NO: 37) | AAGGCCAUGUUGGUGCUGUTT (SEQ ID NO: 161) |
| **ELN-11** | TACCTTCCAGGCCATTCAA (SEQ ID NO: 286) | UACCUUCCAGGCCAUUCAATT (SEQ ID NO: 38) | UUGAAUGGCCUGGAAGGUATT (SEQ ID NO: 162) |
| **COL1A1-1** | CCCAGTTCCACTTCTTCTA (SEQ ID NO: 287) | CCCAGUUCCACUUCUUCUATT (SEQ ID NO: 39) | UAGAAGAAGUGGAACUGGGTT (SEQ ID NO: 163) |
| **COL1A1-2** | TCTCCATTCCAACTCCCAA (SEQ ID NO: 288) | UCUCCAUUCCAACUCCCAATT (SEQ ID NO: 40) | UUGGGAGUUGGAAUGGAGATT (SEQ ID NO: 164) |
| **COL1A1-3** | CTTAGCTCTTGCCAGGATA (SEQ ID NO: 289) | CUUAGCUCUUGCCAGGAUATT (SEQ ID NO: 41) | UAUCCUGGCAAGAGCUAAGTT (SEQ ID NO: 165) |
| **COL1A1-4** | CTCCATTCCAACTCCCAAA (SEQ ID NO: 290) | CUCCAUUCCAACUCCCAAATT (SEQ ID NO: 42) | UUUGGGAGUUGGAAUGGAGTT (SEQ ID NO: 166) |
| **COL1A1-5** | TCTTTATGCCCCTCCCTTA (SEQ ID NO: 291) | UCUUUAUGCCCCUCCCUUATT (SEQ ID NO: 43) | UAAGGGAGGGGCAUAAAGATT (SEQ ID NO: 167) |
| **COL1A1-8** | CTCTTTATGCCCCTCCCTT (SEQ ID NO: 292) | CUCUUUAUGCCCCUCCCUUTT (SEQ ID NO: 44) | AAGGGAGGGGCAUAAAGAGTT (SEQ ID NO: 168) |
| **COL1A1-9** | AACCCTTCCACCTTTGGAA (SEQ ID NO: 293) | AACCCUUCCACCUUUGGAATT (SEQ ID NO: 45) | UUCCAAAGGUGGAAGGGUUTT (SEQ ID NO: 169) |

(continued)

| saRNA | Active saRNA target sequence (5'-3') | Sense sequence (5'-3') | Antisense sequence (5'-3') |
|---|---|---|---|
| **COL1A1-11** | AGATTGGAGGTCCCAGGAA (SEQ ID NO: 294) | AGAUUGGAGGUCCCAGGAATT (SEQ ID NO: 46) | UUCCUGGGACCUCCAAUCUTT (SEQ ID NO: 170) |
| **COL1A2-1** | TGAGCCTCAGCAAAGGCAA (SEQ ID NO: 295) | UGAGCCUCAGCAAAGGCAATT (SEQ ID NO: 47) | UUGCCUUUGCUGAGGCUCATT (SEQ ID NO: 171) |
| **COL1A2-2** | GGTGACTCGGTCTAGTCTA (SEQ ID NO: 296) | GGUGACUCGGUCUAGUCUATT (SEQ ID NO: 48) | UAGACUAGACCGAGUCACCTT (SEQ ID NO: 172) |
| **COL1A2-3** | GCCCTTTCCCGAAGTCATA (SEQ ID NO: 297) | GCCCUUUCCCGAAGUCAUATT (SEQ ID NO: 49) | UAUGACUUCGGGAAAGGGCTT (SEQ ID NO: 173) |
| **COL1A2-4** | AGAGTGCATCACTGCTGAA (SEQ ID NO: 298) | AGAGUGCAUCACUGCUGAATT (SEQ ID NO: 50) | UUCAGCAGUGAUGCACUCUTT (SEQ ID NO: 174) |
| **COL1A2-5** | GAGCCCCAAAGCTAGAGAA (SEQ ID NO: 299) | GAGCCCCAAAGCUAGAGAATT (SEQ ID NO: 51) | UUCUCUAGCUUUGGGGCUCTT (SEQ ID NO: 175) |
| **COL1A2-6** | CCTCAGCAAAGGCAAGCTA (SEQ ID NO: 300) | CCUCAGCAAAGGCAAGCUATT (SEQ ID NO: 52) | UAGCUUGCCUUUGCUGAGGTT (SEQ ID NO: 176) |
| **COL1A2-7** | CTAGTCTAGAGTTCGCAAA (SEQ ID NO: 301) | CUAGUCUAGAGUUCGCAAATT (SEQ ID NO: 53) | UUUGCGAACUCUAGACUAGTT (SEQ ID NO: 177) |
| **COL1A2-8** | CCCTTTCCCGAAGTCATAA (SEQ ID NO: 302) | CCCUUUCCCGAAGUCAUAATT (SEQ ID NO: 54) | UUAUGACUUCGGGAAAGGGTT (SEQ ID NO: 178) |
| **COL1A2-9** | TCCCGAAGTCATAAGACAA (SEQ ID NO: 303) | UCCCGAAGUCAUAAGACAATT (SEQ ID NO: 55) | UUGUCUUAUGACUUCGGGATT (SEQ ID NO: 179) |
| **COL1A2-10** | GCCCCAAAGCTAGAGAAAA (SEQ ID NO: 304) | GCCCCAAAGCUAGAGAAAATT (SEQ ID NO: 56) | UUUUCUCUAGCUUUGGGGCTT (SEQ ID NO: 180) |
| **COL1A2-11** | AGCCCACCTGCCAACAAAA (SEQ ID NO: 305) | AGCCCACCUGCCAACAAAATT (SEQ ID NO: 57) | UUUUGUUGGCAGGUGGGCUTT (SEQ ID NO: 181) |
| **COL1A2-12** | TCCCCAGCCCACCTGCCAA (SEQ ID NO: 306) | UCCCCAGCCCACCUGCCAATT (SEQ ID NO: 58) | UUGGCAGGUGGGCUGGGGATT (SEQ ID NO: 182) |
| **COL3A1-1** | CTGGCAAAATCCCAACATA (SEQ ID NO: 307) | CUGGCAAAAUCCCAACAUATT (SEQ ID NO: 59) | UAUGUUGGGAUUUUGCCAGTT (SEQ ID NO: 183) |
| **COL3A1-3** | CTGTGGGTTGTGTCTTCTA (SEQ ID NO: 308) | CUGUGGGUUGUGUCUUCUATT (SEQ ID NO: 60) | UAGAAGACACAACCCACAGTT (SEQ ID NO: 184) |

(continued)

| saRNA | Active saRNA target sequence (5'-3') | Sense sequence (5'-3') | Antisense sequence (5'-3') |
|---|---|---|---|
| **COL3A1-4** | TCTGGCAAAATCCCAACAT (SEQ ID NO: 309) | UCUGGCAAAAUCCCAACAUTT (SEQ ID NO: 61) | AUGUUGGGAUUUUGCCAGATT (SEQ ID NO: 185) |
| **COL3A1-6** | TTCTGGCAAAATCCCAACA (SEQ ID NO: 310) | UUCUGGCAAAAUCCCAACATT (SEQ ID NO: 62) | UGUUGGGAUUUUGCCAGAATT (SEQ ID NO: 186) |
| **COL3A1-10** | AACTCCAGATGTGCTCTTT (SEQ ID NO: 311) | AACUCCAGAUGUGCUCUUUTT (SEQ ID NO: 63) | AAAGAGCACAUCUGGAGUUTT (SEQ ID NO: 187) |
| **COL3A1-11** | TGTGGGTTGTGTCTTCTAT (SEQ ID NO: 312) | UGUGGGUUGUGUCUUCUAUTT (SEQ ID NO: 64) | AUAGAAGACACAACCCACATT (SEQ ID NO: 188) |
| **COL3A1-12** | GGGTTGTGTCTTCTATAAG (SEQ ID NO: 313) | GGGUUGUGUCUUCUAUAAGTT (SEQ ID NO: 65) | CUUAUAGAAGACACAACCCTT (SEQ ID NO: 189) |
| **COL3A1-13** | CGGCTCTCATATTTCAGAA (SEQ ID NO: 314) | CGGCUCUCAUAUUUCAGAATT (SEQ ID NO: 66) | UUCUGAAAUAUGAGAGCCGTT (SEQ ID NO: 190) |
| **COL3A1-14** | TGCGGCTCTCATATTTCAG (SEQ ID NO: 315) | UGCGGCUCUCAUAUUUCAGTT (SEQ ID NO: 67) | CUGAAAUAUGAGAGCCGCATT (SEQ ID NO: 191) |
| **COL3A1-15** | GATGAGTCGAAGGGGCATA (SEQ ID NO: 316) | GAUGAGUCGAAGGGGCAUATT (SEQ ID NO: 68) | UAUGCCCCUUCGACUCAUCTT (SEQ ID NO: 192) |
| **COL3A1-18** | UAUGCCCCUUCGACUCAUCTT (SEQ ID NO: 317) | AGGAAGATCAGTTCTGTAA (SEQ ID NO: 69) | UUACAGAACUGAUCUUCCUTT (SEQ ID NO: 193) |
| **HAS1-1** | CAAGTGAGGTGGAAGAGAA (SEQ ID NO: 318) | CAAGUGAGGUGGAAGAGAATT (SEQ ID NO: 70) | UUCUCUUCCACCUCACUUGTT (SEQ ID NO: 194) |
| **HAS1-2** | ATAGTGGGTTAGGGAGAAA (SEQ ID NO: 319) | AUAGUGGGUUAGGGAGAAATT (SEQ ID NO: 71) | UUUCUCCCUAACCCACUAUTT (SEQ ID NO: 195) |
| **HAS1-3** | AGAAAGGGATGGAGGGAAA (SEQ ID NO: 320) | AGAAAGGGAUGGAGGGAAATT (SEQ ID NO: 72) | UUUCCCUCCAUCCCUUUCUTT (SEQ ID NO: 196) |
| **HAS1-4** | AAGTGAGGTGGAAGAGAAA (SEQ ID NO: 321) | AAGUGAGGUGGAAGAGAAATT (SEQ ID NO: 73) | UUUCUCUUCCACCUCACUUTT (SEQ ID NO: 197) |
| **HAS1-5** | GAGAAGTGGAGAGGAAGAA (SEQ ID NO: 322) | GAGAAGUGGAGAGGAAGAATT (SEQ ID NO: 74) | UUCUUCCUCUCCACUUCUCTT (SEQ ID NO: 198) |
| **HAS1-6** | GATGGAGAGAAAGACAGAA (SEQ ID NO: 323) | GAUGGAGAGAAAGACAGAATT (SEQ ID NO: 75) | UUCUGUCUUUCUCUCCAUCTT (SEQ ID NO: 199) |

(continued)

| saRNA | Active saRNA target sequence (5'-3') | Sense sequence (5'-3') | Antisense sequence (5'-3') |
|---|---|---|---|
| **HAS1-8** | GAGATGAGGGTACCAACAA (SEQ ID NO: 324) | GAGAUGAGGGUACCAACAATT (SEQ ID NO: 76) | UUGUUGGUACCCUCAUCUCTT (SEQ ID NO: 200) |
| **HAS1-9** | GTGGGTTAGGGAGAAAGAA (SEQ ID NO: 325) | GUGGGUUAGGGAGAAAGAATT (SEQ ID NO: 77) | UUCUUUCUCCCUAACCCACTT (SEQ ID NO: 201) |
| **HAS1-10** | GTGAGGTGGAAGAGAAAAA (SEQ ID NO: 326) | GUGAGGUGGAAGAGAAAAATT (SEQ ID NO: 78) | UUUUUCUCUUCCACCUCACTT (SEQ ID NO: 202) |
| **HAS2-1** | AGGGCACCATTTGGCTTTA (SEQ ID NO: 327) | AGGGCACCAUUUGGCUUUATT (SEQ ID NO: 79) | UAAAGCCAAAUGGUGCCCUTT (SEQ ID NO: 203) |
| **HAS2-2** | TTCCTCCTTGGGTCGGTTA (SEQ ID NO: 328) | UUCCUCCUUGGGUCGGUUATT (SEQ ID NO: 80) | UAACCGACCCAAGGAGGAATT (SEQ ID NO: 204) |
| **HAS2-4** | GCCAGAGGAAAGTAAGGAA (SEQ ID NO: 329) | GCCAGAGGAAAGUAAGGAATT (SEQ ID NO: 81) | UUCCUUACUUUCCUCUGGCTT (SEQ ID NO: 205) |
| **HAS2-7** | TGCACACGTTAGCAATCAA (SEQ ID NO: 330) | UGCACACGUUAGCAAUCAATT (SEQ ID NO: 82) | UUGAUUGCUAACGUGUGCATT (SEQ ID NO: 206) |
| **HAS2-8** | AAGGCTTTGAATGGCCAAT (SEQ ID NO: 331) | AAGGCUUUGAAUGGCCAAUTT (SEQ ID NO: 83) | AUUGGCCAUUCAAAGCCUUTT (SEQ ID NO: 207) |
| **HAS2-9** | TTGAGTTGGCGGGAAGAAA (SEQ ID NO: 332) | UUGAGUUGGCGGGAAGAAATT (SEQ ID NO: 84) | UUUCUUCCCGCCAACUCAATT (SEQ ID NO: 208) |
| **HAS2-12** | ATCGCTTGGAGGGAACTTG (SEQ ID NO: 333) | AUCGCUUGGAGGGAACUUGTT (SEQ ID NO: 85) | CAAGUUCCCUCCAAGCGAUTT (SEQ ID NO: 209) |
| **HAS2-13** | GGAGTTCAGGGAAATCCAA (SEQ ID NO: 334) | GGAGUUCAGGGAAAUCCAATT (SEQ ID NO: 86) | UUGGAUUUCCCUGAACUCCTT (SEQ ID NO: 210) |
| **HAS2-14** | CCCCTATGTGTCTTGCATT (SEQ ID NO: 335) | CCCCUAUGUGUCUUGCAUUTT (SEQ ID NO: 87) | AAUGCAAGACACAUAGGGGTT (SEQ ID NO: 211) |
| **HAS2-15** | CCTATGTGTCTTGCATTTC (SEQ ID NO: 336) | CCUAUGUGUCUUGCAUUUCTT (SEQ ID NO: 88) | GAAAUGCAAGACACAUAGGTT (SEQ ID NO: 212) |
| **HAS2-16** | GCACACGTTAGCAATCAAG (SEQ ID NO: 337) | GCACACGUUAGCAAUCAAGTT (SEQ ID NO: 89) | CUUGAUUGCUAACGUGUGCTT (SEQ ID NO: 213) |
| **HAS2-17** | GTAGCTCAGAGAAGGCTTT (SEQ ID NO: 338) | GUAGCUCAGAGAAGGCUUUIT (SEQ ID NO: 90) | AAAGCCUUCUCUGAGCUACTT (SEQ ID NO: 214) |

(continued)

| saRNA | Active saRNA target sequence (5'-3') | Sense sequence (5'-3') | Antisense sequence (5'-3') |
|---|---|---|---|
| **HAS2-18** | TAGCTCAGAGAAGGCTTTG (SEQ ID NO: 339) | UAGCUCAGAGAAGGCUUUGTT (SEQ ID NO: 91) | CAAAGCCUUCUCUGAGCUATT (SEQ ID NO: 215) |
| **HAS2-19** | GCTCAGAGAAGGCTTTGAA (SEQ ID NO: 340) | GCUCAGAGAAGGCUUUGAATT (SEQ ID NO: 92) | UUCAAAGCCUUCUCUGAGCTT (SEQ ID NO: 216) |
| **HAS3-1** | GGAACCTCCAAGGTTCCTA (SEQ ID NO: 341) | GGAACCUCCAAGGUUCCUATT (SEQ ID NO: 93) | UAGGAACCUUGGAGGUUCCTT (SEQ ID NO: 217) |
| **HAS3-2** | TCGCCTTCAGCTGGTTCTA (SEQ ID NO: 342) | UCGCCUUCAGCUGGUUCUATT (SEQ ID NO: 94) | UAGAACCAGCUGAAGGCGATT (SEQ ID NO: 218) |
| **HAS3-3** | GAGGTCATGGATGTCGGAA (SEQ ID NO: 343) | GAGGUCAUGGAUGUCGGAATT (SEQ ID NO: 95) | UUCCGACAUCCAUGACCUCTT (SEQ ID NO: 219) |
| **HAS3-6** | ACCTCCAAGGTTCCTACAA (SEQ ID NO: 344) | ACCUCCAAGGUUCCUACAATT (SEQ ID NO: 96) | UUGUAGGAACCUUGGAGGUTT (SEQ ID NO: 220) |
| **HAS3-7** | ACTTCTTGTCTCTGCTAAC (SEQ ID NO: 345) | ACUUCUUGUCUCUGCUAACTT (SEQ ID NO: 97) | GUUAGCAGAGACAAGAAGUTT (SEQ ID NO: 221) |
| **HAS3-9** | ACTCGAGTCTAGCACTATC (SEQ ID NO: 346) | ACUCGAGUCUAGCACUAUCTT (SEQ ID NO: 98) | GAUAGUGCUAGACUCGAGUTT (SEQ ID NO: 222) |
| **HAS3-10** | GGACCTGAAAACTACTGTC (SEQ ID NO: 347) | GGACCUGAAAACUACUGUCTT (SEQ ID NO: 99) | GACAGUAGUUUUCAGGUCCTT (SEQ ID NO: 223) |
| **HAS3-11** | GGGAATTTAGGAGGTCATG (SEQ ID NO: 348) | GGGAAUUUAGGAGGUCAUGTT (SEQ ID NO: 100) | CAUGACCUCCUAAAUUCCCTT (SEQ ID NO: 224) |
| **HAS3-12** | TGGCTTTAAGACACCGGAA (SEQ ID NO: 349) | UGGCUUUAAGACACCGGAATT (SEQ ID NO: 101) | UUCCGGUGUCUUAAAGCCATT (SEQ ID NO: 225) |
| **HAS3-13** | AACCTCCAAGGTTCCTACA (SEQ ID NO: 350) | AACCUCCAAGGUUCCUACATT (SEQ ID NO: 102) | UGUAGGAACCUUGGAGGUUTT (SEQ ID NO: 226) |
| **HAS3-14** | CCTCCAAGGTTCCTACAAA (SEQ ID NO: 351) | CCUCCAAGGUUCCUACAAATT (SEQ ID NO: 103) | UUUGUAGGAACCUUGGAGGTT (SEQ ID NO: 227) |
| **HAS3-15** | TCAGCTGGTTCTAAACTTC (SEQ ID NO: 352) | UCAGCUGGUUCUAAACUUCTT (SEQ ID NO: 104) | GAAGUUUAGAACCAGCUGATT (SEQ ID NO: 228) |
| **HAS3-16** | GCTGGTTCTAAACTTCTTG (SEQ ID NO: 353) | GCUGGUUCUAAACUUCUUGTT (SEQ ID NO: 105) | CAAGAAGUUUAGAACCAGCTT (SEQ ID NO: 229) |

(continued)

| saRNA | Active saRNA target sequence (5'-3') | Sense sequence (5'-3') | Antisense sequence (5'-3') |
|---|---|---|---|
| **HAS3-17** | CTCACTTTACTCGAGTCTA (SEQ ID NO: 354) | CUCACUUUACUCGAGUCUATT (SEQ ID NO: 106) | UAGACUCGAGUAAAGUGAGTT (SEQ ID NO: 230) |
| **HAS3-18** | GATAAGGTCAGTTGGGAAT (SEQ ID NO: 355) | GAUAAGGUCAGUUGGGAAUTT (SEQ ID NO: 107) | AUUCCCAACUGACCUUAUCTT (SEQ ID NO: 231) |
| **HAS3-19** | TGGGAATTTAGGAGGTCAT (SEQ ID NO: 356) | UGGGAAUUUAGGAGGUCAUTT (SEQ ID NO: 108) | AUGACCUCCUAAAUUCCCATT (SEQ ID NO: 232) |
| **HAS3-20** | GTCATGGATGTCGGAAATA (SEQ ID NO: 357) | GUCAUGGAUGUCGGAAAUATT (SEQ ID NO: 109) | UAUUUCCGACAUCCAUGACTT (SEQ ID NO: 233) |
| **MFAP2-1** | GAGGAATGCTTGAGGCCAA (SEQ ID NO: 358) | GAGGAAUGCUUGAGGCCAATT (SEQ ID NO: 110) | UUGGCCUCAAGCAUUCCUCTT (SEQ ID NO: 234) |
| **MFAP2-2** | CCTGTCTCTAGAAAAGGAA (SEQ ID NO: 359) | CCUGUCUCUAGAAAAGGAATT (SEQ ID NO: 111) | UUCCUUUUCUAGAGACAGGTT (SEQ ID NO: 235) |
| **MFAP2-3** | AGACCCTGTCTCTAGAAAA (SEQ ID NO: 360) | AGACCCUGUCUCUAGAAAATT (SEQ ID NO: 112) | UUUUCUAGAGACAGGGUCUTT (SEQ ID NO: 236) |
| **MFAP2-6** | AAGACCCTGTCTCTAGAAA (SEQ ID NO: 361) | AAGACCCUGUCUCUAGAAATT (SEQ ID NO: 113) | UUUCUAGAGACAGGGUCUUTT (SEQ ID NO: 237) |
| **MFAP2-7** | TGATTTCCTCCTCTGTGAA (SEQ ID NO: 362) | UGAUUUCCUCCUCUGUGAATT (SEQ ID NO: 114) | UUCACAGAGGAGGAAAUCATT (SEQ ID NO: 238) |
| **MFAP2-8** | CAAGACCCTGTCTCTAGAA (SEQ ID NO: 363) | CAAGACCCUGUCUCUAGAATT (SEQ ID NO: 115) | UUCUAGAGACAGGGUCUUGTT (SEQ ID NO: 239) |
| **MFAP2-9** | TGGAGAGGCAGAAGGAAAA (SEQ ID NO: 364) | UGGAGAGGCAGAAGGAAAATT (SEQ ID NO: 116) | UUUUCCUUCUGCCUCUCCATT (SEQ ID NO: 240) |
| **MFAP2-10** | TCCTCTGTGAAATGGGCAA (SEQ ID NO: 365) | UCCUCUGUGAAAUGGGCAATT (SEQ ID NO: 117) | UUGCCCAUUUCACAGAGGATT (SEQ ID NO: 241) |
| **MFAP2-14** | AGAGGGAGATCGAGGCTTA (SEQ ID NO: 366) | AGAGGGAGAUCGAGGCUUATT (SEQ ID NO: 118) | UAAGCCUCGAUCUCCCUCUTT (SEQ ID NO: 242) |
| **MFAP2-15** | GTCACTTTGGGAGGCTTGA (SEQ ID NO: 367) | GUCACUUUGGGAGGCUUGATT (SEQ ID NO: 119) | UCAAGCCUCCCAAAGUGACTT (SEQ ID NO: 243) |
| **MFAP2-16** | GATTTCCTCCTCTGTGAAA (SEQ ID NO: 368) | GAUUUCCUCCUCUGUGAAATT (SEQ ID NO: 120) | UUUCACAGAGGAGGAAAUCTT (SEQ ID NO: 244) |

(continued)

| saRNA | Active saRNA target sequence (5'-3') | Sense sequence (5'-3') | Antisense sequence (5'-3') |
|---|---|---|---|
| **MFAP2-17** | CTGAAAGAGGTGCTGGTTA (SEQ ID NO: 369) | CUGAAAGAGGUGCUGGUUATT (SEQ ID NO: 121) | UAACCAGCACCUCUUUCAGTT (SEQ ID NO: 245) |
| **MFAP2-18** | CTCATGCCTGTAATCCCAA (SEQ ID NO: 370) | CUCAUGCCUGUAAUCCCAATT (SEQ ID NO: 122) | UUGGGAUUACAGGCAUGAGTT (SEQ ID NO: 246) |
| **MFAP2-19** | GACCCTGTCTCTAGAAAAG (SEQ ID NO: 371) | GACCCUGUCUCUAGAAAAGTT (SEQ ID NO: 123) | CUUUUCUAGAGACAGGGUCTT (SEQ ID NO: 247) |
| **MFAP2-20** | GAGGCAGAAGGAAAACCAA (SEQ ID NO: 372) | GAGGCAGAAGGAAAACCAATT (SEQ ID NO: 124) | UUGGUUUUCCUUCUGCCUCTT (SEQ ID NO: 248) |

Example 4: saRNAs Promoting Cells to Secrete Hyaluronic Acid

In order to assay whether saRNAs can promote cells to produce hyaluronic acid, saRNAs which can up-regulate the mRNA expression of HAS1, HAS2, and HAS3 were selected to transfect Hs27 cells respectively. 72 h later, the cell culture media were collected, and the contents of hyaluronic acid in the cell culture media were detected using a hyaluronic acid DuoSet kit (R&D systems, product number: DY3614-05). The kit can detect hyaluronic acid proteins encoded by all the three hyaluronic acid genes. The optical densities of the samples were read at a wavelength of 450 nm by a multimode microplate reader (Infinite M200 Pro, Tecan). The optical densities of the samples were read at a wavelength of 450 nm using hyaluronic acid with a known concentration to generate a standard curve. At the same time, the tested cells were collected, and the total protein amounts of the corresponding cells were respectively measured by Pierce Protein Assay (Thermo Scientific). Finally, the content of hyaluronic acid was calculated according to the standard curve and corrected by the total protein amount of the cells of each treated sample.

The hyaluronic acid production promotion rate was calculated by taking the content of hyaluronic acid of control treatment (Mock) cells as 100%. The formula is as follows:

$$\text{hyaluronic acid production promotion rate } (\%) = X/M \times 100$$

In the aforementioned formula, X represents the content of hyaluronic acid of the saRNA-treated sample, and M represents the content of hyaluronic acid of the control treatment (Mock) sample. The assay results are consistent with the RT-qPCR results, suggesting that all the tested saRNAs promote the cells to secrete hyaluronic acid into the media to different degrees (FIG. 12). These results clearly indicate that the saRNAs can enter the cells to activate the expression of HAS gene, thus promoting the cells to secrete hyaluronic acid into the extracellular environment.

Example 5: saRNAs Promoting Fibroblasts to Secrete Collagen

**(1) Quantitative detection method for type I collagen**

In order to detect the amount of synthesized type I collagen in cells, tropocollagen in cell culture media was detected using a procollagen type I C-peptide ELISA kit (Takara, product number: MK101) according to the method provided by the manufacturer. The amount of each culture medium was 20 μL. Standard was prepared with tropocollagen with a known concentration. The optical densities of the samples to be tested were read at a wavelength of 450 nm using tropocollagen with a known concentration by a multimode microplate reader (Infinite M200 Pro, Tecan). At the same time, the cells were collected, and the total protein amount of the corresponding cells was measured by Pierce Protein Assay (Thermo Scientific). The content of collagen was calculated according to the standard curve and corrected by the total protein amount of the cells of each treated sample. The type I collagen production promotion rate was calculated by taking the amount of type I collagen of control treatment (Mock) cells as 100%. The calculation formula is as follows:

$$\text{type I collagen production promotion rate } (\%) = X/M \times 100$$

In the aforementioned formula, X represents the amount of type I collagen of the saRNA-treated sample, and M represents the amount of type I collagen of the control treatment (Mock) sample.

**(2) Quantitative detection method for type III collagen**

In order to detect the content of type III collagen in cell culture media, the saRNAs to be tested were respectively transfected into cells cultured in a 6-well plate. 72 h later, the media were collected and detected using a human collagen type III α1 (COL3A1) ELISA Kit for type III collagen (MyBioSource, Inc, San Diego, USA) according to the method provided by the manufacturer. At the same time, the cells were collected, and the total protein amount of the corresponding cells was measured by Pierce Protein Assay (Thermo Scientific). Optical densities were read at a wavelength of 450 nm by a multifunctional microplate detector (Infinite M200 Pro, Tecan), and the content of collagen was calculated according to the standard curve and corrected by the total protein amount of each treated sample.

In order to verify whether the activation of the collagen genes COL1A1, COL1A2 and COL3A1 mediated by the saRNAs led to the secretion of functional proteins, the content of corresponding collagens in the fibroblast culture media transfected by the saRNAs was detected by the aforementioned ELISA method. The Hs27 cells and NHDF cells was transfected with COL1A1-specific saRNAs (COL1A1-2, COL1A1-5 and COL1A1-8) respectively at final concentrations of 20 nM and 50 nM. 72 h later, the content of type I tropocollagen in the cell culture media was detected using a procollagen type I C-peptide ELISA kit (Takara, product number: MK101). As shown in Table 4, the contents of type I collagen in the media of the Hs27 cells and NHDF cells treated by COL1A1-2, COL1A1-5 and COL1A1-8 increased in a dose-effect relationship, while the control duplex RNA (dsControl) had no obvious effect.

Table 4: Effect of COL1A1 saRNAs on amounts of type I tropocollagen secreted by Hs27 cells and NHDF cells

| Treatment | Concentration (nM) | Change relative to control (Mock) treatment (mean ± SD) | |
|---|---|---|---|
| | | Hs27 | NHDF |
| dsControl | 20 | 94.4% ± 4% | 98.4% ± 4% |
| dsControl | 50 | 95.8% ± 1% | 97.0% ± 1% |
| COL1A1-2 | 20 | 123.5% ± 3% | 127.3% ± 2% |
| COL1A1-2 | 50 | 143.4% ± 5% | 134.3% ± 5% |
| COL1A1-5 | 20 | 148.5% ± 15% | 134.1% ± 11% |
| COL1A1-5 | 50 | 169.1% ± 10% | 145.6% ± 4% |
| COL1A1-8 | 20 | 122.5% ± 2% | 114.8% ± 5% |
| COL1A1-8 | 50 | 132.7% ± 2% | 140.8% ± 15% |

Type I tropocollagen in fibroblast (Hs27 and NHDF) culture media transfected by COL1A2-specific saRNAs (COL1A2-1, COL1A2-2, COL1A2-3, COL1A2-4, COL1A2-6, COL1A2-9 and COL 1A2-12) was detected using the same ELISA kit. It was found that at a transfection concentration of 50 nM and with a transfection time of 72 h, all these saRNAs increased the amount of tropocollagen secreted by the two types of cells, while the control duplex RNA (dsControl) had no obvious effect (Table 5). These results indicate that the saRNAs not only can up-regulate the mRNA expression of the collagen genes, but also can increase the amount of type I tropocollagen secreted by the cells to different degrees.

Table 5: Effect of COL1A2 saRNAs on amounts of type I tropocollagen secreted by Hs27 cells and NHDF cells

| Treatment | Concentration (nM) | Change relative to control (Mock) treatment (mean ± SD) | |
|---|---|---|---|
| | | Hs27 | NHDF |
| dsControl | 50 | 94.4% ± 2% | 96.5% ± 2% |
| COL1A2-1 | 50 | 188.5% ± 10% | 206.8% ± 6% |
| COL1A2-2 | 50 | 249.2% ± 15% | 208.9% ± 5% |
| COL1A2-3 | 50 | 126.2% ± 2% | 119.2% ± 1% |
| COL1A2-4 | 50 | 166.1% ± 3% | 116.6% ± 5% |
| COL1A2-6 | 50 | 186.0% ± 3% | 129.8% ± 3% |
| COL1A2-9 | 50 | 119.4% ± 5% | 151.2% ± 1% |
| COL1A2-12 | 50 | 120.4% ± 5% | 128.3% ± 5% |

Type III tropocollagen in fibroblast (Hs27 and NHDF) culture media transfected by COL3A1-specific saRNAs (COL3A1-1, COL3A1-3, COL3A1-6, COL3A1-13 and COL3A1-14) was detected by using the aforementioned human collagen type III $\alpha$1 (COL3A1) ELISA Kit for type III collagen. The result shows that at a transfection concentration of 50 nM, and with a transfection time of 72 h, all these saRNAs increased the amount of tropocollagen secreted by the two types of cells, while the control duplex RNA (dsControl) had no obvious effect (Table 6). These results indicate that the saRNAs not only can up-regulate the mRNA expression of the COL3A1 gene, but also can significantly increase the amount of type III tropocollagen secreted by the cells.

Table 6: Effect of COL3A1 saRNAs on amounts of type III tropocollagen secreted by Hs27 cells and NHDF cells

| Treatment | Concentration (nM) | Change relative to control (Mock) treatment (%) | |
|---|---|---|---|
| | | Hs27 | NHDF |
| dsControl | 50 | 93.9% ± 1% | 101.5% ± 3% |
| COL3A1-1 | 50 | 174.5% ± 16% | 198.8% ± 21% |
| COL3A1-3 | 50 | 209.3% ± 19% | 238.6% ± 25% |
| COL3A1-6 | 50 | 299.0% ± 18% | 250.7% ± 6% |
| COL3A1-13 | 50 | 151.4% ± 2% | 143.0% ± 1% |
| COL3A1-14 | 50 | 199.3% ± 4% | 139.9% ± 6% |

## Claims

1. A small activating nucleic acid molecule, wherein one fragment of the small activating nucleic acid molecule has at least 75% homology or complementarity to any continuous fragment of 16 to 35 nucleotides in length in the promoter of a human AQP3, AQP9, ELN, COL1A1, COL1A2, COL3A1, HAS1, HAS2, HAS3 or MFAP2 gene, and the small activating nucleic acid molecule activates/upregulates the expression of the AQP3, AQP9, ELN, COL1A1, COL1A2, COL3A1, HAS1, HAS2, HAS3 or MFAP2 gene.

2. The small activating nucleic acid molecule of claim 1, wherein the small activating nucleic acid molecule comprises a sense nucleic acid fragment and an antisense nucleic acid fragment, the sense nucleic acid fragment and the antisense nucleic acid fragment comprise complementary regions, the complementary regions can form a double-stranded nucleic acid structure, and the double-stranded nucleic acid can activate or upregulate the expression of the human AQP3, AQP9, ELN, COL1A1, COL1A2, COL3A1, HAS1, HAS2, HAS3 or MFAP2 gene in a cell.

3. The small activating nucleic acid molecule of claim 2, wherein the sense nucleic acid fragment and the antisense nucleic acid fragment of the small activating nucleic acid molecule are present on two different nucleic acid strands.

4. The small activating nucleic acid molecule of claim 2, wherein the sense nucleic acid fragment and the antisense nucleic acid fragment of the small activating nucleic acid molecule are present on one nucleic acid strand, preferably the small activating nucleic acid molecule is a hairpin single-stranded nucleic acid molecule, wherein the complementary regions of the sense nucleic acid fragment and the antisense nucleic acid fragment form a double-stranded nucleic acid structure.

5. The small activating nucleic acid molecule of claim 3, wherein at least one strand of the small activating nucleic acid molecule has a 3' overhang of 0 to 6 nucleotides in length, and preferably, both strands of the small activating nucleic acid molecule have 3' overhangs of 2 or 3 nucleotides in length.

6. The small activating nucleic acid molecule of claim 1, wherein the lengths of the sense nucleic acid fragment and the antisense nucleic acid fragment of the small activating nucleic acid molecule are 16 to 35 nucleotides.

7. The small activating nucleic acid molecule of claim 4, wherein the small activating nucleic acid molecule is operably linked to a vector, and preferably, the vector includes a plasmid vector, a lentiviral vector, an adenovirus vector or an adeno-associated virus vector.

**8.** The small activating nucleic acid molecule of claim 1, wherein one strand of the small activating nucleic acid molecule has at least 75% homology or complementarity to any nucleotide sequence selected from SEQ ID NOs: 249-372.

**9.** The small activating nucleic acid molecule of claim 2, wherein the sense strand of the small activating nucleic acid molecule has at least 75% homology to any nucleotide sequence selected from SEQ ID NOs: 1-124, and the antisense strand of the small activating nucleic acid molecule has at least 75% homology to any nucleotide sequence selected from SEQ ID NOs: 125-248.

**10.** The small activating nucleic acid molecule of claim 2, wherein the sense strand of the small activating nucleic acid molecule comprises or is selected from any of nucleotide sequences set forth in SEQ ID NOs: 1-124, and the antisense strand of the small activating nucleic acid molecule comprises or is selected from any of nucleotide sequences set forth in SEQ ID NOs: 125-248.

**11.** The small activating nucleic acid molecule of any of claims 1-6 or 8-10, wherein the small activating nucleic acid molecule comprises at least one chemically modified nucleotide.

**12.** The small activating nucleic acid molecule of claim 11, wherein the chemical modification is at least one of the following modifications:

(1) modification of a phosphodiester bond connecting nucleotides in the nucleotide sequence of the small activating nucleic acid molecule;
(2) modification of 2'-OH of a ribose in the nucleotide sequence of the small activating nucleic acid molecule;
(3) modification of a base in the nucleotide sequence of the small activating nucleic acid molecule; and
(4) at least one nucleotide in the nucleotide sequence of the small activating nucleic acid molecule being a locked nucleic acid.

**13.** A nucleic acid coding the small activating nucleic acid molecule of any of claims 1-12.

**14.** The nucleic acid of claim 13, wherein the nucleic acid is a DNA molecule.

**15.** A cell comprising the small activating nucleic acid molecule of any of claims 1-12 or the nucleic acid of claim 13 or 14.

**16.** A composition, comprising an agonist interfering with the expression and function of at least one gene selected from the following group: human AQP3, AQP9, ELN, COL1A1, COL1A2, COL3A1, HAS1, HAS2, HAS3 and MFAP2 genes.

**17.** The composition of claim 16, wherein the agonist is one or more of the small activating nucleic acid molecules of claims 1-12 or one or more of the nucleic acids of claim 13 or claim 14.

**18.** The composition of claim 16 or 17, wherein the composition further comprises a carrier acceptable to cosmetics/skin-care products/beauty products.

**19.** The composition of claim 18, wherein the carrier acceptable to the cosmetics/skin-care products/beauty products includes an aqueous carrier, a liposome, a macromolecular polymer or a polypeptide.

**20.** The composition of claim 19, wherein the aqueous carrier comprises RNase-free water.

**21.** The composition of claim 18, wherein the composition comprises 1 nM to 100 nM, preferably 1 nM to 50 nM of the small activating nucleic acid molecule.

**22.** A use of the agonist interfering with the expression and function of at least one gene selected from the following group in preparing a skin-care composition: human AQP3, AQP9, ELN, COL1A1, COL1A2, COL3A1, HAS1, HAS2, HAS3 and MFAP2 genes.

**23.** The use of claim 22, wherein the agonist comprises one or more of the small activating nucleic acid molecules of claims 1-12 or one or more of the nucleic acids of claim 13 or claim 14.

**24.** A use of one or more of the small activating nucleic acid molecules of claims 1-12, one or more of the nucleic acids of claim 13 or claim 14 or the composition of any of claims 16-21 in preparing a formulation for activating/upregulating the expression of one or more of the human AQP3, AQP9, ELN, COL1A1, COL1A2, COL3A1, HAS1, HAS2, HAS3 and MFAP2 genes in a cell.

**25.** The use of claim 24, wherein one or more of the small activating nucleic acid molecules of claims 1-12, one or more of the nucleic acids of claim 13 or 14 or the composition of any of claims 16-21 are directly introduced into the cell.

**26.** The use of claim 24, wherein the small activating nucleic acid molecule is produced in the cell after a nucleotide sequence coding the small activating nucleic acid molecule is introduced into the cell.

**27.** The use of any of claims 24-26, wherein the cell is a human cell.

**28.** The use of any of claims 24-26, wherein the cell is present in a human body.

**29.** A method for activating/upregulating the expression of one or more of the human AQP3, AQP9, ELN, COL1A1, COL1A2, COL3A1, HAS1, HAS2, HAS3 and MFAP2 genes in a cell, comprising administering one or more of the small activating nucleic acid molecules of claims 1-12, one or more of the nucleic acids of claim 13 or claim 14 or the composition of any of claims 16-21 to the cell.

**30.** The method of claim 29, wherein the small activating nucleic acid molecule of any of claims 1-12, the nucleic acid of claim 13 or claim 14 or the composition of any of claims 16-21 is directly introduced into the cell.

**31.** The method of claim 30, wherein the small activating nucleic acid molecule is produced in the cell after a nucleotide sequence coding the small activating nucleic acid molecule is introduced into the cell.

**32.** The method of any of claims 29-31, wherein the cell is a human cell.

**33.** A method for caring skin or improving skin conditions, comprising administering one or more of the small activating nucleic acid molecules of claims 1-12, one or more of the nucleic acids of claim 13 or 14 or the composition of any of claims 16-21 to an individual.

**34.** The method of claim 33, wherein the individual is a mammal, and preferably, the individual is a human.

**35.** The method of claim 33 or claim 34, wherein improving skin conditions comprises increasing or restoring skin elasticity, improving skin sagging, increasing the moisture content of the skin, reducing or eliminating skin wrinkles and preventing the appearance of fine lines and dry lines.

**36.** An isolated small activating nucleic acid molecule target sites, wherein the target site comprises or is selected from any continuous sequence of 16 to 35 nucleotides on any of sequences set forth in SEQ ID NOs: 249-372.

**37.** The small activating nucleic acid molecule target site of claim 36, wherein the target site comprises or is selected from any of nucleotide sequences set forth in SEQ ID NOs: 249-372.

*FIG. 1*

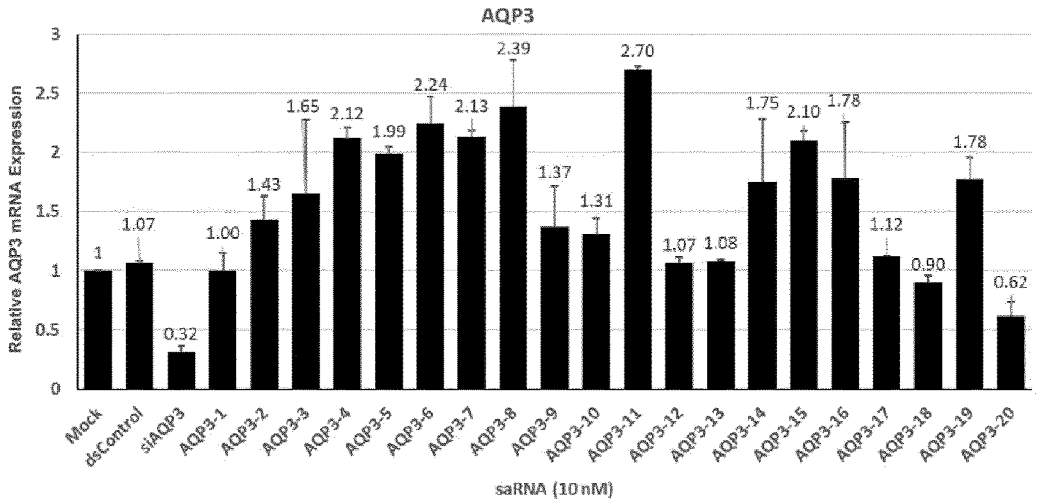
AQP3
Relative AQP3 mRNA Expression
3
2.5
2
1.5
1
0.5
0
1
1.07
0.32
1.00
1.43
1.65
2.12
1.99
2.24
2.13
2.39
1.37
1.31
2.70
1.07
1.08
1.75
2.10
1.78
1.12
0.90
1.78
0.62
Mock
dsControl
siAQP3
AQP3-1
AQP3-2
AQP3-3
AQP3-4
AQP3-5
AQP3-6
AQP3-7
AQP3-8
AQP3-9
AQP3-10
AQP3-11
AQP3-12
AQP3-13
AQP3-14
AQP3-15
AQP3-16
AQP3-17
AQP3-18
AQP3-19
AQP3-20
saRNA (10 nM)

*FIG. 2*

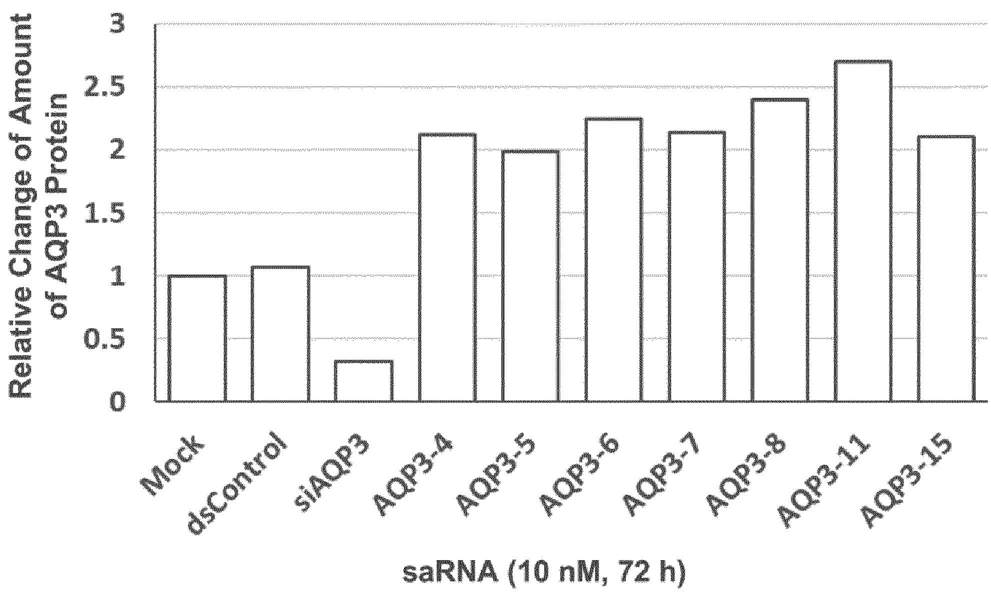
Relative Change of Amount of AQP3 Protein
3
2.5
2
1.5
1
0.5
0
Mock
dsControl
siAQP3
AQP3-4
AQP3-5
AQP3-6
AQP3-7
AQP3-8
AQP3-11
AQP3-15
saRNA (10 nM, 72 h)

FIG. 3

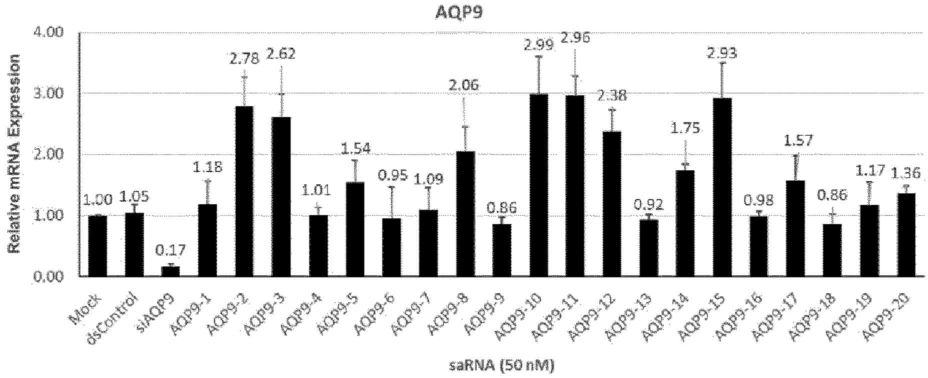


FIG. 4A

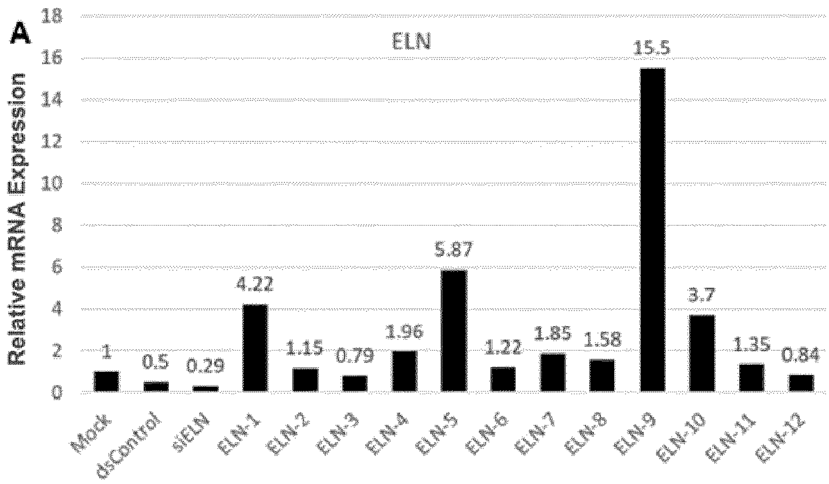


FIG. 4B

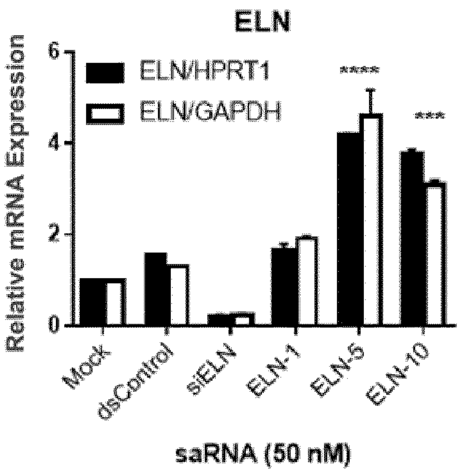


FIG. 4C

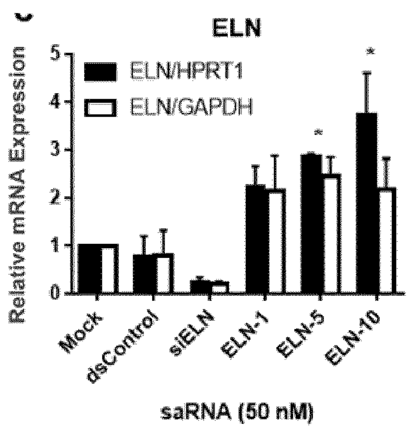

*FIG. 5A*

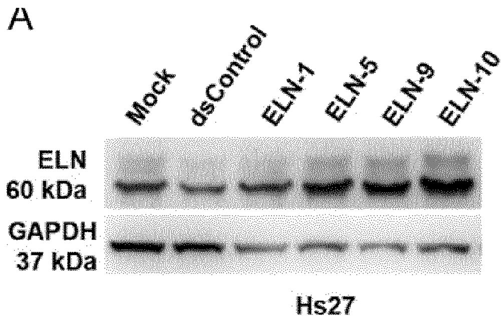


*FIG. 5B*

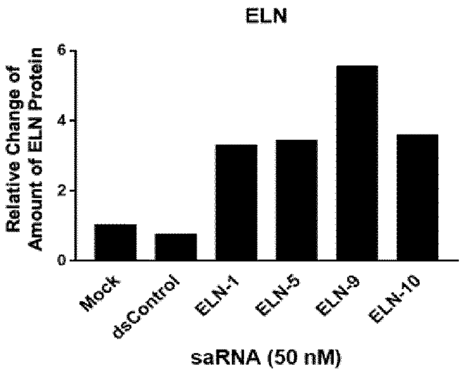


*FIG. 6*

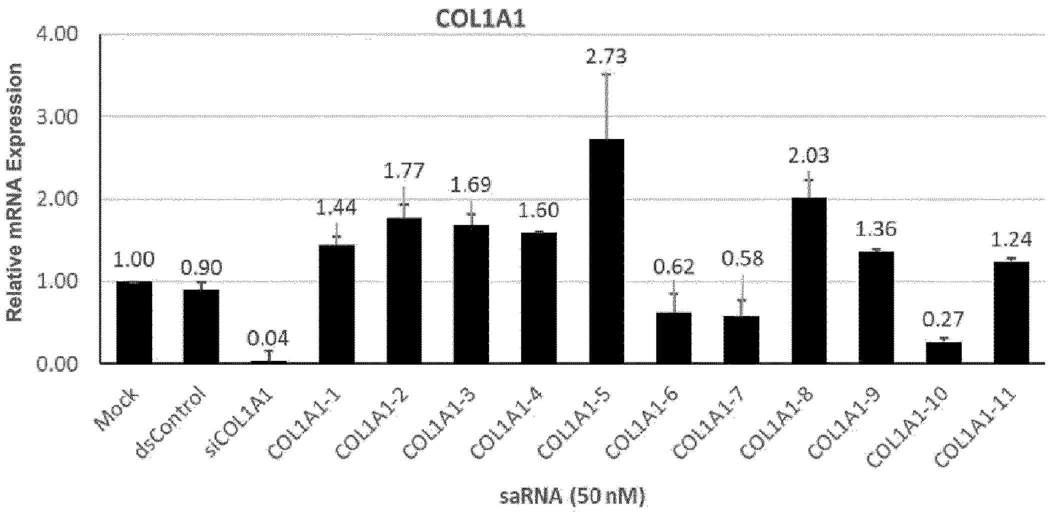

*FIG. 7*

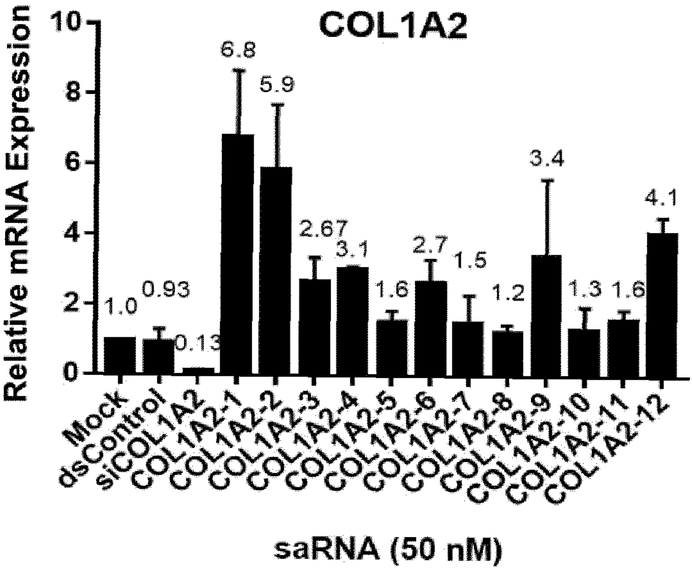
COL1A2
Relative mRNA Expression
10
8
6
4
2
0
1.0
0.93
0.13
6.8
5.9
2.67
3.1
1.6
2.7
1.5
1.2
3.4
1.3
1.6
4.1
Mock
dsControl
siCOL1A2
COL1A2-1
COL1A2-2
COL1A2-3
COL1A2-4
COL1A2-5
COL1A2-6
COL1A2-7
COL1A2-8
COL1A2-9
COL1A2-10
COL1A2-11
COL1A2-12
saRNA (50 nM)

*FIG. 8*

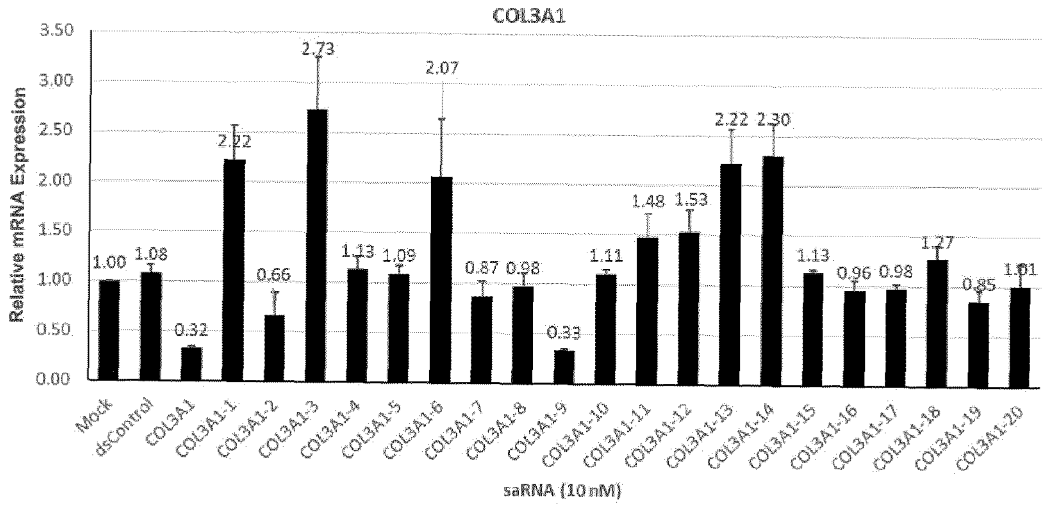
COL3A1
Relative mRNA Expression
3.50
3.00
2.50
2.00
1.50
1.00
0.50
0.00
1.00
1.08
0.32
2.22
0.66
2.73
1.13
1.09
2.07
0.87
0.98
0.33
1.11
1.48
1.53
2.22
2.30
1.13
0.96
0.98
1.27
0.85
1.01
Mock
dsControl
COL3A1
COL3A1-1
COL3A1-2
COL3A1-3
COL3A1-4
COL3A1-5
COL3A1-6
COL3A1-7
COL3A1-8
COL3A1-9
COL3A1-10
COL3A1-11
COL3A1-12
COL3A1-13
COL3A1-14
COL3A1-15
COL3A1-16
COL3A1-17
COL3A1-18
COL3A1-19
COL3A1-20
saRNA (10 nM)

FIG. 9

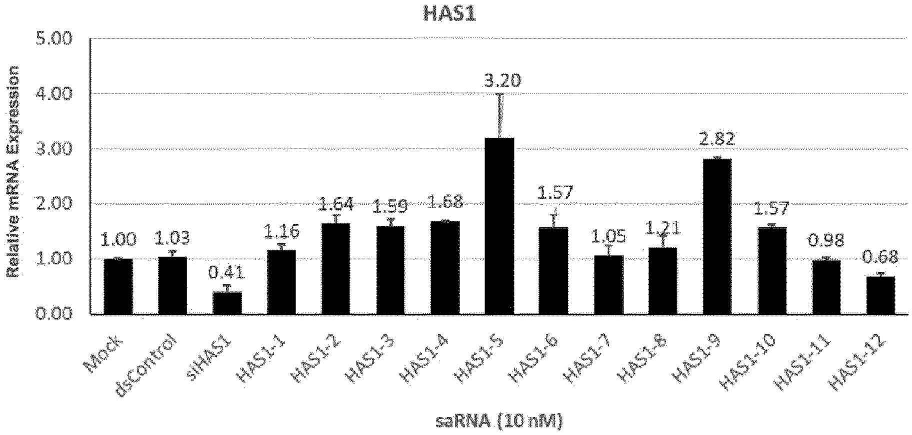


FIG. 10

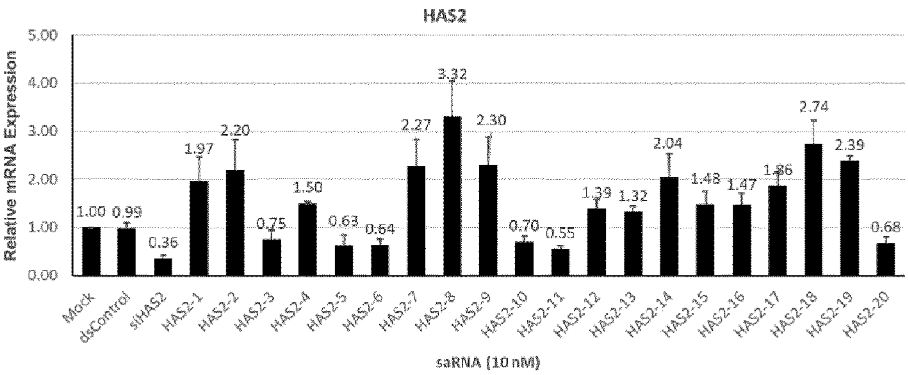


FIG. 11

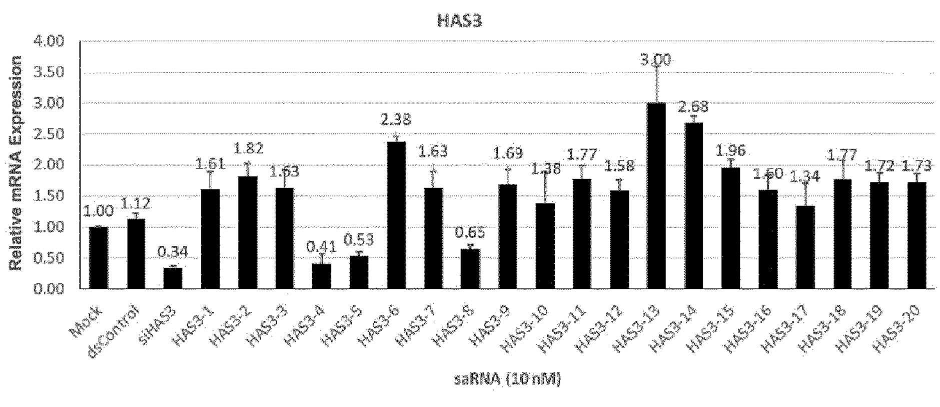

*FIG. 12*

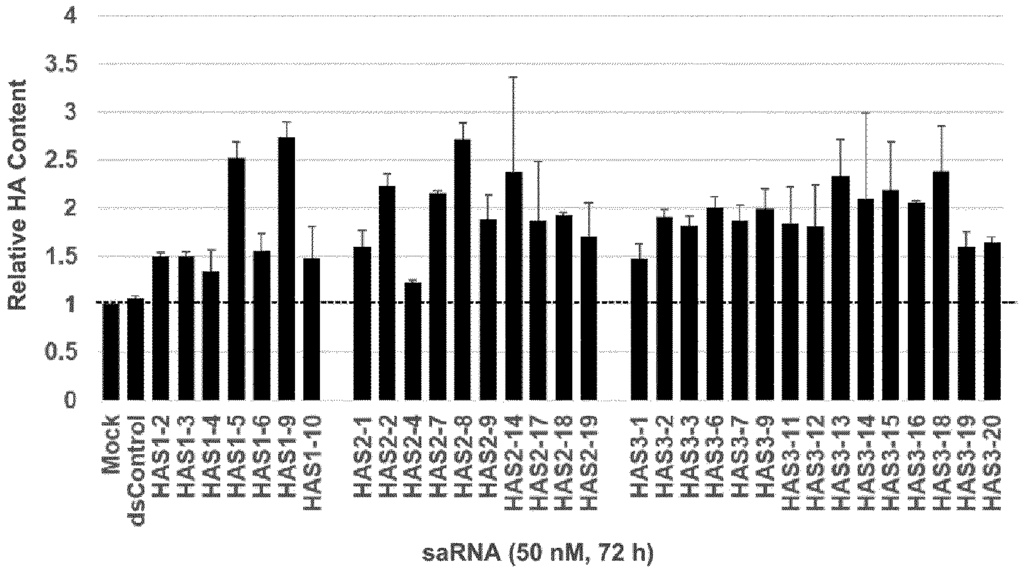
Relative HA Content
4
3.5
3
2.5
2
1.5
1
0.5
0
Mock
dsControl
HAS1-2
HAS1-3
HAS1-4
HAS1-5
HAS1-6
HAS1-9
HAS1-10
HAS2-1
HAS2-2
HAS2-4
HAS2-7
HAS2-8
HAS2-9
HAS2-14
HAS2-17
HAS2-18
HAS2-19
HAS3-1
HAS3-2
HAS3-3
HAS3-6
HAS3-7
HAS3-9
HAS3-11
HAS3-12
HAS3-13
HAS3-14
HAS3-15
HAS3-16
HAS3-18
HAS3-19
HAS3-20
saRNA (50 nM, 72 h)

*FIG. 13*

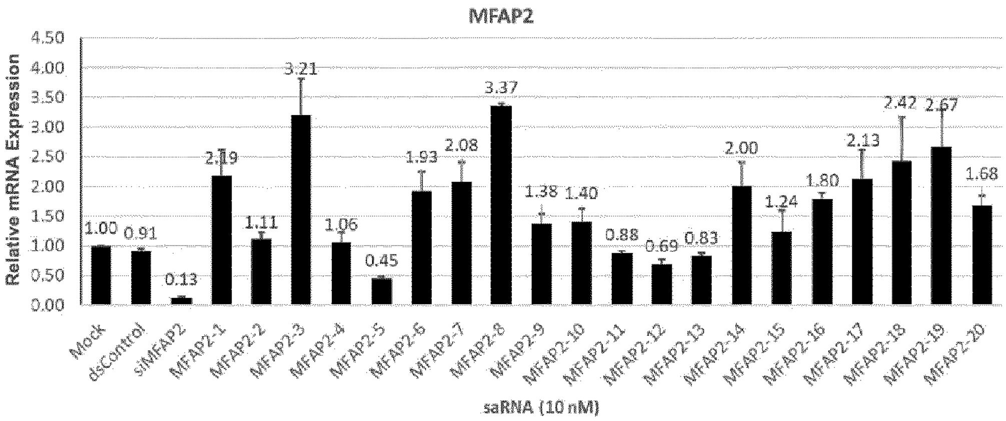
MFAP2
Relative mRNA Expression
4.50
4.00
3.50
3.00
2.50
2.00
1.50
1.00
0.50
0.00
1.00
0.91
0.13
2.19
1.11
3.21
1.06
0.45
1.93
2.08
3.37
1.38
1.40
0.88
0.69
0.83
2.00
1.24
1.80
2.13
2.42
2.67
1.68
Mock
dsControl
siMFAP2
MFAP2-1
MFAP2-2
MFAP2-3
MFAP2-4
MFAP2-5
MFAP2-6
MFAP2-7
MFAP2-8
MFAP2-9
MFAP2-10
MFAP2-11
MFAP2-12
MFAP2-13
MFAP2-14
MFAP2-15
MFAP2-16
MFAP2-17
MFAP2-18
MFAP2-19
MFAP2-20
saRNA (10 nM)

**INTERNATIONAL SEARCH REPORT**

International application No.
**PCT/CN2020/073663**

**A. CLASSIFICATION OF SUBJECT MATTER**

C12N 15/113(2010.01)i; A61K 48/00(2006.01)i; A61K 31/713(2006.01)i; A61P 17/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, CNKI, 百度学术, BAIDU SCHOLAR, ISI-WEB OF SCIENCE, Genbank+EMBL+DDBJ, 中国专利生物序列检索系统, China Patents Biological Sequence Search System: 小激活核酸, 小激活RNA, 短激活核酸, 短激活RNA, 水通道蛋白, 皮肤, small activating nucleic acid, small activating RNA, saRNA, short activating nucleic acid, short activating RNA, aquaporin, AQP, skin, 对序列SEQ ID NO:249的检索, search for SEQ ID NO: 249

**C. DOCUMENTS CONSIDERED TO BE RELEVANT**

| Category* | Citation of document, with indication, where appropriate, of the relevant passages | Relevant to claim No. |
|---|---|---|
| A | CN 106032532 A (PEKING UNION MEDICAL COLLEGE HOSPITAL, CHINESE ACADEMY OF MEDICAL SCIENCES) 19 October 2016 (2016-10-19)<br>see abstract, description, paragraphs [0005]-[0038] | 1-8 (partially), 11-28 (partially), 36 and 37 (partially) |
| A | WO 2015162422 A1 (MINA THERAPEUTICS LTD) 29 October 2015 (2015-10-29)<br>see entire document | 1-8 (partially), 11-28 (partially), 36 and 37 (partially) |
| A | 陈忠等 (CHEN, Zhong et al.). "RNA激活研究进展 (Non-official translation: Recent Advances in RNA Activation)"<br>*现代泌尿生殖肿瘤杂志 (Journal of Contemporary Urologic and Reproductive Oncology),* Vol. 4, No. 2,, 22 April 2012 (2012-04-22),<br>ISSN: 1674-4624,<br>see pages 65-67 | 1-8 (partially), 11-28 (partially), 36 and 37 (partially) |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 April 2020** | **24 April 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
**PCT/CN2020/073663**

**C. DOCUMENTS CONSIDERED TO BE RELEVANT**

| Category* | Citation of document, with indication, where appropriate, of the relevant passages | Relevant to claim No. |
|---|---|---|
| A | HUANG, V. et al. "RNAa Is Conserved in Mammalian Cells" *PLOS ONE,* Vol. 5, No. 1, 22 January 2010 (2010-01-22), ISSN: 1932-6203, see document number: e8848, pages 1-7 | 1-8 (partially), 11-28 (in part), 36 and 37 (partially) |

Form PCT/ISA/210 (second sheet) (January 2015)

INTERNATIONAL SEARCH REPORT

International application No.
PCT/CN2020/073663

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29-35**
because they relate to subject matter not required to be searched by this Authority, namely:

[1] Claims 29-35 relate to a treatment method of a human or animal body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Inventions 1-124: claims 1-7 (partially), 8, 11-35 (partially), 36 and 37 respectively relate to a strand, and a small activated nucleic acid molecule with at least 75% homology or complementarity with any nucleotide sequence selected from SEQ ID NO: 249-372, or a small activated nucleic acid molecule target site point including or selected from any nucleotide sequence of SEQ ID NO: 249-372 or any consecutive 16-35 nucleotide sequences on it;

[2] Inventions 125-248: claims 1-7 (partially), and 9-35 (partially) respectively relate to a sense strand, and any nucleotide sequence including or selected from SEQ ID NO: 1-124 or a small activated nucleic acid molecule with at least 75% homology with it;

[3] Inventions 249-372: claims 1-7 (partially), and 9-35 (partially) respectively relate to an antisense strand, and any nucleotide sequence including or selected from SEQ ID NO: 125-248 or a small activated nucleic acid molecule with at least 75% homology with it;

[4] The small activated nucleic acid molecule or the small activated nucleic acid molecule target site point related to in inventions 1-372 are different from one another in sequence structure and the action sites of the biological function thereof are also different from one another, thus the above-mentioned inventions do not share the same or corresponding special technical feature, are not linked technically, do not fall within a single general inventive concept, and do not comply with PCT Rule 13.1, 13.2 and 13.3.

Form PCT/ISA/210 (continuation of first sheet) (January 2015)

**INTERNATIONAL SEARCH REPORT**

International application No.
**PCT/CN2020/073663**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-7 (partially), 8, and 11-35 (partially), and the part related to SEQ ID NO: 249 in claims 36 and 37.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2020/073663**

| Patent document cited in search report | | | Publication date (day/month/year) | Patent family member(s) | | | Publication date (day/month/year) |
|---|---|---|---|---|---|---|---|
| CN | 106032532 | A | 19 October 2016 | None | | | |
| WO | 2015162422 | A1 | 29 October 2015 | US | 2017044540 | A1 | 16 February 2017 |
| | | | | US | 2018258429 | A1 | 13 September 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SCHRADER et al.** *Skin Pharmacol Physiol,* 2012, vol. 25 (4), 192-9
- **LI et al.** *Australasian Journal of Dermatology,* May 2010, vol. 51 (2), 106-12
- **HAMMAM et al.** *Advances in Environmental Biology,* January 2016, vol. 10 (1), 237-49
- **DUMAS et al.** *Journal of drugs in dermatology,* June 2007, vol. 6 (6), 20-4
- **KARLSSON et al.** *Biochem. Biophys. Res. Commun.,* 2013, vol. 430, 993-998
- *Biochem. Biophys. Acta.,* vol. 279, 265-275
- *Carbohydr. Res.,* vol. 159, 127-136
- *Int. J. Dermatol.,* vol. 33, 119-122
- **FLEISCHMAJER et al.** *Biochem. Biophys. Acta.,* 1972, vol. 279, 265-275
- **SAMBROOK et al.** Molecular Cloning: Laboratory Manual. Cold Spring Harbor Laboratory Press, 1989

SEQUENCE LISTING

<110> Ractigen Therapeutics

<120> OLIGOMERIC NUCLEIC ACID FOR SKIN CARE

<130> Docket No.: 065786.11018(4US1)

<140> PCT/CN2020/073663
<141> 2020-01-22

<150> CN 201910067625.2
<151> 2019-01-24

<160> 428

<170> PatentIn version 3.5

<210> 1
<211> 21
<212> DNA
<213> Artificial Sequence

<220>
<223> Synthetic Construct

<220>
<221> misc_feature
<222> (20)..(21)
<223> deoxythymine (dT) base

<400> 1
gcagguacac gugugacaat t 21

<210> 2
<211> 21
<212> DNA
<213> Artificial Sequence

<220>
<223> Synthetic Construct

<220>
<221> misc_feature
<222> (20)..(21)
<223> deoxythymine (dT) base

<400> 2
cacuagcugu ccccuugaat t 21

<210> 3
<211> 21
<212> DNA
<213> Artificial Sequence

<220>
<223> Synthetic Construct

<220>
<221> misc_feature
<222> (20)..(21)
<223> deoxythymine (dT) base

<400> 3
uugaagguc ggugccuuat t 21

<210> 4
<211> 21
<212> DNA
<213> Artificial Sequence

<220>
<223> Synthetic Construct

<220>
<221> misc_feature
<222> (20)..(21)
<223> deoxythymine (dT) base

<400> 4
cccugaaccc ugccuauaat t 21

<210> 5
<211> 21
<212> DNA
<213> Artificial Sequence

<220>
<223> Synthetic Construct

<220>
<221> misc_feature
<222> (20)..(21)
<223> deoxythymine (dT) base

<400> 5
ggcucucaga ucugccuaat t 21

<210> 6
<211> 21
<212> DNA
<213> Artificial Sequence

<220>
<223> Synthetic Construct

<220>
<221> misc_feature
<222> (20)..(21)
<223> deoxythymine (dT) base

<400> 6
uugacguccc cucccuuaat t 21

<210> 7
<211> 21
<212> DNA
<213> Artificial Sequence

<220>
<223> Synthetic Construct

<220>
<221> misc_feature
<222> (20)..(21)
<223> deoxythymine (dT) base

<400> 7
ugaagggucg gugccuuaut t 21

<210> 8
<211> 21
<212> DNA
<213> Artificial Sequence

<220>
<223> Synthetic Construct

<220>
<221> misc_feature
<222> (20)..(21)
<223> deoxythymine (dT) base

<400> 8
cuuaucuguc cugacagaat t 21

<210> 9
<211> 21
<212> DNA
<213> Artificial Sequence

<220>
<223> Synthetic Construct

<220>
<221> misc_feature
<222> (20)..(21)
<223> deoxythymine (dT) base

<400> 9
uggcucucag aucugccuat t 21

<210> 10
<211> 21
<212> DNA
<213> Artificial Sequence

<220>
<223> Synthetic Construct

<220>
<221> misc_feature
<222> (20)..(21)
<223> deoxythymine (dT) base

<400> 10
gugccuuauc uguccugact t 21

<210> 11
<211> 21
<212> DNA
<213> Artificial Sequence

<220>
<223> Synthetic Construct

<220>
<221> misc_feature
<222> (20)..(21)
<223> deoxythymine (dT) base

<400> 11
ucucuaugac agcugcauct t 21

<210> 12
<211> 21
<212> DNA
<213> Artificial Sequence

<220>
<223> Synthetic Construct

<220>
<221> misc_feature
<222> (20)..(21)
<223> deoxythymine (dT) base

<400> 12
ugacacuagc uguccccuut t 21

<210> 13
<211> 21
<212> DNA
<213> Artificial Sequence

<220>
<223> Synthetic Construct

<220>
<221> misc_feature
<222> (20)..(21)
<223> deoxythymine (dT) base

<400> 13
guccugacag aagagacagt t 21

<210> 14
<211> 21
<212> DNA
<213> Artificial Sequence

<220>
<223> Artificial Sequence

<220>
<221> misc_feature
<222> (20)..(21)
<223> deoxythymine (dT) base

<400> 14
acccugccua uaaucccact t 21

<210> 15
<211> 21
<212> DNA
<213> Artificial Sequence

<220>
<223> Synthetic Construct

<220>
<221> misc_feature
<222> (20)..(21)
<223> deoxythymine (dT) base

<400> 15
ggucucuaug acagcugcat t 21

<210> 16
<211> 21
<212> DNA
<213> Artificial Sequence

<220>
<223> Synthetic Construct

<220>
<221> misc_feature
<222> (20)..(21)
<223> deoxythymine (dT) base

<400> 16
cuggaaugcu ucuucagaat t 21

<210> 17
<211> 21
<212> DNA
<213> Artificial Sequence

<220>
<223> Synthetic Construct

<220>
<221> misc_feature
<222> (20)..(21)
<223> deoxythymine (dT) base

<400> 17
auggccuaug ugguucuuat t 21

<210> 18
<211> 21
<212> DNA
<213> Artificial Sequence

<220>
<223> Synthetic Construct

<220>
<221> misc_feature
<222> (20)..(21)
<223> deoxythymine (dT) base

<400> 18
ccugauuuga caguuccaat t 21

<210> 19
<211> 21
<212> DNA
<213> Artificial Sequence

<220>
<223> Synthetic Construct

<220>
<221> misc_feature
<222> (20)..(21)
<223> deoxythymine (dT) base

<400> 19
ggugacucca ucuguccuat t 21

<210> 20
<211> 21
<212> DNA
<213> Artificial Sequence

<220>
<223> Synthetic Construct

<220>
<221> misc_feature
<222> (20)..(21)
<223> deoxythymine (dT) base

<400> 20
ccaucucuag gauggccuat t 21

<210> 21
<211> 21
<212> DNA
<213> Artificial Sequence

<220>
<223> Synthetic Construct

<220>
<221> misc_feature
<222> (20)..(21)
<223> deoxythymine (dT) base

<400> 21
ugcaguguga agcuugcaat t 21

<210> 22
<211> 21
<212> DNA
<213> Artificial Sequence

<220>
<223> Synthetic Construct

<220>
<221> misc_feature
<222> (20)..(21)
<223> deoxythymine (dT) base

<400> 22
ugguaucaug aagccacugt t 21

<210> 23
<211> 21
<212> DNA
<213> Artificial Sequence

<220>
<223> Synthetic Construct

<220>
<221> misc_feature
<222> (20)..(21)
<223> deoxythymine (dT) base

<400> 23
gacuccucca ucuuucugat t 21

<210> 24
<211> 21
<212> DNA
<213> Artificial Sequence

<220>
<223> Synthetic Construct

<220>
<221> misc_feature
<222> (20)..(21)
<223> deoxythymine (dT) base

<400> 24
gcaaugguuu cucuguaugt t 21

<210> 25
<211> 21
<212> DNA
<213> Artificial Sequence

<220>
<223> Synthetic Construct

<220>
<221> misc_feature
<222> (20)..(21)
<223> deoxythymine (dT) base

<400> 25
gccuaugugg uucuuacuat t 21

<210> 26
<211> 21
<212> DNA
<213> Artificial Sequence

<220>
<223> Synthetic Construct

<220>
<221> misc_feature
<222> (20)..(21)
<223> deoxythymine (dT) base

<400> 26
ccacuucuga augagaagat t 21

<210> 27
<211> 21
<212> DNA
<213> Artificial Sequence

<220>
<223> Synthetic Construct

<220>
<221> misc_feature
<222> (20)..(21)
<223> deoxythymine (dT) base

<400> 27
guccuacaga cuugcagugt t 21

<210> 28
<211> 21
<212> DNA
<213> Artificial Sequence

<220>
<223> Synthetic Construct

<220>
<221> misc_feature
<222> (20)..(21)
<223> deoxythymine (dT) base

<400> 28
gcccaguuuc auaaccgaat t 21

<210> 29
<211> 21
<212> DNA
<213> Artificial Sequence

<220>
<223> Synthetic Construct

<220>
<221> misc_feature
<222> (20)..(21)
<223> deoxythymine (dT) base

<400> 29
gaccuugacc caugcagaat t 21

<210> 30
<211> 21
<212> DNA
<213> Artificial Sequence

<220>
<223> Synthetic Construct

<220>
<221> misc_feature
<222> (20)..(21)
<223> deoxythymine (dT) base

<400> 30
gaucccuccc agagcaauat t 21

<210> 31
<211> 21
<212> DNA
<213> Artificial Sequence

<220>
<223> Synthetic Construct

<220>
<221> misc_feature
<222> (20)..(21)
<223> deoxythymine (dT) base

<400> 31
ccuugaccca ugcagaauat t 21

<210> 32
<211> 21
<212> DNA
<213> Artificial Sequence

<220>
<223> Synthetic Construct

<220>
<221> misc_feature
<222> (20)..(21)
<223> deoxythymine (dT) base

<400> 32
cagcaccaac auggccuuat t 21

<210> 33
<211> 21
<212> DNA
<213> Artificial Sequence

<220>
<223> Synthetic Construct

<220>
<221> misc_feature
<222> (20)..(21)
<223> deoxythymine (dT) base

<400> 33
augcagaguu cuccaggaat t 21

<210> 34
<211> 21
<212> DNA
<213> Artificial Sequence

<220>
<223> Synthetic Construct

<220>
<221> misc_feature
<222> (20)..(21)
<223> deoxythymine (dT) base

<400> 34
ggaggugacc cagugauaat t 21

<210> 35
<211> 21
<212> DNA
<213> Artificial Sequence

<220>
<223> Synthetic Construct

<220>
<221> misc_feature
<222> (20)..(21)
<223> deoxythymine (dT) base

<400> 35
gacccaguga uaaugggaat t 21

<210> 36
<211> 21
<212> DNA
<213> Artificial Sequence

<220>
<223> Synthetic Construct

<220>
<221> misc_feature
<222> (20)..(21)
<223> deoxythymine (dT) base

<400> 36
ugacccaugc agaauagaat t 21

<210> 37
<211> 21
<212> DNA
<213> Artificial Sequence

<220>
<223> Synthetic Construct

<220>
<221> misc_feature
<222> (20)..(21)
<223> deoxythymine (dT) base

<400> 37
acagcaccaa cauggccuut t 21

<210> 38
<211> 21
<212> DNA
<213> Artificial Sequence

<220>
<223> Synthetic Construct

```
<220>
<221>  misc_feature
<222>  (20)..(21)
<223>  deoxythymine (dT) base

<400>  38
uaccuuccag gccauucaat t                                                 21


<210>  39
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  39
cccaguucca cuucuucuat t                                                 21


<210>  40
<211>  21
<212>  DNA
<213>  Artificial Sequence

<220>
<223>  Synthetic Construct

<400>  40
ucuccauucc aacucccaat t                                                 21


<210>  41
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  41
cuuagcucuu gccaggauat t                                                 21


<210>  42
<211>  21
<212>  DNA
<213>  Artificial Sequence

<220>
```

```
<223>  Synthetic Construct


<220>
<221>  misc_feature
<222>  (20)..(21)
<223>  deoxythymine (dT) base

<400>  42
cuccauucca acucccaaat t                                                  21


<210>  43
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  43
ucuuuaugcc ccucccuuat t                                                  21


<210>  44
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  44
cucuuuaugc cccucccuut t                                                  21


<210>  45
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  45
```

aacccuucca ccuuuggaat t 21

<210> 46
<211> 21
<212> DNA
<213> Artificial Sequence

<220>
<223> Synthetic Construct

<220>
<221> misc_feature
<222> (20)..(21)
<223> deoxythymine (dT) base

<400> 46
agauuggagg ucccaggaat t 21

<210> 47
<211> 21
<212> DNA
<213> Artificial Sequence

<220>
<223> Synthetic Construct

<220>
<221> misc_feature
<222> (20)..(21)
<223> deoxythymine (dT) base

<400> 47
ugagccucag caaaggcaat t 21

<210> 48
<211> 21
<212> DNA
<213> Artificial Sequence

<220>
<223> Synthetic Construct

<220>
<221> misc_feature
<222> (20)..(21)
<223> deoxythymine (dT) base

<400> 48
ggugacucgg ucuagucuat t 21

<210> 49
<211> 21
<212> DNA
<213> Artificial Sequence

<220>

<223>  deoxythymine (dT) base

<400>  49
gcccuuuccc gaagucauat t                                                 21


<210>  50
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  50
agagugcauc acugcugaat t                                                 21


<210>  51
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  51
gagccccaaa gcuagagaat t                                                 21


<210>  52
<211>  21
<212>  DNA
<213>  Artificial Sequence

<220>
<223>  Synthetic Construct


<220>
<221>  misc_feature
<222>  (20)..(21)
<223>  deoxythymine (dT) base

<400>  52
ccucagcaaa ggcaagcuat t                                                 21


<210>  53
<211>  21
<212>  DNA

<213> Artificial Sequence

<220>
<223> Synthetic Construct

<220>
<221> misc_feature
<222> (20)..(21)
<223> deoxythymine (dT) base

<400> 53
cuagucuaga guucgcaaat t 21

<210> 54
<211> 21
<212> DNA
<213> Artificial Sequence

<220>
<223> Synthetic Construct

<220>
<221> misc_feature
<222> (20)..(21)
<223> deoxythymine (dT) base

<400> 54
cccuuucccg aagucauaat t 21

<210> 55
<211> 21
<212> DNA
<213> Artificial Sequence

<220>
<223> Synthetic Construct

<220>
<221> misc_feature
<222> (20)..(21)
<223> deoxythymine (dT) base

<400> 55
ucccgaaguc auaagacaat t 21

<210> 56
<211> 21
<212> DNA
<213> Artificial Sequence

<220>
<223> Synthetic Construct

<220>
<221> misc_feature
<222> (20)..(21)

<223> deoxythymine (dT) base

<400> 56
gccccaaagc uagagaaaat t 21

<210> 57
<211> 21
<212> DNA
<213> Artificial Sequence

<220>
<223> Synthetic Construct

<220>
<221> misc_feature
<222> (20)..(21)
<223> deoxythymine (dT) base

<400> 57
agcccaccug ccaacaaaat t 21

<210> 58
<211> 21
<212> DNA
<213> Artificial Sequence

<220>
<223> Synthetic Construct

<220>
<221> misc_feature
<222> (20)..(21)
<223> deoxythymine (dT) base

<400> 58
uccccagccc accugccaat t 21

<210> 59
<211> 21
<212> DNA
<213> Artificial Sequence

<220>
<223> Synthetic Construct

<220>
<221> misc_feature
<222> (20)..(21)
<223> deoxythymine (dT) base

<400> 59
cuggcaaaau cccaacauat t 21

<210> 60
<211> 21
<212> DNA

<213> Artificial Sequence

<220>
<223> Synthetic Construct

<220>
<221> misc_feature
<222> (20)..(21)
<223> deoxythymine (dT) base

<400> 60
cuguggguug ugucuucuat t 21

<210> 61
<211> 21
<212> DNA
<213> Artificial Sequence

<220>
<223> Synthetic Construct

<220>
<221> misc_feature
<222> (20)..(21)
<223> deoxythymine (dT) base

<400> 61
ucuggcaaaa ucccaacaut t 21

<210> 62
<211> 21
<212> DNA
<213> Artificial Sequence

<220>
<223> Synthetic Construct

<220>
<221> misc_feature
<222> (20)..(21)
<223> deoxythymine (dT) base

<400> 62
uucuggcaaa aucccaacat t 21

<210> 63
<211> 21
<212> DNA
<213> Artificial Sequence

<220>
<223> Synthetic Construct

<220>
<221> misc_feature
<222> (20)..(21)

<223> deoxythymine (dT) base

<400> 63
aacuccagau gugcucuuut t 21

<210> 64
<211> 21
<212> DNA
<213> Artificial Sequence

<220>
<223> Synthetic Construct

<220>
<221> misc_feature
<222> (20)..(21)
<223> deoxythymine (dT) base

<400> 64
uguggguugu gucuucuaut t 21

<210> 65
<211> 21
<212> DNA
<213> Artificial Sequence

<220>
<223> Synthetic Construct

<220>
<221> misc_feature
<222> (20)..(21)
<223> deoxythymine (dT) base

<400> 65
ggguuguguc uucuauaagt t 21

<210> 66
<211> 21
<212> DNA
<213> Artificial Sequence

<220>
<223> Synthetic Construct

<220>
<221> misc_feature
<222> (20)..(21)
<223> deoxythymine (dT) base

<400> 66
cggcucucau auuucagaat t 21

<210> 67
<211> 21
<212> DNA

<213> Artificial Sequence

<220>
<223> Synthetic Construct

<220>
<221> misc_feature
<222> (20)..(21)
<223> deoxythymine (dT) base

<400> 67
ugcggcucuc auauuucagt t 21

<210> 68
<211> 21
<212> DNA
<213> Artificial Sequence

<220>
<223> Synthetic Construct

<220>
<221> misc_feature
<222> (20)..(21)
<223> deoxythymine (dT) base

<400> 68
gaugagucga aggggcauat t 21

<210> 69
<211> 19
<212> DNA
<213> Artificial Sequence

<220>
<223> Synthetic Construct

<220>
<221> misc_feature
<222> (20)..(21)
<223> deoxythymine (dT) base

<400> 69
aggaagatca gttctgtaa 19

<210> 70
<211> 21
<212> DNA
<213> Artificial Sequence

<220>
<223> Synthetic Construct

<220>
<221> misc_feature
<222> (20)..(21)

<223> deoxythymine (dT) base

<400> 70
caagugaggu ggaagagaat t 21

<210> 71
<211> 21
<212> DNA
<213> Artificial Sequence

<220>
<223> Synthetic Construct

<220>
<221> misc_feature
<222> (20)..(21)
<223> deoxythymine (dT) base

<400> 71
auagugggu agggagaaat t 21

<210> 72
<211> 21
<212> DNA
<213> Artificial Sequence

<220>
<223> Synthetic Construct

<220>
<221> misc_feature
<222> (20)..(21)
<223> deoxythymine (dT) base

<400> 72
agaaagggau ggagggaaat t 21

<210> 73
<211> 21
<212> DNA
<213> Artificial Sequence

<220>
<223> Synthetic Construct

<220>
<221> misc_feature
<222> (20)..(21)
<223> deoxythymine (dT) base

<400> 73
aagugaggug gaagagaaat t 21

<210> 74
<211> 21
<212> DNA

<213> Artificial Sequence

<220>
<223> Synthetic Construct

<220>
<221> misc_feature
<222> (20)..(21)
<223> deoxythymine (dT) base

<400> 74
gagaagugga gaggaagaat t 21

<210> 75
<211> 21
<212> DNA
<213> Artificial Sequence

<220>
<223> Synthetic Construct

<220>
<221> misc_feature
<222> (20)..(21)
<223> deoxythymine (dT) base

<400> 75
gauggagaga aagacagaat t 21

<210> 76
<211> 21
<212> DNA
<213> Artificial Sequence

<220>
<223> Synthetic Construct

<220>
<221> misc_feature
<222> (20)..(21)
<223> deoxythymine (dT) base

<400> 76
gagaugaggg uaccaacaat t 21

<210> 77
<211> 21
<212> DNA
<213> Artificial Sequence

<220>
<223> Synthetic Construct

<220>
<221> misc_feature
<222> (20)..(21)

<223> deoxythymine (dT) base

<400> 77
gugggguuagg gagaaagaat t 21

<210> 78
<211> 21
<212> DNA
<213> Artificial Sequence

<220>
<223> Synthetic Construct

<220>
<221> misc_feature
<222> (20)..(21)
<223> deoxythymine (dT) base

<400> 78
gugaggugga agagaaaaat t 21

<210> 79
<211> 21
<212> DNA
<213> Artificial Sequence

<220>
<223> Synthetic Construct

<220>
<221> misc_feature
<222> (20)..(21)
<223> deoxythymine (dT) base

<400> 79
agggcaccau uuggcuuuat t 21

<210> 80
<211> 21
<212> DNA
<213> Artificial Sequence

<220>
<223> Synthetic Construct

<220>
<221> misc_feature
<222> (20)..(21)
<223> deoxythymine (dT) base

<400> 80
uuccuccuug ggucgguuat t 21

<210> 81
<211> 21
<212> DNA

<213>  Artificial Sequence

<220>
<223>  Synthetic Construct

<220>
<221>  misc_feature
<222>  (20)..(21)
<223>  deoxythymine (dT) base

<400>  81
gccagaggaa aguaaggaat t    21

<210>  82
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  82
ugcacacguu agcaaucaat t    21

<210>  83
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  83
aaggcuuuga auggccaaut t    21

<210>  84
<211>  21
<212>  DNA
<213>  Artificial Sequence

<220>
<223>  Synthetic Construct

<220>
<221>  misc_feature
<222>  (20)..(21)

<223> deoxythymine (dT) base

<400> 84
uugaguuggc gggaagaaat t 21

<210> 85
<211> 21
<212> DNA
<213> Artificial Sequence

<220>
<223> Synthetic Construct

<220>
<221> misc_feature
<222> (20)..(21)
<223> deoxythymine (dT) base

<400> 85
aucgcuugga gggaacuugt t 21

<210> 86
<211> 21
<212> DNA
<213> Artificial Sequence

<220>
<223> Synthetic Construct

<220>
<221> misc_feature
<222> (20)..(21)
<223> deoxythymine (dT) base

<400> 86
ggaguucagg gaaauccaat t 21

<210> 87
<211> 21
<212> DNA
<213> Artificial Sequence

<220>
<223> Synthetic Construct

<220>
<221> misc_feature
<222> (20)..(21)
<223> deoxythymine (dT) base

<400> 87
ccccuaugug ucuugcauut t 21

<210> 88
<211> 21
<212> DNA

<213> Artificial Sequence

<220>
<223> Synthetic Construct

<220>
<221> misc_feature
<222> (20)..(21)
<223> deoxythymine (dT) base

<400> 88
ccuauguguc uugcauuuct t 21

<210> 89
<211> 21
<212> DNA
<213> Artificial Sequence

<220>
<223> Synthetic Construct

<220>
<221> misc_feature
<222> (20)..(21)
<223> deoxythymine (dT) base

<400> 89
gcacacguua gcaaucaagt t 21

<210> 90
<211> 21
<212> DNA
<213> Artificial Sequence

<220>
<223> Synthetic Construct

<220>
<221> misc_feature
<222> (20)..(21)
<223> deoxythymine (dT) base

<400> 90
guagcucaga gaaggcuuut t 21

<210> 91
<211> 21
<212> DNA
<213> Artificial Sequence

<220>
<223> Synthetic Construct

<220>
<221> misc_feature
<222> (20)..(21)

<223> deoxythymine (dT) base

<400> 91
uagcucagag aaggcuuugt t 21

<210> 92
<211> 21
<212> DNA
<213> Artificial Sequence

<220>
<223> Synthetic Construct

<220>
<221> misc_feature
<222> (20)..(21)
<223> deoxythymine (dT) base

<400> 92
gcucagagaa ggcuuugaat t 21

<210> 93
<211> 21
<212> DNA
<213> Artificial Sequence

<220>
<223> Synthetic Construct

<220>
<221> misc_feature
<222> (20)..(21)
<223> deoxythymine (dT) base

<400> 93
ggaaccucca agguuccuat t 21

<210> 94
<211> 21
<212> DNA
<213> Artificial Sequence

<220>
<223> Synthetic Construct

<220>
<221> misc_feature
<222> (20)..(21)
<223> deoxythymine (dT) base

<400> 94
ucgccuucag cugguucuat t 21

<210> 95
<211> 21
<212> DNA

<213> Artificial Sequence

<220>
<223> Synthetic Construct

<220>
<221> misc_feature
<222> (20)..(21)
<223> deoxythymine (dT) base

<400> 95
gaggucaugg augucggaat t 21

<210> 96
<211> 21
<212> DNA
<213> Artificial Sequence

<220>
<223> Synthetic Construct

<220>
<221> misc_feature
<222> (20)..(21)
<223> deoxythymine (dT) base

<400> 96
accuccaagg uuccuacaat t 21

<210> 97
<211> 21
<212> DNA
<213> Artificial Sequence

<220>
<223> Synthetic Construct

<220>
<221> misc_feature
<222> (20)..(21)
<223> deoxythymine (dT) base

<400> 97
acuucuuguc ucugcuaact t 21

<210> 98
<211> 21
<212> DNA
<213> Artificial Sequence

<220>
<223> Synthetic Construct

<220>
<221> misc_feature
<222> (20)..(21)

<223> deoxythymine (dT) base

<400> 98
acucgagucu agcacuauct t 21

<210> 99
<211> 21
<212> DNA
<213> Artificial Sequence

<220>
<223> Synthetic Construct

<220>
<221> misc_feature
<222> (20)..(21)
<223> deoxythymine (dT) base

<400> 99
ggaccugaaa acuacuguct t 21

<210> 100
<211> 21
<212> DNA
<213> Artificial Sequence

<220>
<223> Synthetic Construct

<220>
<221> misc_feature
<222> (20)..(21)
<223> deoxythymine (dT) base

<400> 100
gggaauuuag gaggucaugt t 21

<210> 101
<211> 21
<212> DNA
<213> Artificial Sequence

<220>
<223> Synthetic Construct

<220>
<221> misc_feature
<222> (20)..(21)
<223> deoxythymine (dT) base

<400> 101
uggcuuuaag acaccggaat t 21

<210> 102
<211> 21
<212> DNA

<213> Artificial Sequence

<220>
<223> Synthetic Construct

<220>
<221> misc_feature
<222> (20)..(21)
<223> deoxythymine (dT) base

<400> 102
aaccuccaag guuccuacat t 21

<210> 103
<211> 21
<212> DNA
<213> Artificial Sequence

<220>
<223> Synthetic Construct

<220>
<221> misc_feature
<222> (20)..(21)
<223> deoxythymine (dT) base

<400> 103
ccuccaaggu uccuacaaat t 21

<210> 104
<211> 21
<212> DNA
<213> Artificial Sequence

<220>
<223> Synthetic Construct

<220>
<221> misc_feature
<222> (20)..(21)
<223> deoxythymine (dT) base

<400> 104
ucagcugguu cuaaacuuct t 21

<210> 105
<211> 21
<212> DNA
<213> Artificial Sequence

<220>
<223> Synthetic Construct

<220>
<221> misc_feature
<222> (20)..(21)

<223> deoxythymine (dT) base

<400> 105
gcugguucua aacuucuugt t 21

<210> 106
<211> 21
<212> DNA
<213> Artificial Sequence

<220>
<223> Synthetic Construct

<220>
<221> misc_feature
<222> (20)..(21)
<223> deoxythymine (dT) base

<400> 106
cucacuuuac ucgagucuat t 21

<210> 107
<211> 21
<212> DNA
<213> Artificial Sequence

<220>
<223> Synthetic Construct

<220>
<221> misc_feature
<222> (20)..(21)
<223> deoxythymine (dT) base

<400> 107
gauaagguca guugggaaut t 21

<210> 108
<211> 21
<212> DNA
<213> Artificial Sequence

<220>
<223> Synthetic Construct

<220>
<221> misc_feature
<222> (20)..(21)
<223> deoxythymine (dT) base

<400> 108
ugggaauuua ggaggucaut t 21

<210> 109
<211> 21
<212> DNA

<213> Artificial Sequence

<220>
<223> Synthetic Construct

<220>
<221> misc_feature
<222> (20)..(21)
<223> deoxythymine (dT) base

<400> 109
gucauggaug ucggaaauat t 21

<210> 110
<211> 21
<212> DNA
<213> Artificial Sequence

<220>
<223> Synthetic Construct

<220>
<221> misc_feature
<222> (20)..(21)
<223> deoxythymine (dT) base

<400> 110
gaggaaugcu ugaggccaat t 21

<210> 111
<211> 21
<212> DNA
<213> Artificial Sequence

<220>
<223> Synthetic Construct

<220>
<221> misc_feature
<222> (20)..(21)
<223> deoxythymine (dT) base

<400> 111
ccugucucua gaaaaggaat t 21

<210> 112
<211> 21
<212> DNA
<213> Artificial Sequence

<220>
<223> Synthetic Construct

<220>
<221> misc_feature
<222> (20)..(21)

<223> deoxythymine (dT) base

<400> 112
agacccuguc ucuagaaaat t 21

<210> 113
<211> 21
<212> DNA
<213> Artificial Sequence

<220>
<223> Synthetic Construct

<220>
<221> misc_feature
<222> (20)..(21)
<223> deoxythymine (dT) base

<400> 113
aagacccugu cucuagaaat t 21

<210> 114
<211> 21
<212> DNA
<213> Artificial Sequence

<220>
<223> Synthetic Construct

<220>
<221> misc_feature
<222> (20)..(21)
<223> deoxythymine (dT) base

<400> 114
ugauuuccuc cucugugaat t 21

<210> 115
<211> 21
<212> DNA
<213> Artificial Sequence

<220>
<223> Synthetic Construct

<220>
<221> misc_feature
<222> (20)..(21)
<223> deoxythymine (dT) base

<400> 115
caagacccug ucucuagaat t 21

<210> 116
<211> 21
<212> DNA

<213> Artificial Sequence

<220>
<223> Synthetic Construct

<220>
<221> misc_feature
<222> (20)..(21)
<223> deoxythymine (dT) base

<400> 116
uggagaggca gaaggaaaat t 21

<210> 117
<211> 21
<212> DNA
<213> Artificial Sequence

<220>
<223> Synthetic Construct

<220>
<221> misc_feature
<222> (20)..(21)
<223> deoxythymine (dT) base

<400> 117
uccucuguga aaugggcaat t 21

<210> 118
<211> 21
<212> DNA
<213> Artificial Sequence

<220>
<223> Synthetic Construct

<220>
<221> misc_feature
<222> (20)..(21)
<223> deoxythymine (dT) base

<400> 118
agagggagau cgaggcuuat t 21

<210> 119
<211> 21
<212> DNA
<213> Artificial Sequence

<220>
<223> Synthetic Construct

<220>
<221> misc_feature
<222> (20)..(21)

<223> deoxythymine (dT) base

<400> 119
gucacuuugg gaggcuugat t 21

<210> 120
<211> 21
<212> DNA
<213> Artificial Sequence

<220>
<223> Synthetic Construct

<220>
<221> misc_feature
<222> (20)..(21)
<223> deoxythymine (dT) base

<400> 120
gauuuccucc ucugugaaat t 21

<210> 121
<211> 21
<212> DNA
<213> Artificial Sequence

<220>
<223> Synthetic Construct

<220>
<221> misc_feature
<222> (20)..(21)
<223> deoxythymine (dT) base

<400> 121
cugaaagagg ugcugguuat t 21

<210> 122
<211> 21
<212> DNA
<213> Artificial Sequence

<220>
<223> Synthetic Construct

<400> 122
cucaugccug uaaucccaat t 21

<210> 123
<211> 21
<212> DNA
<213> Artificial Sequence

<220>
<223> Synthetic Construct

<220>
<221> misc_feature
<222> (20)..(21)
<223> deoxythymine (dT) base

<400> 123
gacccugucu cuagaaaagt t 21

<210> 124
<211> 21
<212> DNA
<213> Artificial Sequence

<220>
<223> Synthetic Construct

<220>
<221> misc_feature
<222> (20)..(21)
<223> deoxythymine (dT) base

<400> 124
gaggcagaag gaaaaccaat t 21

<210> 125
<211> 21
<212> DNA
<213> Artificial Sequence

<220>
<223> Synthetic Construct

<220>
<221> misc_feature
<222> (20)..(21)
<223> deoxythymine (dT) base

<400> 125
uugucacacg uguaccugct t 21

<210> 126
<211> 21
<212> DNA
<213> Artificial Sequence

<220>
<223> Synthetic Construct

<220>
<221> misc_feature
<222> (20)..(21)
<223> deoxythymine (dT) base

<400> 126
uucaagggga cagcuagugt t 21

<210> 127
<211> 21
<212> DNA
<213> Artificial Sequence

<220>
<223> Synthetic Construct

<220>
<221> misc_feature
<222> (20)..(21)
<223> deoxythymine (dT) base

<400> 127
uaaggcaccg acccuucaat t 21

<210> 128
<211> 21
<212> DNA
<213> Artificial Sequence

<220>
<223> Synthetic Construct

<220>
<221> misc_feature
<222> (20)..(21)
<223> deoxythymine (dT) base

<400> 128
uuauaggcag gguucagggt t 21

<210> 129
<211> 21
<212> DNA
<213> Artificial Sequence

<220>
<223> Synthetic Construct

<220>
<221> misc_feature
<222> (20)..(21)
<223> deoxythymine (dT) base

<400> 129
uuaggcagau cugagagcct t 21

<210> 130
<211> 21
<212> DNA
<213> Artificial Sequence

<220>
<223> Synthetic Construct

<220>
<221> misc_feature
<222> (20)..(21)
<223> deoxythymine (dT) base

<400> 130
uuaagggagg ggacgucaat t 21

<210> 131
<211> 21
<212> DNA
<213> Artificial Sequence

<220>
<223> Synthetic Construct

<220>
<221> misc_feature
<222> (20)..(21)
<223> deoxythymine (dT) base

<400> 131
auaaggcacc gacccuucat t 21

<210> 132
<211> 21
<212> DNA
<213> Artificial Sequence

<220>
<223> Synthetic Construct

<220>
<221> misc_feature
<222> (20)..(21)
<223> deoxythymine (dT) base

<400> 132
uucugucagg acagauaagt t 21

<210> 133
<211> 21
<212> DNA
<213> Artificial Sequence

<220>
<223> Synthetic Construct

<220>
<221> misc_feature
<222> (20)..(21)
<223> deoxythymine (dT) base

<400> 133
uaggcagauc ugagagccat t 21

<210> 134
<211> 21
<212> DNA
<213> Artificial Sequence

<220>
<223> Synthetic Construct

<220>
<221> misc_feature
<222> (20)..(21)
<223> deoxythymine (dT) base

<400> 134
gucaggacag auaaggcact t 21

<210> 135
<211> 21
<212> DNA
<213> Artificial Sequence

<220>
<223> Synthetic Construct

<220>
<221> misc_feature
<222> (20)..(21)
<223> deoxythymine (dT) base

<400> 135
gaugcagcug ucauagagat t 21

<210> 136
<211> 21
<212> DNA
<213> Artificial Sequence

<220>
<223> Synthetic Construct

<220>
<221> misc_feature
<222> (20)..(21)
<223> deoxythymine (dT) base

<400> 136
aaggggacag cuagugucat t 21

<210> 137
<211> 21
<212> DNA
<213> Artificial Sequence

<220>
<223> Synthetic Construct

<220>
<221> misc_feature
<222> (20)..(21)
<223> deoxythymine (dT) base

<400> 137
cugucucuuc ugucaggact t 21

<210> 138
<211> 21
<212> DNA
<213> Artificial Sequence

<220>
<223> Synthetic Construct

<220>
<221> misc_feature
<222> (20)..(21)
<223> deoxythymine (dT) base

<400> 138
gugggauuau aggcagggut t 21

<210> 139
<211> 21
<212> DNA
<213> Artificial Sequence

<220>
<223> Synthetic Construct

<220>
<221> misc_feature
<222> (20)..(21)
<223> deoxythymine (dT) base

<400> 139
ugcagcuguc auagagacct t 21

<210> 140
<211> 21
<212> DNA
<213> Artificial Sequence

<220>
<223> Synthetic Construct

<220>
<221> misc_feature
<222> (20)..(21)
<223> deoxythymine (dT) base

<400> 140
uucugaagaa gcauuccagt t 21

<210> 141
<211> 21
<212> DNA
<213> Artificial Sequence

<220>
<223> Synthetic Construct

<220>
<221> misc_feature
<222> (20)..(21)
<223> deoxythymine (dT) base

<400> 141
uaagaaccac auaggccaut t 21

<210> 142
<211> 21
<212> DNA
<213> Artificial Sequence

<220>
<223> Synthetic Construct

<220>
<221> misc_feature
<222> (20)..(21)
<223> deoxythymine (dT) base

<400> 142
uuggaacugu caaaucaggt t 21

<210> 143
<211> 21
<212> DNA
<213> Artificial Sequence

<220>
<223> Synthetic Construct

<220>
<221> misc_feature
<222> (20)..(21)
<223> deoxythymine (dT) base

<400> 143
uaggacagau ggagucacct t 21

<210> 144
<211> 21
<212> DNA
<213> Artificial Sequence

<220>
<223> Synthetic Construct

<220>
<221> misc_feature
<222> (20)..(21)
<223> deoxythymine (dT) base

<400> 144
uaggccaucc uagagauggt t 21

<210> 145
<211> 21
<212> DNA
<213> Artificial Sequence

<220>
<223> Synthetic Construct

<220>
<221> misc_feature
<222> (20)..(21)
<223> deoxythymine (dT) base

<400> 145
uugcaagcuu cacacugcat t 21

<210> 146
<211> 21
<212> DNA
<213> Artificial Sequence

<220>
<223> Synthetic Construct

<220>
<221> misc_feature
<222> (20)..(21)
<223> deoxythymine (dT) base

<400> 146
caguggcuuc augauaccat t 21

<210> 147
<211> 21
<212> DNA
<213> Artificial Sequence

<220>
<223> Synthetic Construct

<220>
<221> misc_feature
<222> (20)..(21)
<223> deoxythymine (dT) base

<400> 147
ucagaaagau ggaggagucut t 21

<210> 148
<211> 21
<212> DNA
<213> Artificial Sequence

<220>
<223> Synthetic Construct

<220>
<221> misc_feature
<222> (20)..(21)
<223> deoxythymine (dT) base

<400> 148
cauacagaga aaccauugct t 21

<210> 149
<211> 21
<212> DNA
<213> Artificial Sequence

<220>
<223> Synthetic Construct

<220>
<221> misc_feature
<222> (20)..(21)
<223> deoxythymine (dT) base

<400> 149
uaguaagaac cacauaggct t 21

<210> 150
<211> 21
<212> DNA
<213> Artificial Sequence

<220>
<223> Synthetic Construct

<220>
<221> misc_feature
<222> (20)..(21)
<223> deoxythymine (dT) base

<400> 150
ucuucucauu cagaaguggt t 21

<210> 151
<211> 21
<212> DNA
<213> Artificial Sequence

<220>
<223> Synthetic Construct

<220>
<221> misc_feature
<222> (20)..(21)
<223> deoxythymine (dT) base

<400> 151
cacugcaagu cuguaggact t 21

<210> 152
<211> 21
<212> DNA
<213> Artificial Sequence

<220>
<223> Synthetic Construct

<220>
<221> misc_feature
<222> (20)..(21)
<223> deoxythymine (dT) base

<400> 152
uucgguuaug aaacugggct t 21

<210> 153
<211> 21
<212> DNA
<213> Artificial Sequence

<220>
<223> Synthetic Construct

<220>
<221> misc_feature
<222> (20)..(21)
<223> deoxythymine (dT) base

<400> 153
uucugcaugg gucaagguct t 21

<210> 154
<211> 21
<212> DNA
<213> Artificial Sequence

<220>
<223> Synthetic Construct

<220>
<221> misc_feature
<222> (20)..(21)
<223> deoxythymine (dT) base

<400> 154
uauugcucug ggagggauct t 21

<210> 155
<211> 21
<212> DNA
<213> Artificial Sequence

<220>
<223> Synthetic Construct

<220>
<221> misc_feature
<222> (20)..(21)
<223> deoxythymine (dT) base

<400> 155
uauucugcau gggucaaggt t 21

<210> 156
<211> 21
<212> DNA
<213> Artificial Sequence

<220>
<223> Synthetic Construct

<220>
<221> misc_feature
<222> (20)..(21)
<223> deoxythymine (dT) base

<400> 156
uaaggccaug uuggugcugt t 21

<210> 157
<211> 21
<212> DNA
<213> Artificial Sequence

<220>
<223> Synthetic Construct

<220>
<221> misc_feature
<222> (20)..(21)
<223> deoxythymine (dT) base

<400> 157
uuccuggaga acucugcaut t 21

<210> 158
<211> 21
<212> DNA
<213> Artificial Sequence

<220>
<223> Synthetic Construct

<220>
<221> misc_feature
<222> (20)..(21)
<223> deoxythymine (dT) base

<400> 158
uuaucacugg gucaccucct t 21

<210> 159
<211> 21
<212> DNA
<213> Artificial Sequence

<220>
<223> Synthetic Construct

<220>
<221> misc_feature
<222> (20)..(21)
<223> deoxythymine (dT) base

<400> 159
uucccauuau cacuggguct t 21

<210> 160
<211> 21
<212> DNA
<213> Artificial Sequence

<220>
<223> Synthetic Construct

<220>
<221> misc_feature
<222> (20)..(21)
<223> deoxythymine (dT) base

<400> 160
uucuauucug caugggucat t 21

<210> 161
<211> 21
<212> DNA
<213> Artificial Sequence

<220>
<223> Synthetic Construct

<220>
<221> misc_feature
<222> (20)..(21)
<223> deoxythymine (dT) base

<400> 161
aaggccaugu uggugcugut t 21

<210> 162
<211> 21
<212> DNA
<213> Artificial Sequence

<220>
<223> Synthetic Construct

<220>
<221> misc_feature
<222> (20)..(21)
<223> deoxythymine (dT) base

<400> 162
uugaauggcc uggaagguat t 21

<210> 163
<211> 21
<212> DNA
<213> Artificial Sequence

<220>
<223> Synthetic Construct

<220>
<221> misc_feature
<222> (20)..(21)
<223> deoxythymine (dT) base

<400> 163
uagaagaagu ggaacugggt t 21

<210> 164
<211> 21
<212> DNA
<213> Artificial Sequence

<220>
<223> Synthetic Construct

<220>
<221> misc_feature
<222> (20)..(21)
<223> deoxythymine (dT) base

<400> 164
uugggaguug gaauggagat t 21

<210> 165
<211> 21
<212> DNA
<213> Artificial Sequence

<220>
<223> Synthetic Construct

<220>
<221> misc_feature
<222> (20)..(21)
<223> deoxythymine (dT) base

<400> 165
uauccuggca agagcuaagt t 21

<210> 166
<211> 21
<212> DNA
<213> Artificial Sequence

<220>
<223> Synthetic Construct

<220>
<221> misc_feature
<222> (20)..(21)
<223> deoxythymine (dT) base

<400> 166
uuugggaguu ggaauggagt t 21

<210> 167
<211> 21
<212> DNA
<213> Artificial Sequence

<220>
<223> Synthetic Construct

<220>
<221> misc_feature
<222> (20)..(21)
<223> deoxythymine (dT) base

<400> 167
uaagggaggg gcauaaagat t 21

<210> 168
<211> 21
<212> DNA
<213> Artificial Sequence

<220>
<223> Synthetic Construct

<220>
<221> misc_feature
<222> (20)..(21)
<223> deoxythymine (dT) base

<400> 168
aagggagggg cauaaagagt t 21

<210> 169
<211> 21
<212> DNA
<213> Artificial Sequence

<220>
<223> Synthetic Construct

<220>
<221> misc_feature
<222> (20)..(21)
<223> deoxythymine (dT) base

<400> 169
uuccaaaggu ggaaggguut t 21

<210> 170
<211> 21
<212> DNA
<213> Artificial Sequence

<220>
<223> Synthetic Construct

<220>
<221> misc_feature
<222> (20)..(21)
<223> deoxythymine (dT) base

<400> 170
uuccugggac cuccaaucut t 21

<210> 171
<211> 21
<212> DNA
<213> Artificial Sequence

<220>
<223> Synthetic Construct

<220>
<221> misc_feature
<222> (20)..(21)
<223> deoxythymine (dT) base

<400> 171
uugccuuugc ugaggcucat t 21

<210> 172
<211> 21
<212> DNA
<213> Artificial Sequence

<220>
<223> Synthetic Construct

<220>
<221> misc_feature
<222> (20)..(21)
<223> deoxythymine (dT) base

<400> 172
uagacuagac cgagucacct t 21

<210> 173
<211> 21
<212> DNA
<213> Artificial Sequence

<220>
<223> Synthetic Construct

<220>
<221> misc_feature
<222> (20)..(21)
<223> deoxythymine (dT) base

<400> 173
uaugacuucg ggaaagggct t 21

<210> 174
<211> 21
<212> DNA
<213> Artificial Sequence

<220>
<223> Synthetic Construct

<220>
<221> misc_feature
<222> (20)..(21)
<223> deoxythymine (dT) base

<400> 174
uucagcagug augcacucut t 21

<210> 175
<211> 21
<212> DNA
<213> Artificial Sequence

<220>
<223> Synthetic Construct

<220>
<221> misc_feature
<222> (20)..(21)
<223> deoxythymine (dT) base

<400> 175
uucucuagcu uuggggcuct t 21

<210> 176
<211> 21
<212> DNA
<213> Artificial Sequence

<220>
<223> Synthetic Construct

<220>
<221> misc_feature
<222> (20)..(21)
<223> deoxythymine (dT) base

<400> 176
uagcuugccu uugcugaggt t 21

<210> 177
<211> 21
<212> DNA
<213> Artificial Sequence

<220>
<223> Synthetic Construct

<220>
<221> misc_feature
<222> (20)..(21)
<223> deoxythymine (dT) base

<400> 177
uuugcgaacu cuagacuagt t 21

<210> 178
<211> 21
<212> DNA
<213> Artificial Sequence

<220>
<223> Synthetic Construct

<220>
<221> misc_feature
<222> (20)..(21)
<223> deoxythymine (dT) base

<400> 178
uuaugacuuc gggaaagggt t 21

<210> 179
<211> 21
<212> DNA
<213> Artificial Sequence

<220>
<223> Synthetic Construct

<220>
<221> misc_feature
<222> (20)..(21)
<223> deoxythymine (dT) base

<400> 179
uugucuuaug acuucgggat t 21

<210> 180
<211> 21
<212> DNA
<213> Artificial Sequence

<220>
<223> Synthetic Construct

<220>
<221> misc_feature
<222> (20)..(21)
<223> deoxythymine (dT) base

<400> 180
uuuucucuag cuuuggggct t 21

<210> 181
<211> 21
<212> DNA
<213> Artificial Sequence

<220>
<223> Synthetic Construct

<220>
<221> misc_feature
<222> (20)..(21)
<223> deoxythymine (dT) base

<400> 181
uuuuguuggc aggugggcut t 21

<210> 182
<211> 21
<212> DNA
<213> Artificial Sequence

<220>
<223> Synthetic Construct

<220>
<221> misc_feature
<222> (20)..(21)
<223> deoxythymine (dT) base

<400> 182
uuggcaggug ggcuggggat t 21

<210> 183
<211> 21
<212> DNA
<213> Artificial Sequence

<220>
<223> Synthetic Construct

<220>
<221> misc_feature
<222> (20)..(21)
<223> deoxythymine (dT) base

<400> 183
uauguuggga uuuugccagt t 21

<210> 184
<211> 21
<212> DNA
<213> Artificial Sequence

<220>
<223> Synthetic Construct

<220>
<221> misc_feature
<222> (20)..(21)
<223> deoxythymine (dT) base

<400> 184
uagaagacac aacccacagt t 21

<210> 185
<211> 21
<212> DNA
<213> Artificial Sequence

<220>
<223> Synthetic Construct

<220>
<221> misc_feature
<222> (20)..(21)
<223> deoxythymine (dT) base

<400> 185
auguugggau uuugccagat t 21

<210> 186
<211> 21
<212> DNA
<213> Artificial Sequence

<220>
<223> Synthetic Construct

<220>
<221> misc_feature
<222> (20)..(21)
<223> deoxythymine (dT) base

<400> 186
uguugggauu uugccagaat t 21

<210> 187
<211> 21
<212> DNA
<213> Artificial Sequence

<220>
<223> Synthetic Construct

<220>
<221> misc_feature
<222> (20)..(21)
<223> deoxythymine (dT) base

<400> 187
aaagagcaca ucuggaguut t 21

<210> 188
<211> 21
<212> DNA
<213> Artificial Sequence

<220>
<223> Synthetic Construct

<220>
<221> misc_feature
<222> (20)..(21)
<223> deoxythymine (dT) base

<400> 188
auagaagaca caacccacat t 21

<210> 189
<211> 21
<212> DNA
<213> Artificial Sequence

<220>
<223> Synthetic Construct

<220>
<221> misc_feature
<222> (20)..(21)
<223> deoxythymine (dT) base

<400> 189
cuuauagaag acacaaccct t 21

<210> 190
<211> 21
<212> DNA
<213> Artificial Sequence

<220>
<223> Synthetic Construct

<220>
<221> misc_feature
<222> (20)..(21)
<223> deoxythymine (dT) base

<400> 190
uucugaaaua ugagagccgt t 21

<210> 191
<211> 21
<212> DNA
<213> Artificial Sequence

<220>
<223> Synthetic Construct

<220>
<221> misc_feature
<222> (20)..(21)
<223> deoxythymine (dT) base

<400> 191
cugaaauaug agagccgcat t 21

<210> 192
<211> 21
<212> DNA
<213> Artificial Sequence

<220>
<223> Synthetic Construct

<220>
<221> misc_feature
<222> (20)..(21)
<223> deoxythymine (dT) base

<400> 192
uaugccccuu cgacucauct t 21

<210> 193
<211> 21
<212> DNA
<213> Artificial Sequence

<220>
<223> Synthetic Construct

<220>
<221> misc_feature
<222> (20)..(21)
<223> deoxythymine (dT) base

<400> 193
uuacagaacu gaucuuccut t 21

<210> 194
<211> 21
<212> DNA
<213> Artificial Sequence

<220>
<223> Synthetic Construct

<220>
<221> misc_feature
<222> (20)..(21)
<223> deoxythymine (dT) base

<400> 194
uucucuucca ccucacuugt t 21

<210> 195
<211> 21
<212> DNA
<213> Artificial Sequence

<220>
<223> Synthetic Construct

<220>
<221> misc_feature
<222> (20)..(21)
<223> deoxythymine (dT) base

<400> 195
uuucucccua acccacuaut t 21

<210> 196
<211> 21
<212> DNA
<213> Artificial Sequence

<220>
<223> Synthetic Construct

<220>
<221> misc_feature
<222> (20)..(21)
<223> deoxythymine (dT) base

<400> 196
uuucccucca ucccuuucut t 21

<210> 197
<211> 21
<212> DNA
<213> Artificial Sequence

<220>
<223> Synthetic Construct

<220>
<221> misc_feature
<222> (20)..(21)
<223> deoxythymine (dT) base

<400> 197
uuucucuucc accucacuut t 21

<210> 198
<211> 21
<212> DNA
<213> Artificial Sequence

<220>
<223> Synthetic Construct

<220>
<221> misc_feature
<222> (20)..(21)
<223> deoxythymine (dT) base

<400> 198
uucuuccucu ccacuucuct t 21

<210> 199
<211> 21
<212> DNA
<213> Artificial Sequence

<220>
<223> Synthetic Construct

<220>
<221> misc_feature
<222> (20)..(21)
<223> deoxythymine (dT) base

<400> 199
uucugucuuu cucuccauct t 21

<210> 200
<211> 21
<212> DNA
<213> Artificial Sequence

<220>
<223> Synthetic Construct

<220>
<221> misc_feature
<222> (20)..(21)
<223> deoxythymine (dT) base

<400> 200
uuguugguac ccucaucuct t 21

<210> 201
<211> 21
<212> DNA
<213> Artificial Sequence

<220>
<223> Synthetic Construct

<220>
<221> misc_feature
<222> (20)..(21)
<223> deoxythymine (dT) base

<400> 201
uucuuucucc cuaacccact t 21

<210> 202
<211> 21
<212> DNA
<213> Artificial Sequence

<220>
<223> Synthetic Construct

<220>
<221> misc_feature
<222> (20)..(21)
<223> deoxythymine (dT) base

<400> 202
uuuuucucuu ccaccucact t 21

<210> 203
<211> 21
<212> DNA
<213> Artificial Sequence

<220>
<223> Synthetic Construct

<220>
<221> misc_feature
<222> (20)..(21)
<223> deoxythymine (dT) base

<400> 203
uaaagccaaa uggugcccut t 21

<210> 204
<211> 21
<212> DNA
<213> Artificial Sequence

<220>
<223> Synthetic Construct

<220>
<221> misc_feature
<222> (20)..(21)
<223> deoxythymine (dT) base

<400> 204
uaaccgaccc aaggaggaat t 21

<210> 205
<211> 21
<212> DNA
<213> Artificial Sequence

<220>
<223> Synthetic Construct

<220>
<221> misc_feature
<222> (20)..(21)
<223> deoxythymine (dT) base

<400> 205
uuccuuacuu uccucuggct t 21

<210> 206
<211> 21
<212> DNA
<213> Artificial Sequence

<220>
<223> Synthetic Construct

<220>
<221> misc_feature
<222> (20)..(21)
<223> deoxythymine (dT) base

<400> 206
uugauugcua acgugugcat t 21

<210> 207
<211> 21
<212> DNA
<213> Artificial Sequence

<220>
<223> Synthetic Construct

<220>
<221> misc_feature
<222> (20)..(21)
<223> deoxythymine (dT) base

<400> 207
auuggccauu caaagccuut t 21

<210> 208
<211> 21
<212> DNA
<213> Artificial Sequence

<220>
<223> Synthetic Construct

<220>
<221> misc_feature
<222> (20)..(21)
<223> deoxythymine (dT) base

<400> 208
uuucuucccg ccaacucaat t 21

<210> 209
<211> 21
<212> DNA
<213> Artificial Sequence

<220>
<223> Synthetic Construct

<220>
<221> misc_feature
<222> (20)..(21)
<223> deoxythymine (dT) base

<400> 209
caaguucccu ccaagcgaut t 21

<210> 210
<211> 21
<212> DNA
<213> Artificial Sequence

<220>
<223> Synthetic Construct

<220>
<221> misc_feature
<222> (20)..(21)
<223> deoxythymine (dT) base

<400> 210
uuggauuucc cugaacucct t 21

<210> 211
<211> 21
<212> DNA
<213> Artificial Sequence

<220>
<223> Synthetic Construct

<220>
<221> misc_feature
<222> (20)..(21)
<223> deoxythymine (dT) base

<400> 211
aaugcaagac acauaggggt t 21

<210> 212
<211> 21
<212> DNA
<213> Artificial Sequence

<220>
<223> Synthetic Construct

<220>
<221> misc_feature
<222> (20)..(21)
<223> deoxythymine (dT) base

<400> 212
gaaaugcaag acacauaggt t 21

<210> 213
<211> 21
<212> DNA
<213> Artificial Sequence

<220>
<223> Synthetic Construct

<220>
<221> misc_feature
<222> (20)..(21)
<223> deoxythymine (dT) base

<400> 213
cuugauugcu aacgugugct t 21

<210> 214
<211> 21
<212> DNA
<213> Artificial Sequence

<220>
<223> Synthetic Construct

<220>
<221> misc_feature
<222> (20)..(21)
<223> deoxythymine (dT) base

<400> 214
aaagccuucu cugagcuact t 21

<210> 215
<211> 21
<212> DNA
<213> Artificial Sequence

<220>
<223> Synthetic Construct

<220>
<221> misc_feature
<222> (20)..(21)
<223> deoxythymine (dT) base

<400> 215
caaagccuuc ucugagcuat t 21

<210> 216
<211> 21
<212> DNA
<213> Artificial Sequence

<220>
<223> Synthetic Construct

<220>
<221> misc_feature
<222> (20)..(21)
<223> deoxythymine (dT) base

<400> 216
uucaaagccu ucucugagct t 21

<210> 217
<211> 21
<212> DNA
<213> Artificial Sequence

<220>
<223> Synthetic Construct

<220>
<221> misc_feature
<222> (20)..(21)
<223> deoxythymine (dT) base

<400> 217
uaggaaccuu ggagguucct t 21

<210> 218
<211> 21
<212> DNA
<213> Artificial Sequence

<220>
<223> Synthetic Construct

<220>
<221> misc_feature
<222> (20)..(21)
<223> deoxythymine (dT) base

<400> 218
uagaaccagc ugaaggcgat t 21

<210> 219
<211> 21
<212> DNA
<213> Artificial Sequence

<220>
<223> Synthetic Construct

<220>
<221> misc_feature
<222> (20)..(21)
<223> deoxythymine (dT) base

<400> 219
uuccgacauc caugaccuct t 21

<210> 220
<211> 21
<212> DNA
<213> Artificial Sequence

<220>
<223> Synthetic Construct

<220>
<221> misc_feature
<222> (20)..(21)
<223> deoxythymine (dT) base

<400> 220
uuguaggaac cuuggaggut t 21

<210> 221
<211> 21
<212> DNA
<213> Artificial Sequence

<220>
<223> Synthetic Construct

<220>
<221> misc_feature
<222> (20)..(21)
<223> deoxythymine (dT) base

<400> 221
guuagcagag acaagaagut t 21

<210> 222
<211> 21
<212> DNA
<213> Artificial Sequence

<220>
<223> Synthetic Construct

<220>
<221> misc_feature
<222> (20)..(21)
<223> deoxythymine (dT) base

<400> 222
gauagugcua gacucgagut t 21

<210> 223
<211> 21
<212> DNA
<213> Artificial Sequence

<220>
<223> Synthetic Construct

<220>
<221> misc_feature
<222> (20)..(21)
<223> deoxythymine (dT) base

<400> 223
gacaguaguu uucaggucct t 21

<210> 224
<211> 21
<212> DNA
<213> Artificial Sequence

<220>
<223> Synthetic Construct

<220>
<221> misc_feature
<222> (20)..(21)
<223> deoxythymine (dT) base

<400> 224
caugaccucc uaaauuccct t 21

<210> 225
<211> 21
<212> DNA
<213> Artificial Sequence

<220>
<223> Synthetic Construct

<220>
<221> misc_feature
<222> (20)..(21)
<223> deoxythymine (dT) base

<400> 225
uuccgguguc uuaaagccat t 21

<210> 226
<211> 21
<212> DNA
<213> Artificial Sequence

<220>
<223> Synthetic Construct

<220>
<221> misc_feature
<222> (20)..(21)
<223> deoxythymine (dT) base

<400> 226
uguaggaacc uuggagguut t 21

<210> 227
<211> 21
<212> DNA
<213> Artificial Sequence

<220>
<223> Synthetic Construct

<220>
<221> misc_feature
<222> (20)..(21)
<223> deoxythymine (dT) base

<400> 227
uuuguaggaa ccuuggaggt t 21

<210> 228
<211> 21
<212> DNA
<213> Artificial Sequence

<220>
<223> Synthetic Construct

<220>
<221> misc_feature
<222> (20)..(21)
<223> deoxythymine (dT) base

<400> 228
gaaguuuaga accagcugat t 21

<210> 229
<211> 21
<212> DNA
<213> Artificial Sequence

<220>
<223> Synthetic Construct

<220>
<221> misc_feature
<222> (20)..(21)
<223> deoxythymine (dT) base

<400> 229
caagaaguuu agaaccagct t 21

<210> 230
<211> 21
<212> DNA
<213> Artificial Sequence

<220>
<223> Synthetic Construct

<220>
<221> misc_feature
<222> (20)..(21)
<223> deoxythymine (dT) base

<400> 230
uagacucgag uaaagugagt t 21

<210> 231
<211> 21
<212> DNA
<213> Artificial Sequence

<220>
<223> Synthetic Construct

<220>
<221> misc_feature
<222> (20)..(21)
<223> deoxythymine (dT) base

<400> 231
auucccaacu gaccuuauct t 21

<210> 232
<211> 21
<212> DNA
<213> Artificial Sequence

<220>
<223> Synthetic Construct

<220>
<221> misc_feature
<222> (20)..(21)
<223> deoxythymine (dT) base

<400> 232
augaccuccu aaauucccat t 21

<210> 233
<211> 21
<212> DNA
<213> Artificial Sequence

<220>
<223> Synthetic Construct

<220>
<221> misc_feature
<222> (20)..(21)
<223> deoxythymine (dT) base

<400> 233
uauuuccgac auccaugact t 21

<210> 234
<211> 21
<212> DNA
<213> Artificial Sequence

<220>
<223> Synthetic Construct

<220>
<221> misc_feature
<222> (20)..(21)
<223> deoxythymine (dT) base

<400> 234
uuggccucaa gcauuccuct t 21

<210> 235
<211> 21
<212> DNA
<213> Artificial Sequence

<220>
<223> Synthetic Construct

<220>
<221> misc_feature
<222> (20)..(21)
<223> deoxythymine (dT) base

<400> 235
uuccuuuucu agagacaggt t 21

<210> 236
<211> 21
<212> DNA
<213> Artificial Sequence

<220>
<223> Synthetic Construct

<220>
<221> misc_feature
<222> (20)..(21)
<223> deoxythymine (dT) base

<400> 236
uuuucuagag acagggucut t 21

<210> 237
<211> 21
<212> DNA
<213> Artificial Sequence

<220>
<223> Synthetic Construct

<220>
<221> misc_feature
<222> (20)..(21)
<223> deoxythymine (dT) base

<400> 237
uuucuagaga cagggucuut t 21

<210> 238
<211> 21
<212> DNA
<213> Artificial Sequence

<220>
<223> Synthetic Construct

<220>
<221> misc_feature
<222> (20)..(21)
<223> deoxythymine (dT) base

<400> 238
uucacagagg aggaaaucat t 21

<210> 239
<211> 21
<212> DNA
<213> Artificial Sequence

<220>
<223> Synthetic Construct

<220>
<221> misc_feature
<222> (20)..(21)
<223> deoxythymine (dT) base

<400> 239
uucuagagac agggucuugt t 21

<210> 240
<211> 21
<212> DNA
<213> Artificial Sequence

<220>
<223> Synthetic Construct

<220>
<221> misc_feature
<222> (20)..(21)
<223> deoxythymine (dT) base

<400> 240
uuuuccuucu gccucuccat t 21

<210> 241
<211> 21
<212> DNA
<213> Artificial Sequence

<220>
<223> Synthetic Construct

<220>
<221> misc_feature
<222> (20)..(21)
<223> deoxythymine (dT) base

<400> 241
uugcccauuu cacagaggat t 21

<210> 242
<211> 21
<212> DNA
<213> Artificial Sequence

<220>
<223> Synthetic Construct

<220>
<221> misc_feature
<222> (20)..(21)
<223> deoxythymine (dT) base

<400> 242
uaagccucga ucucccucut t 21

<210> 243
<211> 21
<212> DNA
<213> Artificial Sequence

<220>
<223> Synthetic Construct

<220>
<221> misc_feature
<222> (20)..(21)
<223> deoxythymine (dT) base

<400> 243
ucaagccucc caaagugact t 21

<210> 244
<211> 21
<212> DNA
<213> Artificial Sequence

<220>
<223> Synthetic Construct

<220>
<221> misc_feature
<222> (20)..(21)
<223> deoxythymine (dT) base

<400> 244
uuucacagag gaggaaauct t 21

<210> 245
<211> 21
<212> DNA
<213> Artificial Sequence

<220>
<223> Synthetic Construct

<220>
<221> misc_feature
<222> (20)..(21)
<223> deoxythymine (dT) base

<400> 245
uaaccagcac cucuuucagt t 21

<210> 246
<211> 21
<212> DNA
<213> Artificial Sequence

<220>
<223> Synthetic Construct

<220>
<221> misc_feature
<222> (20)..(21)
<223> deoxythymine (dT) base

<400> 246
uugggauuac aggcaugagt t 21

<210> 247
<211> 21
<212> DNA
<213> Artificial Sequence

<220>
<223> Synthetic Construct

<220>
<221> misc_feature
<222> (20)..(21)
<223> deoxythymine (dT) base

<400> 247
cuuuucuaga gacaggguct t 21

<210> 248
<211> 21
<212> DNA
<213> Artificial Sequence

<220>
<223> Synthetic Construct

<220>
<221> misc_feature
<222> (20)..(21)
<223> deoxythymine (dT) base

<400> 248
uugguuuucc uucugccuct t 21

<210> 249
<211> 19
<212> DNA
<213> Artificial Sequence

<220>
<223> Synthetic Construct

<400> 249

gcaggtacac gtgtgacaa 19

<210> 250
<211> 19
<212> DNA
<213> Artificial Sequence

<220>
<223> Synthetic Construct

<400> 250
cactagctgt ccccttgaa 19

<210> 251
<211> 19
<212> DNA
<213> Artificial Sequence

<220>
<223> Synthetic Construct

<400> 251
ttgaagggtc ggtgcctta 19

<210> 252
<211> 19
<212> DNA
<213> Artificial Sequence

<220>
<223> Synthetic Construct

<400> 252
ccctgaaccc tgcctataa 19

<210> 253
<211> 19
<212> DNA
<213> Artificial Sequence

<220>
<223> Synthetic Construct

<400> 253
ggctctcaga tctgcctaa 19

<210> 254
<211> 19
<212> DNA
<213> Artificial Sequence

<220>
<223> Synthetic Construct

<400> 254
ttgacgtccc ctcccttaa 19

<210> 255
<211> 19
<212> DNA
<213> Artificial Sequence

<220>
<223> Synthetic Construct

<400> 255
tgaagggtcg gtgccttat 19

<210> 256
<211> 19
<212> DNA
<213> Artificial Sequence

<220>
<223> Synthetic Construct

<400> 256
cttatctgtc ctgacagaa 19

<210> 257
<211> 19
<212> DNA
<213> Artificial Sequence

<220>
<223> Synthetic Construct

<400> 257
tggctctcag atctgccta 19

<210> 258
<211> 19
<212> DNA
<213> Artificial Sequence

<220>
<223> Synthetic Construct

<400> 258
gtgccttatc tgtcctgac 19

<210> 259
<211> 19
<212> DNA
<213> Artificial Sequence

<220>
<223> Synthetic Construct

<400> 259
tctctatgac agctgcatc 19

<210> 260
<211> 19
<212> DNA

<213> Artificial Sequence

<220>
<223> Synthetic Construct

<400> 260
tgacactagc tgtcccctt 19

<210> 261
<211> 19
<212> DNA
<213> Artificial Sequence

<220>
<223> Synthetic Construct

<400> 261
gtcctgacag aagagacag 19

<210> 262
<211> 19
<212> DNA
<213> Artificial Sequence

<220>
<223> Synthetic Construct

<400> 262
accctgccta taatcccac 19

<210> 263
<211> 19
<212> DNA
<213> Artificial Sequence

<220>
<223> Synthetic Construct

<400> 263
ggtctctatg acagctgca 19

<210> 264
<211> 19
<212> DNA
<213> Artificial Sequence

<220>
<223> Synthetic Construct

<400> 264
ctggaatgct tcttcagaa 19

<210> 265
<211> 19
<212> DNA
<213> Artificial Sequence

<220>

<223> Synthetic Construct

<400> 265
atggcctatg tggttctta 19

<210> 266
<211> 19
<212> DNA
<213> Artificial Sequence

<220>
<223> Synthetic Construct

<400> 266
cctgatttga cagttccaa 19

<210> 267
<211> 19
<212> DNA
<213> Artificial Sequence

<220>
<223> Synthetic Construct

<400> 267
ggtgactcca tctgtccta 19

<210> 268
<211> 19
<212> DNA
<213> Artificial Sequence

<220>
<223> Synthetic Construct

<400> 268
ccatctctag gatggccta 19

<210> 269
<211> 19
<212> DNA
<213> Artificial Sequence

<220>
<223> Synthetic Construct

<400> 269
tgcagtgtga agcttgcaa 19

<210> 270
<211> 19
<212> DNA
<213> Artificial Sequence

<220>
<223> Synthetic Construct

<400> 270

tggtatcatg aagccactg 19

<210> 271
<211> 19
<212> DNA
<213> Artificial Sequence

<220>
<223> Synthetic Construct

<400> 271
gactcctcca tctttctga 19

<210> 272
<211> 19
<212> DNA
<213> Artificial Sequence

<220>
<223> Synthetic Construct

<400> 272
gcaatggttt ctctgtatg 19

<210> 273
<211> 19
<212> DNA
<213> Artificial Sequence

<220>
<223> Synthetic Construct

<400> 273
gcctatgtgg ttcttacta 19

<210> 274
<211> 19
<212> DNA
<213> Artificial Sequence

<220>
<223> Synthetic Construct

<400> 274
ccacttctga atgagaaga 19

<210> 275
<211> 19
<212> DNA
<213> Artificial Sequence

<220>
<223> Synthetic Construct

<400> 275
gtcctacaga cttgcagtg 19

<210> 276
<211> 19
<212> DNA
<213> Artificial Sequence

<220>
<223> Synthetic Construct

<400> 276
gcccagtttc ataaccgaa 19

<210> 277
<211> 19
<212> DNA
<213> Artificial Sequence

<220>
<223> Synthetic Construct

<400> 277
gaccttgacc catgcagaa 19

<210> 278
<211> 19
<212> DNA
<213> Artificial Sequence

<220>
<223> Synthetic Construct

<400> 278
gatccctccc agagcaata 19

<210> 279
<211> 19
<212> DNA
<213> Artificial Sequence

<220>
<223> Synthetic Construct

<400> 279
ccttgaccca tgcagaata 19

<210> 280
<211> 19
<212> DNA
<213> Artificial Sequence

<220>
<223> Synthetic Construct

<400> 280
cagcaccaac atggcctta 19

<210> 281
<211> 19
<212> DNA

<213>  Artificial Sequence

<220>
<223>  Synthetic Construct

<400>  281
atgcagagtt ctccaggaa                                                    19

<210>  282
<211>  19
<212>  DNA
<213>  Artificial Sequence

<220>
<223>  Synthetic Construct

<400>  282
ggaggtgacc cagtgataa                                                    19

<210>  283
<211>  19
<212>  DNA
<213>  Artificial Sequence

<220>
<223>  Synthetic Construct

<400>  283
gacccagtga taatgggaa                                                    19

<210>  284
<211>  19
<212>  DNA
<213>  Artificial Sequence

<220>
<223>  Synthetic Construct

<400>  284
tgacccatgc agaatagaa                                                    19

<210>  285
<211>  19
<212>  DNA
<213>  Artificial Sequence

<220>
<223>  Synthetic Construct

<400>  285
acagcaccaa catggcctt                                                    19

<210>  286
<211>  19
<212>  DNA
<213>  Artificial Sequence

<220>

<223> Synthetic Construct

<400> 286
taccttccag gccattcaa 19

<210> 287
<211> 19
<212> DNA
<213> Artificial Sequence

<220>
<223> Synthetic Construct

<400> 287
cccagttcca cttcttcta 19

<210> 288
<211> 19
<212> DNA
<213> Artificial Sequence

<220>
<223> Synthetic Construct

<400> 288
tctccattcc aactcccaa 19

<210> 289
<211> 19
<212> DNA
<213> Artificial Sequence

<220>
<223> Synthetic Construct

<400> 289
cttagctctt gccaggata 19

<210> 290
<211> 19
<212> DNA
<213> Artificial Sequence

<220>
<223> Synthetic Construct

<400> 290
ctccattcca actcccaaa 19

<210> 291
<211> 19
<212> DNA
<213> Artificial Sequence

<220>
<223> Synthetic Construct

<400> 291

tctttatgcc cctccctta 19

<210> 292
<211> 19
<212> DNA
<213> Artificial Sequence

<220>
<223> Synthetic Construct

<400> 292
ctctttatgc ccctccctt 19

<210> 293
<211> 19
<212> DNA
<213> Artificial Sequence

<220>
<223> Synthetic Construct

<400> 293
aacccttcca cctttggaa 19

<210> 294
<211> 19
<212> DNA
<213> Artificial Sequence

<220>
<223> Synthetic Construct

<400> 294
agattggagg tcccaggaa 19

<210> 295
<211> 19
<212> DNA
<213> Artificial Sequence

<220>
<223> Synthetic Construct

<400> 295
tgagcctcag caaaggcaa 19

<210> 296
<211> 19
<212> DNA
<213> Artificial Sequence

<220>
<223> Synthetic Construct

<400> 296
ggtgactcgg tctagtcta 19

<210> 297
<211> 19
<212> DNA
<213> Artificial Sequence

<220>
<223> Synthetic Construct

<400> 297
gccctttccc gaagtcata 19

<210> 298
<211> 19
<212> DNA
<213> Artificial Sequence

<220>
<223> Synthetic Construct

<400> 298
agagtgcatc actgctgaa 19

<210> 299
<211> 19
<212> DNA
<213> Artificial Sequence

<220>
<223> Synthetic Construct

<400> 299
gagccccaaa gctagagaa 19

<210> 300
<211> 19
<212> DNA
<213> Artificial Sequence

<220>
<223> Synthetic Construct

<400> 300
cctcagcaaa ggcaagcta 19

<210> 301
<211> 19
<212> DNA
<213> Artificial Sequence

<220>
<223> Synthetic Construct

<400> 301
ctagtctaga gttcgcaaa 19

<210> 302
<211> 19
<212> DNA

<213> Artificial Sequence

<220>
<223> Synthetic Construct

<400> 302
ccctttcccg aagtcataa 19

<210> 303
<211> 19
<212> DNA
<213> Artificial Sequence

<220>
<223> Synthetic Construct

<400> 303
tcccgaagtc ataagacaa 19

<210> 304
<211> 19
<212> DNA
<213> Artificial Sequence

<220>
<223> Synthetic Construct

<400> 304
gccccaaagc tagagaaaa 19

<210> 305
<211> 19
<212> DNA
<213> Artificial Sequence

<220>
<223> Synthetic Construct

<400> 305
agcccacctg ccaacaaaa 19

<210> 306
<211> 19
<212> DNA
<213> Artificial Sequence

<220>
<223> Synthetic Construct

<400> 306
tccccagccc acctgccaa 19

<210> 307
<211> 19
<212> DNA
<213> Artificial Sequence

<220>

<223> Synthetic Construct

<400> 307
ctggcaaaat cccaacata 19

<210> 308
<211> 19
<212> DNA
<213> Artificial Sequence

<220>
<223> Synthetic Construct

<400> 308
ctgtgggttg tgtcttcta 19

<210> 309
<211> 19
<212> DNA
<213> Artificial Sequence

<220>
<223> Synthetic Construct

<400> 309
tctggcaaaa tcccaacat 19

<210> 310
<211> 19
<212> DNA
<213> Artificial Sequence

<220>
<223> Synthetic Construct

<400> 310
ttctggcaaa atcccaaca 19

<210> 311
<211> 19
<212> DNA
<213> Artificial Sequence

<220>
<223> Synthetic Construct

<400> 311
aactccagat gtgctcttt 19

<210> 312
<211> 19
<212> DNA
<213> Artificial Sequence

<220>
<223> Synthetic Construct

<400> 312

tgtgggttgt gtcttctat 19

<210> 313
<211> 19
<212> DNA
<213> Artificial Sequence

<220>
<223> Synthetic Construct

<400> 313
gggttgtgtc ttctataag 19

<210> 314
<211> 19
<212> DNA
<213> Artificial Sequence

<220>
<223> Synthetic Construct

<400> 314
cggctctcat atttcagaa 19

<210> 315
<211> 19
<212> DNA
<213> Artificial Sequence

<220>
<223> Synthetic Construct

<400> 315
tgcggctctc atatttcag 19

<210> 316
<211> 19
<212> DNA
<213> Artificial Sequence

<220>
<223> Synthetic Construct

<400> 316
gatgagtcga aggggcata 19

<210> 317
<211> 21
<212> DNA
<213> Artificial Sequence

<220>
<223> Synthetic Construct

<220>
<221> misc_feature
<222> (20)..(21)

<223> deoxythymine (dT) base

<400> 317
uaugccccuu cgacucauct t 21

<210> 318
<211> 19
<212> DNA
<213> Artificial Sequence

<220>
<223> Synthetic Construct

<400> 318
caagtgaggt ggaagagaa 19

<210> 319
<211> 19
<212> DNA
<213> Artificial Sequence

<220>
<223> Synthetic Construct

<400> 319
atagtgggtt agggagaaa 19

<210> 320
<211> 19
<212> DNA
<213> Artificial Sequence

<220>
<223> Synthetic Construct

<400> 320
agaaagggat ggagggaaa 19

<210> 321
<211> 19
<212> DNA
<213> Artificial Sequence

<220>
<223> Synthetic Construct

<400> 321
agaaagggat ggagggaaa 19

<210> 322
<211> 19
<212> DNA
<213> Artificial Sequence

<220>
<223> Synthetic Construct

<400> 322

aagtgaggtg gaagagaaa 19

<210> 323
<211> 19
<212> DNA
<213> Artificial Sequence

<220>
<223> Synthetic Construct

<400> 323
gagaagtgga gaggaagaa 19

<210> 324
<211> 19
<212> DNA
<213> Artificial Sequence

<220>
<223> Synthetic Construct

<400> 324
gatggagaga aagacagaa 19

<210> 325
<211> 19
<212> DNA
<213> Artificial Sequence

<220>
<223> Synthetic Construct

<400> 325
gagatgaggg taccaacaa 19

<210> 326
<211> 19
<212> DNA
<213> Artificial Sequence

<220>
<223> Synthetic Construct

<400> 326
gtgggttagg gagaaagaa 19

<210> 327
<211> 19
<212> DNA
<213> Artificial Sequence

<220>
<223> Synthetic Construct

<400> 327
gtgaggtgga agagaaaaa 19

<210> 328
<211> 19
<212> DNA
<213> Artificial Sequence

<220>
<223> Synthetic Construct

<400> 328
agggcaccat ttggcttta 19

<210> 329
<211> 19
<212> DNA
<213> Artificial Sequence

<220>
<223> Synthetic Construct

<400> 329
ttcctccttg ggtcggtta 19

<210> 330
<211> 19
<212> DNA
<213> Artificial Sequence

<220>
<223> Synthetic Construct

<400> 330
gccagaggaa agtaaggaa 19

<210> 331
<211> 19
<212> DNA
<213> Artificial Sequence

<220>
<223> Synthetic Construct

<400> 331
tgcacacgtt agcaatcaa 19

<210> 332
<211> 19
<212> DNA
<213> Artificial Sequence

<220>
<223> Synthetic Construct

<400> 332
aaggctttga atggccaat 19

<210> 333
<211> 19
<212> DNA

<213> Artificial Sequence

<220>
<223> Synthetic Construct

<400> 333
ttgagttggc gggaagaaa 19

<210> 334
<211> 19
<212> DNA
<213> Artificial Sequence

<220>
<223> Synthetic Construct

<400> 334
atcgcttgga gggaacttg 19

<210> 335
<211> 19
<212> DNA
<213> Artificial Sequence

<220>
<223> Synthetic Construct

<400> 335
ggagttcagg gaaatccaa 19

<210> 336
<211> 19
<212> DNA
<213> Artificial Sequence

<220>
<223> Synthetic Construct

<400> 336
cccctatgtg tcttgcatt 19

<210> 337
<211> 19
<212> DNA
<213> Artificial Sequence

<220>
<223> Synthetic Construct

<400> 337
cctatgtgtc ttgcatttc 19

<210> 338
<211> 19
<212> DNA
<213> Artificial Sequence

<220>

<223> Synthetic Construct

<400> 338
gcacacgtta gcaatcaag 19

<210> 339
<211> 19
<212> DNA
<213> Artificial Sequence

<220>
<223> Synthetic Construct

<400> 339
gtagctcaga gaaggcttt 19

<210> 340
<211> 19
<212> DNA
<213> Artificial Sequence

<220>
<223> Synthetic Construct

<400> 340
tagctcagag aaggctttg 19

<210> 341
<211> 19
<212> DNA
<213> Artificial Sequence

<220>
<223> Synthetic Construct

<400> 341
gctcagagaa ggctttgaa 19

<210> 342
<211> 19
<212> DNA
<213> Artificial Sequence

<220>
<223> Synthetic Construct

<400> 342
ggaacctcca aggttccta 19

<210> 343
<211> 19
<212> DNA
<213> Artificial Sequence

<220>
<223> Synthetic Construct

<400> 343

tcgccttcag ctggttcta 19

<210> 344
<211> 19
<212> DNA
<213> Artificial Sequence

<220>
<223> Synthetic Construct

<400> 344
gaggtcatgg atgtcggaa 19

<210> 345
<211> 19
<212> DNA
<213> Artificial Sequence

<220>
<223> Synthetic Construct

<400> 345
acctccaagg ttcctacaa 19

<210> 346
<211> 19
<212> DNA
<213> Artificial Sequence

<220>
<223> Synthetic Construct

<400> 346
acttcttgtc tctgctaac 19

<210> 347
<211> 19
<212> DNA
<213> Artificial Sequence

<220>
<223> Synthetic Construct

<400> 347
actcgagtct agcactatc 19

<210> 348
<211> 19
<212> DNA
<213> Artificial Sequence

<220>
<223> Synthetic Construct

<400> 348
ggacctgaaa actactgtc 19

<210> 349
<211> 19
<212> DNA
<213> Artificial Sequence

<220>
<223> Synthetic Construct

<400> 349
gggaatttag gaggtcatg 19

<210> 350
<211> 19
<212> DNA
<213> Artificial Sequence

<220>
<223> Synthetic Construct

<400> 350
tggctttaag acaccggaa 19

<210> 351
<211> 19
<212> DNA
<213> Artificial Sequence

<220>
<223> Synthetic Construct

<400> 351
aacctccaag gttcctaca 19

<210> 352
<211> 19
<212> DNA
<213> Artificial Sequence

<220>
<223> Synthetic Construct

<400> 352
cctccaaggt tcctacaaa 19

<210> 353
<211> 19
<212> DNA
<213> Artificial Sequence

<220>
<223> Synthetic Construct

<400> 353
tcagctggtt ctaaacttc 19

<210> 354
<211> 19
<212> DNA

<213> Artificial Sequence

<220>
<223> Synthetic Construct

<400> 354
gctggttcta aacttcttg 19

<210> 355
<211> 19
<212> DNA
<213> Artificial Sequence

<220>
<223> Synthetic Construct

<400> 355
ctcactttac tcgagtcta 19

<210> 356
<211> 19
<212> DNA
<213> Artificial Sequence

<220>
<223> Synthetic Construct

<400> 356
gataaggtca gttgggaat 19

<210> 357
<211> 19
<212> DNA
<213> Artificial Sequence

<220>
<223> Synthetic Construct

<400> 357
tgggaattta ggaggtcat 19

<210> 358
<211> 19
<212> DNA
<213> Artificial Sequence

<220>
<223> Synthetic Construct

<400> 358
gtcatggatg tcggaaata 19

<210> 359
<211> 19
<212> DNA
<213> Artificial Sequence

<220>

<223> Synthetic Construct

<400> 359
gaggaatgct tgaggccaa 19

<210> 360
<211> 19
<212> DNA
<213> Artificial Sequence

<220>
<223> Synthetic Construct

<400> 360
cctgtctcta gaaaaggaa 19

<210> 361
<211> 19
<212> DNA
<213> Artificial Sequence

<220>
<223> Synthetic Construct

<400> 361
agaccctgtc tctagaaaa 19

<210> 362
<211> 19
<212> DNA
<213> Artificial Sequence

<220>
<223> Synthetic Construct

<400> 362
aagaccctgt ctctagaaa 19

<210> 363
<211> 19
<212> DNA
<213> Artificial Sequence

<220>
<223> Synthetic Construct

<400> 363
tgatttcctc ctctgtgaa 19

<210> 364
<211> 19
<212> DNA
<213> Artificial Sequence

<220>
<223> Synthetic Construct

<400> 364

caagaccctg tctctagaa 19

<210> 365
<211> 19
<212> DNA
<213> Artificial Sequence

<220>
<223> Synthetic Construct

<400> 365
tggagaggca gaaggaaaa 19

<210> 366
<211> 19
<212> DNA
<213> Artificial Sequence

<220>
<223> Synthetic Construct

<400> 366
tcctctgtga aatgggcaa 19

<210> 367
<211> 19
<212> DNA
<213> Artificial Sequence

<220>
<223> Synthetic Construct

<400> 367
agagggagat cgaggctta 19

<210> 368
<211> 19
<212> DNA
<213> Artificial Sequence

<220>
<223> Synthetic Construct

<400> 368
gtcactttgg gaggcttga 19

<210> 369
<211> 19
<212> DNA
<213> Artificial Sequence

<220>
<223> Synthetic Construct

<400> 369
gatttcctcc tctgtgaaa 19

<210> 370
<211> 19
<212> DNA
<213> Artificial Sequence

<220>
<223> Synthetic Construct

<400> 370
ctgaaagagg tgctggtta 19

<210> 371
<211> 19
<212> DNA
<213> Artificial Sequence

<220>
<223> Synthetic Construct

<400> 371
ctcatgcctg taatcccaa 19

<210> 372
<211> 19
<212> DNA
<213> Artificial Sequence

<220>
<223> Synthetic Construct

<400> 372
gaccctgtct ctagaaaag 19

<210> 373
<211> 1000
<212> DNA
<213> Artificial Sequence

<220>
<223> Synthetic Construct

<400> 373
gtggaggttg cagggagccg agatcacgcc attgcactcc acctaggcga cagagagaga 60

ctccgtctaa aaaaaaaaaa gagacagact cttccttggc tgggggtaag tcagatggga 120

gaggagaggg ttaaaaacag ctgggactca gcctgctggc aaacatgtgg catgtggcat 180

gtcggggcaa ctgcagctca gcctctggag ccatgtgagc aatgcacgca ggtacacgtg 240

tgacaagcta ggtcacctag ccatgttcaa caggcatgtg cacagccacg aggaatgccc 300

agccgtacaa ttaggcacac aggacatccg ccatgtgtag acacagctgt ggacatagct 360

ggccaggaca tgcgacacac gacgtgctca tagcacaggg agaagggccc atgaagtctg 420

gttggaactc agcacgtgtg tctgtgtgcc cacctgagtc tggactgctg cccctctgac 480

actagctgtc cccttgaagg gtcggtgcct tatctgtcct gacagaagag acagtgttgc 540

ttctcacttg gggctcgcag cctcctcctc ctgcctcgaa ctgaggatct gttgggtcca 600

gtcatcctgg agagatgcgg ccagtttctt tctgacaggt ctcctcctgc ccgcaaggaa 660

gtggggtgat cacagggcgc aggtggtctc tatgacagct gcatcctctc cagccatggc 720

cctgaaccct gcctataatc ccaccattgg ctctcagatc tgcctaagcc tctcagcccc 780

cttgacgtcc cctcccttaa gcgccctccg aaggccaccc gtccctcaaa gctcctcaca 840

ctccatgccc gcagctccct ccacccggcg tccgcaccag cctcccagcc gaggtggggc 900

gggggcgagg gggcgcgcac tcctcggcgc tccgggactg cagggcgggg ctgcagggcg 960

ggcggggccc gtgtctccag cgctcctata aagggagcca 1000

<210> 374
<211> 500
<212> DNA
<213> Artificial Sequence

<220>
<223> Synthetic Construct

<400> 374
tattttcaaa ctggaatgct tcttcagaag aaacccaaac tggaatgttt cttcagaaga 60

aacttcagaa gtcaccaaaa aggtgactcc tccatctttc tgattcttgg atcctaaagt 120

atcttcagca agtgaagccc agtttcataa ccgaagaggt gactccatct gtcctacaga 180

cttgcagtgt gaagcttgca atggtttctc tgtatgttaa tttctccatc tctaggatgg 240

cctatgtggt tcttactaac ctcagagtgg tatcatgaag ccactgaaat gataccagaa 300

gaaaacaaga gtgattaatc ttctaggatg ataaatctat attttcttca aattcttcat 360

tttgcataaa aaccttttc agaaaagaat gttaacattg gatatatcta cacctgattt 420

gacagttcca aactacaccc caaccaccca cttctgaatg agaagaaaaa aaaatcagaa 480

gcctgcaatt gtgtaacatg 500

<210> 375
<211> 1000
<212> DNA
<213> Artificial Sequence

<220>
<223> Synthetic Construct

<400> 375
tcctattctc taggtctcac atttcttctc ctctagcagt agtgggaagt gaggggtggg 60

ggacacgacc ctcccctgtt ccatcccaca ctccaacccc caaaatcccc cagggtcccc 120

gtccagctca gtcctggggg cagaaatgca gagttctcca ggaacgtggt cccagctgtt 180

tcagtgcagg ccgccccctc ctggccacca gcggaatgtc agccttccca gaggggccgg 240

gagaacagca gtcgagaagc tcccagactg gtgtgggcgc tagctgtgct cagcgtgggg 300

atgggaggtg acccagtgat aatgggaagc tgggctgcct gtcagtctgc ggggggctcc 360

cacctccctg ttcccccaca gggcacctgg ggatccagcc tgatttttac cagaccctgc 420

ggcctgcatg ggctgggta tagggctgtg accttgaccc atgcagaata gaaccctgtg 480

tgtcgggatc ctccatgtgc tccagatgcc cctggggaca gcaccaacat ggccttaact 540

cccaagccat tcccctgcct ctaacccct ggcatctgca ggcatccacc ccagacccac 600

ccaacacctc ctccccagct tcaggcgcta ggcagagacc ttggcccctg cagaatgcag 660

ccctgtccag ggtcccctac cttcccccca gatccctccc agagcaatac caacccgggc 720

ctaccttcca ggccattcaa cctgcagccc cccggcctct gtagacatcg cacccccaa 780

acccccagac ctgcccaatg cctcccctcc ccagctttgg gcagaacctg tctctagcca 840

gacctggggg tgttggggag tctggagggc cggggtgggg gctgaggcgc gggacagctg 900

gcccgtatcc tcacactggg cccggggccc agccggaggg gcgggggcct ggccactcgg 960

gccttggctg ggctgggat ttttggcctg gccgccaggc 1000

<210> 376
<211> 500
<212> DNA
<213> Artificial Sequence

<220>
<223> Synthetic Construct

<400> 376
gttttggaga ggtcctcagc atgcctcttt atgcccctcc cttagctctt gccaggatat 60

cagagggtga ctggggcaca gccaggagga ccccctcccc aacaccccca acccttccac 120

ctttggaagt ctccccaccc agctccccag ttccccagtt ccacttcttc tagattggag 180

gtcccaggaa gagagcagag gggcacccct acccactggt tagcccacgc cattctgagg 240

acccagctgc accctacca cagcacctct ggcccaggct gggctggggg gctggggagg 300

cagagctgcg aagaggggag atgtggggtg gactcccttc cctcctcctc cccctctcca 360

ttccaactcc caaattgggg gccgggccag gcagctctga ttggctgggg cacgggcggc 420

cggctccccc tctccgaggg gcagggttcc tccctgctct ccatcaggac agtataaaag 480

gggcccgggc cagtcgtcgg 500

<210> 377
<211> 500
<212> DNA
<213> Artificial Sequence

<220>
<223> Synthetic Construct

<400> 377

atgagcctca gcaaaggcaa gctaggaggt cgaaggactt ccccaggtga ctcggtctag 60

tctagagttc gcaaagccta tcctccctgt agccgggtgc caagcagcct cgagcctgct 120

ccccagccca cctgccaaca aaaggcgccc tccgactgca acccagccct ccacagacag 180

gacccgccct ttcccgaagt cataagacaa agagagtgca tcactgctga aacagtgggc 240

gcacacgagc cccaaagcta gagaaaagct ggacggggct gggggcgggg tgcaggggtg 300

gaggggcggg gaggcgggct ccggctgcgc cacgctatcg agtcttccct ccctccttct 360

ctgcccccte cgctcccgct ggagccctcc accctacaag tggcctacag ggcacaggtg 420

aggcgggact ggacagctcc tgctttgatc gccggagatc tgcaaattct gcccatgtcg 480

gggctgcaga gcactccgac 500

<210> 378
<211> 500
<212> DNA
<213> Artificial Sequence

<220>
<223> Synthetic Construct

<400> 378
tgatatttgc ctgaaactta acttctagga cccagggtgg gtggatgagt cgaaggggca 60

tacggtggca tttctttccg tgagtctctt acagtctcct atttaaattg agttaggatt 120

acttctggca aaatcccaac ataaaaatct tctaggaaga tcagttctgt aaattagaca 180

tactagataa atgggcatca agcagttttt caaaattatg cagttgttaa cttcataagg 240

ggaaataaaa atgtatgcat ttacattgta tgtattaaaa caaggcagag catttctata 300

cgttcctaag ttatacaaac atatatgtaa gagtgaaata tgtaaaaaaa cttttacata 360

agcagatgca tacaaactcc agatgtgctc tttttcttac tgtgggttgt gtcttctata 420

agggaaaaag aaatatttat catttctttt actgctgagg ggatgggtgc ggctctcata 480

tttcagaaag gggctggaaa 500

<210> 379
<211> 500
<212> DNA
<213> Artificial Sequence

<220>
<223> Synthetic Construct

<400> 379
agagaagtgg agaggaagaa agggatggag agaaagacag aaacttggag aaatgtggta 60

atggggagag agatgagggt accaacaaag agatgaggag aaatagtggg ttagggagaa 120

agaaagggat ggagggaaat acagggatag gggatggggg gatgagggga acccccaaaa 180

tgcaagtgag gtggaagaga aaaatcaggg gagacaaggg acgagagggg ggaggaagag 240

agaagaagac atagaggagg gagggagagg agggggagtg atggcagaga ggactagagg 300

aggggcgcgg gtagacatgg gggcctggga agggaggccc gtctggctga ggggggtggg 360

agtaatagcg ggaagcgggt ggagctgccc ggctgggccg tgacctcaga gcccctggcc 420

cagcttagcc tgactgacgt cagcgctcgc tcccgccccc gcctgcgctg gtcttcaaat 480

gacccccctc cagctctctg 500

<210> 380
<211> 1000
<212> DNA
<213> Artificial Sequence

<220>
<223> Synthetic Construct

<400> 380
ggagtggtgg ggggaggagt aaccaaaaaa cgaactctac ccagccttgg ggcattgtga 60

aaacgacgag gaaaggccat ctccaagcaa gacatatttg tcctcgtcaa ctttgcagaa 120

ggcttgcaaa aagcaaaagc cagttaagcc atttccttca agatggctaa atataagtcc 180

tccagaaaac aatttttatt attttatttt taatgcgcgc tgtttgagta tgtttacgtt 240

aggggaccag attgagcaaa aatatacata taattacttt tctatgtttt ccgcccaccc 300

acggcagaaa cctctttatg agaaaggctt tgacacttga cgtcagctca gaaacttttg 360

agttggcggg aagaaagggt taacaagtgg tggaccagcg cgatcttttt agagtcccag 420

ccggctgtcg aaggctccag gtacacacac acacgcacac acttacacac acacacacac 480

acgtgcacac acccctccca actgttcctc cttgggtcgg ttacttagct gaagggcacc 540

atttggcttt aaaacaaatt acttcagcgc cacagggagt ttatcgcttg gagggaactt 600

gtaacgcggg agttcaggga aatccaagag gcgagggctc ccacctccgc cgccagagga 660

aagtaaggaa tcaggtggtg gccccctatg tgtcttgcat ttctctgcac acgttagcaa 720

tcaagttaat tgaattcatt ggagtttaga accggcctgt agctcagaga aggctttgaa 780

tggccaattt ctctctctcc ctctccccct ccccgcctcc cgctcgcccg cccgcccgcg 840

ctcccagttc cctcccctca gggttcccca gtccacacct ccctctccac ttccctcacc 900

cccccactcc ctccgccgcc ctattaaaac acccaccagc tcacttgtta agaccccctt 960

aagttggagg aggcagaagg gcaacaacgg cggggaagga 1000

<210> 381
<211> 500
<212> DNA
<213> Artificial Sequence

<220>
<223> Synthetic Construct

<400> 381
gtggtgcgat ttcagctcac tgcaacctcc acctgcgggg ttcaagcgat tctcctgcct 60

cagcctcccg agtagctggg attacgggca tggtgcccgt aacgccgaaa gtgctgagat 120

tacaggcttg agccacggcg cccggccact catttttttaa aaactagtta ttaattataa 180

tttctcttca agttttcctg agatgtaaaa actcatcgcc ttcagctggt tctaaacttc 240

ttgtctctgc taacaactct ccccctcct cgatctcacc ccaccccagt gcaccccaac 300

tcacttcatc tcactttact cgagtctagc actatctttt agggacctga aaactactgt 360

cgataaggtc agttgggaat ttaggaggtc atggatgtcg gaaataaagg tgtgaaaaag 420

aagaggagga attgttttgg ctttaagaca ccggaacctc caaggttcct acaaaaccgt 480

ttgcagcttt tgcaaaccgc 500

<210> 382
<211> 500
<212> DNA
<213> Artificial Sequence

<220>
<223> Synthetic Construct

<400> 382
ggacatttgt gggacacata ggctgggtca gggctgaaag aggtgctggt tatggccggg 60

ggcagggact catgcctgta atcccaacag cccaggagga tgagacagga ggaatgcttg 120

aggccaaaat ttcgaggccg gaagttccag acgagcctgg gcaacacagc aagaccctgt 180

ctctagaaaa ggaaagaaag aaccgctggt tgtggaagcc agccatggcc cagagctcag 240

cagtgtagga gaggagggtg cgggcctgag agaggcagca ggcttggctg gagaggcaga 300

aggaaaacca aggcaggaga gtgtcctgga agctgggaga aggcagaggg agatcgaggc 360

ttactttctg gctgaggggc ctagggtgag tcactttggg aggcttgatt tcctcctctg 420

tgaaatgggc aacacaccta cccttgccca cctcaccccc ctcaccccgt cgacgtcgag 480

tgaggagcag ctgtgaggag 500

<210> 383
<211> 21
<212> DNA
<213> Artificial Sequence

<220>
<223> Synthetic Construct

<400> 383
acuacugagu gacaguagat t 21

<210> 384
<211> 21

<212> DNA
<213> Artificial Sequence

<220>
<223> Synthetic Construct

<400> 384
ucuacuguca cucaguagut t 21

<210> 385
<211> 20
<212> DNA
<213> Artificial Sequence

<220>
<223> Synthetic Construct

<400> 385
cactctgggc atcctcatcg 20

<210> 386
<211> 23
<212> DNA
<213> Artificial Sequence

<220>
<223> Synthetic Construct

<400> 386
gcgaagtgcc agattgcatc ata 23

<210> 387
<211> 20
<212> DNA
<213> Artificial Sequence

<220>
<223> Synthetic Construct

<400> 387
tcctcagaga agccccaaga 20

<210> 388
<211> 21
<212> DNA
<213> Artificial Sequence

<220>
<223> Synthetic Construct

<400> 388
agccacatcc aaggacaatc a 21

<210> 389
<211> 18
<212> DNA
<213> Artificial Sequence

<220>
<223> Synthetic Construct

<400> 389
cggctcagag tcacccac 18

<210> 390
<211> 19
<212> DNA
<213> Artificial Sequence

<220>
<223> Synthetic Construct

<400> 390
aagtccaggc tgtccaggg 19

<210> 391
<211> 20
<212> DNA
<213> Artificial Sequence

<220>
<223> Synthetic Construct

<400> 391
cagccggaga tagaggacca 20

<210> 392
<211> 20
<212> DNA
<213> Artificial Sequence

<220>
<223> Synthetic Construct

<400> 392
cagcaaagtt cccaccgaga 20

<210> 393
<211> 20
<212> DNA
<213> Artificial Sequence

<220>
<223> Synthetic Construct

<400> 393
ccaggagcta acggtctcag 20

<210> 394
<211> 20
<212> DNA
<213> Artificial Sequence

<220>
<223> Synthetic Construct

<400> 394
caggtttcc atctcttcca 20

<210> 395
<211> 20
<212> DNA
<213> Artificial Sequence

<220>
<223> Synthetic Construct

<400> 395
tcctggtgga gttcctggag 20

<210> 396
<211> 20
<212> DNA
<213> Artificial Sequence

<220>
<223> Synthetic Construct

<400> 396
ccgggaactg gcttaagagg 20

<210> 397
<211> 23
<212> DNA
<213> Artificial Sequence

<220>
<223> Synthetic Construct

<400> 397
taggaataac ctcttgcagc agt 23

<210> 398
<211> 20
<212> DNA
<213> Artificial Sequence

<220>
<223> Synthetic Construct

<400> 398
gacctggagg tgtacttggt 20

<210> 399
<211> 21
<212> DNA
<213> Artificial Sequence

<220>
<223> Synthetic Construct

<400> 399
cagaatccaa acagacagtt c 21

<210> 400
<211> 21
<212> DNA
<213> Artificial Sequence

<220>
<223> Synthetic Construct

<400> 400
taaggtgttg tgtgtgactg a 21

<210> 401
<211> 20
<212> DNA
<213> Artificial Sequence

<220>
<223> Synthetic Construct

<400> 401
ggaaggtttt gctgccttgg 20

<210> 402
<211> 17
<212> DNA
<213> Artificial Sequence

<220>
<223> Synthetic Construct

<400> 402
gcaccggcat cctgcaa 17

<210> 403
<211> 21
<212> DNA
<213> Artificial Sequence

<220>
<223> Synthetic Construct

<400> 403
caacttggtc tcacagtggc t 21

<210> 404
<211> 23
<212> DNA
<213> Artificial Sequence

<220>
<223> Synthetic Construct

<400> 404
gtgtcagaga ggacagctga aaa 23

<210> 405
<211> 21

<212>  DNA
<213>  Artificial Sequence

<220>
<223>  Synthetic Construct

<400>  405
atggacagga ctgaacgtct t                                                 21

<210>  406
<211>  20
<212>  DNA
<213>  Artificial Sequence

<220>
<223>  Synthetic Construct

<400>  406
tccagcaggt cagcaaagaa                                                   20

<210>  407
<211>  21
<212>  DNA
<213>  Artificial Sequence

<220>
<223>  Synthetic Construct

<400>  407
atcaccatct tccaggagcg a                                                 21

<210>  408
<211>  21
<212>  DNA
<213>  Artificial Sequence

<220>
<223>  Synthetic Construct

<400>  408
ttctccatgg tggtgaagac g                                                 21

<210>  409
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  409
ccggcaucuu ugcuaccuat t                                                 21

<210> 410
<211> 21
<212> DNA
<213> Artificial Sequence

<220>
<223> Synthetic Construct

<220>
<221> misc_feature
<222> (20)..(21)
<223> deoxythymine (dT) base

<400> 410
uagguagcaa agaugccggt t 21

<210> 411
<211> 21
<212> DNA
<213> Artificial Sequence

<220>
<223> Synthetic Construct

<220>
<221> misc_feature
<222> (20)..(21)
<223> deoxythymine (dT) base

<400> 411
gcaguugcaa uggccauuut t 21

<210> 412
<211> 20
<212> DNA
<213> Artificial Sequence

<220>
<223> Synthetic Construct

<220>
<221> misc_feature
<222> (20)..(21)
<223> deoxythymine (dT) base

<400> 412
aaauggccau ugcaacugct 20

<210> 413
<211> 21
<212> DNA
<213> Artificial Sequence

<220>
<223> Synthetic Construct

<220>
<221> misc_feature
<222> (20)..(21)
<223> deoxythymine (dT) base

<400> 413
gucucgcugu gauagaucat t 21

<210> 414
<211> 21
<212> DNA
<213> Artificial Sequence

<220>
<223> Synthetic Construct

<220>
<221> misc_feature
<222> (20)..(21)
<223> deoxythymine (dT) base

<400> 414
ugaucuauca cagcgagact t 21

<210> 415
<211> 21
<212> DNA
<213> Artificial Sequence

<220>
<223> Synthetic Construct

<220>
<221> misc_feature
<222> (20)..(21)
<223> deoxythymine (dT) base

<400> 415
caaucaccug cguacagaat t 21

<210> 416
<211> 21
<212> DNA
<213> Artificial Sequence

<220>
<223> Synthetic Construct

<220>
<221> misc_feature
<222> (20)..(21)
<223> deoxythymine (dT) base

<400> 416
uucuguacgc aggugauugt t 21

<210> 417
<211> 21
<212> DNA
<213> Artificial Sequence

<220>
<223> Synthetic Construct

<220>
<221> misc_feature
<222> (20)..(21)
<223> deoxythymine (dT) base

<400> 417
gcugcuugca guaaccuuat t 21

<210> 418
<211> 21
<212> DNA
<213> Artificial Sequence

<220>
<223> Synthetic Construct

<220>
<221> misc_feature
<222> (20)..(21)
<223> deoxythymine (dT) base

<400> 418
uaagguuacu gcaagcagct t 21

<210> 419
<211> 21
<212> DNA
<213> Artificial Sequence

<220>
<223> Synthetic Construct

<220>
<221> misc_feature
<222> (20)..(21)
<223> deoxythymine (dT) base

<400> 419
gcucugcuuc aucccacuat t 21

<210> 420
<211> 21
<212> DNA
<213> Artificial Sequence

<220>
<223> Synthetic Construct

<220>
<221> misc_feature
<222> (20)..(21)
<223> deoxythymine (dT) base

<400> 420
uagugggaug aagcagagct t 21

<210> 421
<211> 21
<212> DNA
<213> Artificial Sequence

<220>
<223> Synthetic Construct

<220>
<221> misc_feature
<222> (20)..(21)
<223> deoxythymine (dT) base

<400> 421
cugcaucagc gguccucuat t 21

<210> 422
<211> 21
<212> DNA
<213> Artificial Sequence

<220>
<223> Synthetic Construct

<220>
<221> misc_feature
<222> (20)..(21)
<223> deoxythymine (dT) base

<400> 422
uagaggaccg cugaugcagt t 21

<210> 423
<211> 21
<212> DNA
<213> Artificial Sequence

<220>
<223> Synthetic Construct

<220>
<221> misc_feature
<222> (20)..(21)
<223> deoxythymine (dT) base

<400> 423
ccagacuacu uaaggaaaut t 21

<210> 424
<211> 21
<212> DNA
<213> Artificial Sequence

<220>
<223> Synthetic Construct

<220>
<221> misc_feature
<222> (20)..(21)
<223> deoxythymine (dT) base

<400> 424
auuuccuuaa guagucuggt t 21

<210> 425
<211> 21
<212> DNA
<213> Artificial Sequence

<220>
<223> Synthetic Construct

<220>
<221> misc_feature
<222> (20)..(21)
<223> deoxythymine (dT) base

<400> 425
ccuacaugcu ggacaucuut t 21

<210> 426
<211> 21
<212> DNA
<213> Artificial Sequence

<220>
<223> Synthetic Construct

<220>
<221> misc_feature
<222> (20)..(21)
<223> deoxythymine (dT) base

<400> 426
aagaugucca gcauguaggt t 21

<210> 427
<211> 21
<212> DNA
<213> Artificial Sequence

<220>
<223> Synthetic Construct

<220>
<221> misc_feature
<222> (20)..(21)
<223> deoxythymine (dT) base

<400> 427
cccacuauag cgaccagaut t 21

<210> 428
<211> 20
<212> DNA
<213> Artificial Sequence

<220>
<223> Synthetic Construct

<220>
<221> misc_feature
<222> (20)..(21)
<223> deoxythymine (dT) base

<400> 428
aucuggucgc uauagugggt 20